# EUROPÄISCHE PATENTSCHRIFT

(11) **EP 1 561 116 B1**
(45) Veröffentlichungstag und Bekanntmachung des Hinweises auf die Patenterteilung: **07.05.2008**
(21) Anmeldenummer: 03779880.8
(22) Anmeldetag: 10.11.2003
(51) Int. Cl.: G01N 33/68, C07K 14/705

(54) **SCD40L, PAPP-A UND PLAZENTALER-WACHSTUMSFAKTOR (PIGF) ALS BIOCHEMISCHE MARKERKOMBINATIONEN BEI KARDIOVASKULÄREN ERKRANKUNGEN**
SCD40L AND PLACENTAL GROWTH FACTOR (PLGF) USED AS A BIOCHEMICAL MARKER COMBINATION IN CARDIOVASCULAR DISEASES
SCD40L ET FACTEUR DE CROISSANCE PLACENTAIRE (PIGF) SERVANT DE COMBINAISONS DE MARQUEURS BIOCHIMIQUES POUR DES MALADIES CARDIOVASCULAIRES

(30) Priorität: 16.11.2002 DE 10253525; 08.04.2003 DE 10316059
(43) Veröffentlichungstag der Anmeldung: 10.08.2005
(73) Patentinhaber: Dade Behring Marburg GmbH, 35041 Marburg (DE)
(72) Erfinder: ZEIHER, Andreas, M., 60594 Frankfurt am Main (DE); HEESCHEN, Christopher, 60431 Frankfurt am Main (DE); DIMMELER, Stefanie, 60594 Frankfurt am Main (DE); HAMM, Christian, 61350 Bad Homburg (DE)
(74) Vertreter: Krauss, Jan
(86) Internationale Anmeldenummer: PCT/EP2003/012531
(87) Internationale Veröffentlichungsnummer: WO 2004/046722

(56) Entgegenhaltungen:
- WO-A-01/56593
- WO-A-01/85796
- WO-A-98/20155
- WO-A-02/056015
- WO-A-03/040691
- SCHONBECK UWE ET AL: "Soluble CD40L and cardiovascular risk in women" CIRCULATION, Bd. 104, Nr. 19, 6. November 2001 (2001-11-06), Seiten 2266-2268, XP002273044 ISSN: 0009-7322 in der Anmeldung erwähnt
- PENG DAO-QUAN ET AL: "Elevated soluble CD40 ligand is related to the endothelial adhesion molecules in patients with acute coronary syndrome" CLINICA CHIMICA ACTA, Bd. 319, Nr. 1, 7. Mai 2002 (2002-05-07), Seiten 19-26, XP002273045 ISSN: 0009-8981 in der Anmeldung erwähnt
- YAN JINCHUAN ET AL: "Clinical implications of increased expression of CD40L in patients with acute coronary syndromes" CHINESE MEDICAL JOURNAL (ENGLISH EDITION), Bd. 115, Nr. 4, April 2002 (2002-04), Seiten 491-493, XP008028508 ISSN: 0366-6999
- AUKRUST PAL ET AL: "Enhanced levels of soluble and membrane-bound CD40 ligand in patients with unstable angina: Possible reflection of T lymphocyte and platelet involvement in the pathogenesis of acute coronary syndromes" CIRCULATION, Bd. 100, Nr. 6, 10. August 1999 (1999-08-10), Seiten 614-620, XP002273046 ISSN: 0009-7322 in der Anmeldung erwähnt
- HEESCHEN CHRISTOPHER ET AL: "CD40 ligand serum levels independently predicts outcome and effect of anti-platelet therapy in patients with unstable angina." CIRCULATION, Bd. 106, Nr. 19 Supplement, 5. November 2002 (2002-11-05), Seite II.402, XP008028499 Abstracts from Scientific Sessions;Chicago, IL, USA; November 17-20, 2002 ISSN: 0009-7322 (ISSN print)
- GARLICHS C D ET AL: "Patients with acute coronary syndromes express enhanced CD40 ligand/CD154 on platelets" HEART (LONDON), Bd. 86, Nr. 6, Dezember 2001 (2001-12), Seiten 649-655, XP008028515 ISSN: 1355-6037
- BAYES-GENIS ANTONIO ET AL: "Identification of pregnancy-associated plasma protein A (PAPP-A) as a new marker of acute coronary syndromes" JOURNAL OF THE AMERICAN COLLEGE OF CARDIOLOGY, Bd. 37, Nr. 2 Supplement A, Februar 2001 (2001-02), Seite 301A, XP008028486 50th Annual Scientific Session of the American College of Cardiology;Orlando, Florida, USA; March 18-21, 2001 ISSN: 0735-1097
- VARO NEREA ET AL: "Soluble CD40L: risk prediction after acute coronary syndromes." CIRCULATION. UNITED STATES 2 SEP 2003, Bd. 108, Nr. 9, 2. September 2003 (2003-09-02), Seiten 1049-1052, XP002273047 ISSN: 1524-4539
- BAYES-GENIS ANTONI ET AL: "Pregnancy-associated plasma protein A as a marker of acute coronary syndromes" NEW ENGLAND JOURNAL OF MEDICINE, Bd. 345, Nr. 14, 4. Oktober 2001 (2001-10-04), Seiten 1022-1029, XP002909589 ISSN: 0028-4793
- KHOSRAVI JAVAD ET AL: "Pregnancy associated plasma protein-A: Ultrasensitive immunoassay and determination in coronary heart disease." CLINICAL BIOCHEMISTRY, Bd. 35, Nr. 7, Oktober 2002 (2002-10), Seiten 531-538, XP001188913 ISSN: 0009-9120
- DENKTAS A E ET AL: "PREGNANCY ASSOCIATED PLASMA PROTEIN-A LEVELS ARE ELEVATED IN PATIENTS WITH UNSTABLE ANGINA" EUROPEAN HEART JOURNAL, THE EUROPEAN SOCIETY OF CARDIOLOGY, XX, Bd. 23, Nr. ABSTR SUPPL, 31. August 2002 (2002-08-31), Seite 466, XP008028493 ISSN: 0195-668X
- HAJEK P ET AL: "Pregnancy-associated plasma protein a (PAPP-A) in determination of unstable coronary plaque." EUROPEAN HEART JOURNAL, Bd. 23, Nr. Abstract Supplement, 2002, Seite 293, XP008028492 & CONGRESS OF THE EUROPEAN SOCIETY OF CARDIOLOGY; BERLIN, GERMANY; AUGUST 31-SEPTEMBER 04, 2002 ISSN: 0195-668X
- FUTTERMAN LAURIE G ET AL: "Novel markers in the acute coronary syndrome: BNP, IL-6, PAPP-A." AMERICAN JOURNAL OF CRITICAL CARE : AN OFFICIAL PUBLICATION, AMERICAN ASSOCIATION OF CRITICAL-CARE NURSES. MAR 2002, Bd. 11, Nr. 2, März 2002 (2002-03), Seiten 168-172, XP008031012 ISSN: 1062-3264
- MAGLIONE D ET AL: "RECOMBINANT PRODUCTION OF PIGF-1 AND ITS ACTIVITY IN ANIMAL MODELS" FARMACO, SOCIETA CHIMICA ITALIANA, PAVIA, IT, Bd. 55, 2000, Seiten 165-167, XP001000056 ISSN: 0014-827X
- LUTTUN A ET AL: "Revascularization of ischemic tissues by PlGF treatment, and inhibition of tumor angiogenesis, arthritis and atherosclerosis by anti-Flt1" NATURE MEDICINE, NATURE PUBLISHING, CO, US, Bd. 8, Nr. 8, August 2002 (2002-08), Seiten 831-840, XP002258708 ISSN: 1078-8956

## Beschreibung

Die Erfindung betrifft neue Marker der vaskulären Entzündung und Kombinationen davon als Werkzeuge zur Diagnose und deren Prognose in Patienten mit kardiovaskulären Erkrankungen. Die Marker dienen weiterhin als Werkzeuge, die die Auswahl von Wirkstoffen zur Behandlung solcher Erkrankungen erleichtern und schließlich als Ansatzpunkt für die Behandlung von kardiovaskulären Erkrankungen. Weiterhin betrifft die Erfindung die Erstellung eines individuellen Risikoprofils von negativen Vorgängen, die mit dem Fortschreiten der Arteriosklerose zusammenhängen.

### Hintergrund der Erfindung

Eine Thrombusbildung im Koronargefäß ist das auslösende Ereignis einer instabilen koronaren Herzerkrankung. Die zentrale Rolle der Plättchenaktivierung wird bei Patienten mit einer instabilen koronaren Herzerkrankung zusätzlich noch durch Thromboxan- und Prostaglandin-Metaboliten, die von den Plättchen freigesetzt werden, gefördert. Deshalb ist die Plättchenaktivierung ein allgemeines therapeutisches Ziel. Bisher haben derartige Therapien die Verwendung von Aspirin, Tienopyridinen und einem direkten Glycoprotein IIb/IIIa-Inhibitor umfaßt. Allerdings konnte ein zuverlässiger biochemischer Marker der Plättchenaktivierung bis heute nicht identifiziert werden. Ergebnisse mit P-Selektin, dem bisher aussichtsreichsten Marker für eine Plättchenaktivierung, sind bis heute kontrovers.

Individuen, die an einer kardiovaskulären Erkrankung leiden, können in Individuen eingeteilt werden, die keine Symptome zeigen und diejenigen, die Brustschmerz zeigen. Die zuletzt genannte Gruppe kann in Individuen eingeteilt werden, die eine stabile Angina pectoris (SAP) aufweisen und diejenigen, mit akuten koronaren Syndromen (ACS). ACS Patienten können eine instabile Angina pectoris (IAP) aufweisen oder diese Patienten litten bereits an einem Myokardinfarkt (MI). Der MI kann ein ST-erhöhter MI oder ein nicht ST-erhöhter MI sein. Das Auftreten eines MI kann von einer linken ventrikulären Dysfunktion (LVD) begleitet werden. Zuletzt durchlaufen LVD Patienten ein kongestives Herzversagen (CHF) mit einer Sterblichkeitsrate von ungefähr 15 % oder zeigen keine Symptome.

Patienten, die mit Brustschmerzen eingeliefert werden, werden auf eine ST Erhöhung oder Verringerung hin untersucht. Falls dies der Fall ist wirkt das Individuen mit einer Wahrscheinlichkeit von nahezu 100 % im Krankenhaus verbleiben. Da jedoch nicht alle Individuen mit einem MI ST-Abnormalitäten zeigen, wird der Troponin (TnT) Spiegel bestimmt, der im Falle von ungewöhnlich hohen Werten eine hohe Wahrscheinlichkeit anzeigt, daß ein MI auf getreten ist.

Kürzliche Fortschritte in der Grundlagenforschung haben eine fundamentale Rolle für die Entzündung bei der Vermittlung aller Phasen der Arteriosklerose, von ihrem Beginn über Fortschreiten und, letztendlich, den thrombotischen Komplikationen von arteriosklerotischen Läsionen etabliert [Libby P, Ridker PM, Maseri A. Inflammation and atherosclerosis. Circulation 2002;105(9): 1135-43. Ross R. Atherosclerosis-an inflammatory disease. N Engl J Med 1999;340(2):115-26. Davies MJ, Thomas AC. Plaque fissuring-the cause of acute myocardial infarction, sudden ischaemic death, and crescendo angina. Br Heart J 1985;53(4):363-73. Libby P. Molecular bases of the acute coronary syndromes. Circulation 1995;91(11):2844-50.]. Die aus dem Zusammenhang zwischen Entzündung und Arteriosklerose erhaltenen Ergebnisse bilden die Rationale zur Verwendung von zirkulierenden Entzündungsmarkern als potentielle prädiktive Instrumente bei Patienten mit akuten Koronarsyndromen. In der Tat gehen erhöhte Spiegel von Entzündungsmarkern, wie zum Beispiel hoch sensitives C-reaktives Protein (hsCRP), Serum Amyloid A, und Interleukin-6 (IL-6) nicht nur allgemein mit akuten Koronarsyndromen einher [Berk BC, Weintraub WS, Alexander RW. Elevation of C-reactive protein in "active" coronary artery disease. Am J Cordial 1990;65(3):168-72., Biasucci LM, Vitelli A, Liuzzo G, et al. Elevated levels of interleukin-6 in unstable angina. Circulation 1996;94(5):874-7.], sondern - was wichtiger ist - können auch eine Aussage über den klinischen Ausgang von Patienten mit akuten Koronarsyndromen vorhersagen [Liuzzo G, Biasucci LM, Gallimore JR, et al. The prognostic value of C-reactive protein and serum amyloid a protein in severe unstable angina. N Engl J Med 1994;331(7):417-24., Biasucci LM, Liuzzo G, Grillo RL, et al. Elevated levels of C-reactive protein at discharge in patients with unstable angina predict recurrent instability. Circulation 1999;99(7):855-60., Toss H, Lindahl B, Siegbahn A, Wallentin L. Prognostic influence of increased fibrinogen and C-reactive protein levels in unstable coronary artery disease. FRISC Study Group. Fragmin during Instability in Coronary Artery Disease. Circulation 1997;96(12):4204-10.]. Obwohl das "klassische" akute Phase Protein hsCRP als der am meisten versprechende Biomarker für klinische Zwecke angesehen wird, besteht eine substantielle Heterogenität im Hinblick auf die Prävalenz von erhöhten hsCRP Spiegeln in Patienten mit akuten Koronarsyndromen [Biasucci LM, Liuzzo G, Colizzi C, Rizzello V. Clinical use of C-reactive protein .for the prognostic stratification of patients with ischemic heart disease. Ital Heart J 2001 ;2(3): 164-71.].

So zeigen mehr als 30 % der Patienten mit schwerer instabiler Angina keine erhöhten hsCRP Spiegel [Liuzzo G, Biasucci LM, Gallimore JR, et al. The prognostic value of C-reactive protein and serum amyloid a protein in severe unstable angina. N Engl J Med 1994;331(7):417-24., Heeschen C, Hamm CW, Bruemmer J, Simeons ML. Predictive value of C-reactive protein and troponin T in patients with unstable angina: a comparative analysis. CAPTURE Investigators. Chimeric c7E3 AntiPlatelet Therapy in Unstable angina refractory to standard treatment trial. J Am Coll Cardiol 2000;35(6):1535-42.]. Darüber hinaus können individuelle Unterschiede im Ausmaß der Response auf bestimmte entzündliche Stimuli möglicherweise die Spiegel der "stromabwärts" akute-Phase Reaktanten, wie hsCRP beeinflussen [Pepys MB, Hirschfield GM. C-reactive protein and its role in the pathogenesis of myocardial infarction. Ital Heart J2001,2(11):804-6., Liuzzo G, Biasucci LM, Rebuzzi AG, et al. Plasma protein acute-phase response in unstable angina is not induced by ischemic injury. Circulation 1996;94(10):2373-80.]. Daher besteht immer noch eine wichtige Herausforderung darin, proximale Stimuli für vaskuläre Entzündung zu identifizieren, die als Risikovorhersagende Serummarker in Patienten mit koronarer Arteriosklerose verwendet werden können.

Inzwischen mehren sich Hinweise darauf, daß das CD40-CD40L-System eine wichtige Rolle in der Pathophysiologie von Patienten mit einer instabilen koronaren Herzerkrankung spielt. Außer in der zellassoziierten Form tritt CD40L auch in einer löslichen, biologisch vollständig aktiven Form, nämlich sCD40L, auf. sCD40L wird von stimulierten Lymphozyten abgesto-ßen und auf Plättchenstimulation hin aktiv freigesetzt. sCD40L wirkt pro-imflammatorisch auf Endothelzellen und fördert die Koagulation, indem Monozyten und Endothelzellen zur Expression von Gewebefaktoren veranlaßt werden. Darüber hinaus enthält sCD40L eine KGD-Sequenz, ein bekanntes Bindungsmotiv, das für das vorherrschende Plättchen-Integrin αIIbβ3 spezifisch ist. CD40L ist tatsächlich ein αIIβ3-Ligand, ein Plättchenagonist, und notwendig für die Stabilität von arteriellen Thromben. In Serum von Patienten mit akuten Koronarsyndromen ist eine Erhöhung von sCD40L nachweisbar. Es wurde berichtet (Schonbeck U, Varo N, Libby P, Buring J, Ridker PM. Circulation 2001; 104: 2266-8), daß scheinbar gesunde Frauen, die erhöhte Plasmakonzentrationen an sCD40L aufwiesen, zugleich ein erhöhtes Risiko für kardiovaskuläre Ereignisse trugen.

Neuere Befunde zeigen, daß eine Ruptur von Plaques und die anschließende Bildung eines Thrombus bei Patienten mit akuten Koronarsyndromen zu einer Aktivierung der Exposition von CD40L in zirkulierenden Plättchen führen kann (Lee Y, Lee WH, Lee SC, Ahn KJ, Choi YH, Park SW, Seo JD, Park JE. Cardiology. 1999; 92: 11-6). Auch wurden erhöhte Konzentrationen an sCD40L bei Patienten mit Angina nachgewiesen, wobei die Konzentrationen bei Patienten mit instabiler Angina besonders hoch waren (Aukrust P, Muller F, Ueland T, Berget T, Aaser E, Brunsvig A, Solum NO, Forfang K, Froland SS, Gullestad L. Cirulation 1999; 100: 614-20). Diese Befunde legen nahe, daß eine CD40L-CD40-Interaktion eine wichtige Rolle bei der Pathogenese atherosklerotischer Prozesse und der Entwicklung koronarer Syndrome spielt.

Die Etablierung der korrekten Diagnose, verbunden mit einer geeigneten Behandlung von Patienten mit akuten koronaren Syndromen, die nicht mit einer Hebung der ST-Strecke einhergehen, kann sehr mühsam sein. Der Ausschluß von akuten Myokardinfarkten nach heutigen Standards ist nicht zufriedenstellend. In den letzten Jahren hat eine Konzentration auf die Risikostratifizierung und Steuerung der Behandlung stattgefunden mit dem Ziel, Patienten zu identifizieren, bei denen das Risiko besteht, lebensbedrohliche kardiologische Ereignisse zu entwickeln, und die vor allem von verbesserten therapeutischen und intervenierenden Strategien profitieren (Hamm CW, Bertrand M, Braunwald E. Lancet 2001; 358:1533-8). Das EKG hat in dieser Hinsicht nur begrenzte prognostische Relevanz, da bedeutende Abnormitäten selten sind und ihr Nachweis wenig empfindlich und spezifisch ist (Kaul P, Fu Y, Chang WC, et al., J Am Coll Cardiol 2001; 38:64-71 und Savonitto S, Ardissino D, Granger CB, et al. JAMA 1999; 281:707-13). Deshalb haben sich Marker einer Nekrose von Myokardzellen, vor allem kardiale Troponine, zu wertvollen Werkzeugen bei der Evaluierung von Patienten mit akuten koronaren Syndromen entwickelt (Hamm CW, Braunwald E. Circulation 2000; 102:118-22). Troponine sind allerdings nicht aktiv an der Pathophysiologie akuter koronarer Syndrome beteiligt, sondern stellen eher eine Art Surrogat-Marker für die fragile Thromusbildung dar (Lindahl B, Diderholm E, Lagerqvist B, Venge P, Wallentin L. J Am Coll Cardio. 2001; 38:979-86, Heeschen C, van Den Brand MJ, Hamm CW, Simoons ML. Circulation 1999; 100:1509-14; Benamer H, Steg PG, Benessiano J, et al.. Am Heart J 1999; 137:815-20). Das EKG hat in dieser Hinsicht nur begrenzte prognostische Relevanz, da bedeutende Abnormitäten selten sind und ihr Nachweis wenig empfindlich und spezifisch ist (Kaul P, Fu Y, Chang WC, et al., J Am Coll Cardiol 2001; 38:64-71 und Savonitto S, Ardissino D, Granger CB, et al. JAMA 1999; 281:707-13).

Marker einer Plättchenaktivierung, welche die Aktivität der Erkrankung bestimmen, möglichst bevor eine Myokardnekrose auftritt, könnten wichtige zusätzliche Informationen für die diagnostische und therapeutische Stratifizierung bei Patienten mit akuten Koronarsyndromen darstellen. In zunehmendem Maße gibt es Beweise dafür, daß auch der CD40-Ligand (CD40L, kürzlich umbenannt in CD154) eine wichtige Rolle bei der Krankheitsentwicklung und der Plaque-Destabilisierung spielt (Mach F, Schonbeck U, Sukhova GK, Atkinson E, Libby P. Nature 1998; 394:200-3 und Lutgens E, Gorelik L, Daemen MJ, et al..Nat Med 1999; 5:1313-6). Das CD40-CD40L-System ist bei einer Vielzahl von Leukozyten und nicht-leukozytischen Zellen, einschließlich Endothelzellen und glatte Muskelzellen (Schonbeck U, Libby P. Cell Mol Life Sci 2001; 58:4-43), sowie aktivierten Plättchen, verbreitet (Henn V, Slupsky JR, Grafe M, et al.. Nature 1998; 391:591-4). Zusätzlich zu der zellassoziierten 39-kDa Form tritt CD40L auch in einer löslichen, biologisch vollständig aktiven Form, nämlich sCD40L, auf (Graf D, Muller S, Korthauer U, van Kooten C, Weise C, Kroczek RA. Eur J Immunol 1995; 25:1749-54). sCD40L wird von stimulierten Lymphozyten abgestoßen und auf Plättchenaktivierung hin aktiv freigesetzt (Lee Y, Lee WH, Lee SC, et al.. Cardiology 1999; 92:11-6 und Henn V, Steinbach S, Buchner K, Presek P, Kroczek RA. Blood 2001; 98:1047-54). sCD40L wirkt pro-inflammatorisch auf Endothelzellen und fördert die Koagulation, indem es die Expression von Gewebefaktoren durch Monozyten (Mach F, Schonbeck U, Bennefoy JY, Pober JS, Libby P. Circulation 1997; 96:396-9) und Endothelzellen (Urbich C, Mallat Z, Tedgui A, Clauss M, Zeiher AM, Dimmeler S. J Clin Invest 2001; 108:1451-8) induziert. Darüber hinaus enthält sCD40L eine KGD-Sequenz (Graf D, Muller S, Korthauer U, van Kooten C, Weise C, Kroczek RA. Eur J Immunol 1995; 25:1749-54), ein bekanntes Bindungsmotiv, das für das vorherrschende Plättchen-Integrin αIIbβ3 spezifisch ist (Scarborough RM, Naughton MA, Teng W, et al.. J Biol Chem 1993; 268:1066-73). Es konnte gezeigt werden, daß CD40L tatsächlich einen αIIbβ3-Liganden, einen Plättchenagonisten, darstellt und für die Stabilität arterieller Thromben notwendig ist (Andre P, Prasad KS, Denis CV, et al.. Nat Med 2002; 8:247-52).

Diese Befunde beweisen zwingend, daß sCD40L eine wichtige Rolle bei der Pathophysiologie akuter Koronarsyndrome spielt. Interessanter weise ist eine Erhöhung von sCD40L im Serum von Patienten mit akuten Koronarsyndromen nachweisbar (Aukrust P, Muller F, Ueland T, et al.. Circulation 1999; 100:614-20). Es wurde berichtet, daß scheinbar gesunde Frauen, die erhöhte Plasmakonzentrationen an sCD40L aufwiesen, ein erhöhtes Risiko für kardiovaskuläre Ereignisse trugen (Schonbeck U, Varo N, Libby P, Buring J, Ridker PM. Circulation 2001; 104: 2266-8).

Entzündungsmarker, welche die Aktivität der Erkrankung bestimmen, möglichst bevor eine Myokardnekrose auftritt, könnten wichtige zusätzliche Informationen für die diagnostische und therapeutische Stratifizierung bei Patienten mit akuten Koronarsyndromen darstellen. Die spezifische therapeutische Inhibierung von Cytokinen, die für die Plaque-Stabilität wesentlich sind, mag eine neue Behandlungsstrategie für Patienten mit instabiler und stabiler koronarer Herzerkrankung sein.

Von Plazenta-Wachstumsfaktor (PIGF), einem Mitglied der vaskulären-Endothel-Wachstumsfaktor-Familie (VEGF-Familie) von Wachstumsfaktoren, wurde kürzlich gezeigt, daß dieser in frühen und fortgeschrittenen arteriosklerotischen Läsionen hoch-reguliert ist.

Die US 6,225,088 beschreibt PIGF im Zusammenhang mit proliferativen Erkrankungen, wohingegen in der WO 92/06194 PIGF als angiogenetischer Faktor beschrieben wird.

De Falco et al. (De Falco S, Gigante B, Persico MG. "Structure and function of placental growth factor" Trends Cardiovasc Med 2002 Aug;12(6):241-6) beschreiben den Zusammenhang beeinträchtigter Angiogenese und Arteriogenese während pathologischer Zustände, wie zum Beispiel Ischämie und Tumorbildung in Mäusen. Dabei wird P1GF als ein wesentlicher Faktor für die Angiogenese unter pathologischen Bedingungen beschrieben. PIGF wird als alternatives Ziel für eine angiogenetische Therapie vorgeschlagen.

Luttun et al. (Luttun A, Tjwa M, Moons L, Wu Y, Angelillo-Scherrer A, Liao F, Nagy JA, Hooper A, Priller J, De Klerck B, Compernolle V, Daci E, Bohlen P, Dewerchin M, Herbert JM, Fava R, Matthys P, Carmeliet G, Collen D, Dvorak HF, Hicklin DJ, Carmeliet P. Revascularization of ischemic tissues by PIGF treatment, and inhibition of tumour angiogenesis, arthritis and atherosclerosis by anti-Flt1 Nat Med 2002 Aug;8(8):831-40) beschreiben therapeutische Verfahren von PIGF und Flt-1 im Rahmen der Angiogenese. Weiterhin wird beschrieben, daß Hemmung von PIGF das Wachstum und die Verletzlichkeit von arteriosklerotischen Plaques verringert.

Oura et al. (Oura H, Bertoncini J, Velasco P, Brown LF, Carmeliet P, Detmar M. A critical role of placental growth factor in the induction of inflammation and edema formation. Blood 2003 Jan 15;101(2):560-7) beschreiben die Rolle von PIGF bei kutaner Entzündung und Angiogenese. Weiterhin werden die Effekte von erhöhten und verringerten Spiegeln von P1GF und deren Vergleich mit dem Status der Entzündung beschrieben.

Das pro-inflammatorische Zytokin CD40L wird aus aktivierten Plättchen freigesetzt. Die lösliche Form von CD40L, nämlich sCD40L, ist in Patienten mit akuten koronaren Syndromen vermehrt vorhanden. Deshalb wurde der prognostische Wert von sCD40L als ein Marker für die Plättchenaktivierung auch im Hinblick auf die therapeutische Wirkung einer Hemmung des Glycoprotein IIb/IIIa-Rezeptors untersucht.

Schwangerschafts-assoziertes Plasmaprotein-A (PAPP-A) ist eine hoch-Molekulargewichts Zink-bindende Matrix-Metalloproteinase, die zu der Metzincin-Superfamilie der Metalloproteinasen gehört und ursprünglich im Plasma schwangerer Frauen identifiziert wurde. Es wird breit für das Screening von fötaler Trisomie im ersten Trimester der Gestation. Kürzlich wurde von PAPP-A auch gefunden, daß es jeweils in erodierten und gelockerten Plaques exprimiert wird, wird jedoch nur minimal in stabilen Plaques exprimiert und von dem ein Wiederauftreten mit Symptomen in Patienten mit ACS vermutet wurde. Jedoch ist die genaue Rolle von zirkulierendem PAPP-A Plasmaspiegeln für die Vorhersage von harten Endpunkten, wie Tod oder Myokardinfarkt in Patienten mit ACS nicht genau bestimmt. Zusätzlich ist vollständig unbekannt, ob PAPP-A Plasmaspiegel zusätzliche prognostische Informationen, verglichen mit kürzlich etablierten Biomarkern in Patienten mit ACS zur Verfügung zu stellen. Daher verglichen die Erfinder die prognostische Signifikanz von PAPP-A Plasmaspiegeln mit Markern systemischer Inflammation, der Plättchenaktivierung, Ischämie und myokardialer Nekrose in Patienten mit ACS.

Im Stand der Technik finden sich viele verschiedene molekulare Marker, die für die Diagnose einer kardiovaskulären Erkrankung geeignet sein können. Beispiele solcher Marker sind unter anderem:

Schwangerschaft assoziiertes Plasma Protein A (PAPP-A); C-reaktives Protein (CRP); hsCRP; plazentaler Wachstumsfaktor (PIGF); Interleukin-18 (IL-18/IL-18b); Hirn natriuretisches Peptid (BNP); NT-pro Hirn natriuretisches Peptid (NT-proNP); sCD40L, cTnI/T, IL-10, ICAM-1, VCAN-1, E-Selektin, P-Selektin, IL-6, VEGF, Serum Amyloid A (SAA), CKMB, MPO, LpPLAz, GP-BB, IL1RA, TAF1, lösliches Fibrin, anti-oxLDL, MCP-1, Gewebefaktor (TF), MMP-9, Ang-2, Tffi-2, IL-P, bFGF, PCM, und VEGF-A.

Einige der oben genannten Marker sind bekannte und charakterisierte Marker für die Untersuchung von koronaren Erkrankungen, andere wurden bisher jedoch noch nicht entsprechend untersucht.

Fast alle angegebenen Marker besitzen einen diagnostischen Wert im Hinblick auf bestimmte kardiovaskuläre Vorfälle. Zum Beispiel ist TnT von besonderem Wert für die Diagnose und die Vorhersage von MI (siehe oben). Entzündliche Marker wie CRP sind für die Diagnose und Vorhersage von einer Entzündung wertvoll, die zu einer Plaque-Ruptur und MI führen kann.

Viele der oben genannten Marker besitzen einen diagnostischen Wert für kardiovaskulären Erkrankungen. Die Verwendung einer Kombination von Markern wurde nur sehr zurückhaltend beschrieben.

Lund et al. in Circulation. 2003,108:1924-1926, beschreiben die Kombination von PAPP-A mit TnI (Troponin I); Peng et al. in Clinica Chimica Acta 319 (2002) 19-26, beschreiben die Kombination von sCD40L mit sICAM-1 und sVCAM-1. Lenderink et al. in European Heart Journal (2003) 24, 77-85, beschreiben die Kombination von TnT mit CRP. Heeschen et al. in Journal of the American College of Cardiology; Vol. 35, No. 6, 2000, erwähnen die Kombination von TnT mit CRP.

Heeschen et al. in Circulation. 2003; 107:2109-2114, beschreiben die Kombination von TnT, CRP und IL-10. Blankenberg et al. in Circulation. 2002; 106:24-30, beschreiben die Kombination von CRP und IL-6. Autiero et al. in Journal of Thrombosis and Haemostasis, 1:1356-1370, beschreiben die Kombination von PIGF und VEGF.

WO 01/0156593 offenbart die Verwendung von Plazenta-Wachstumsfaktor (P1GF) zur Behandlung von akuten kardiovaskulären Erkrankungen.

Jedoch ist das Potential der verbesserten Analyse durch die Kombination bis jetzt weder untersucht, noch wurde die Entwicklung gezielter überlegener Marker-Tests abgeschlossen. Welche Marker sich zu einer effektiven Diagnose eignen, läßt sich somit aus den oben genannten Dokumenten nicht ohne weiteres erschließen.

Im Hinblick auf das oben Genannte ist es somit eine Aufgabe der vorliegenden Erfindung, ein Verfahren zur Verfügung zu stellen, mit dem das Risiko, ein kardiovaskulär nachteiliges Ereignis, bedingt durch Koronarthrombose, mit Hilfe eines individuellen Risikoprofils abgeschätzt werden kann. Aufgabe der vorliegenden Erfindung ist weiterhin, ein Verfahren zur Evaluierung der Wahrscheinlichkeit, daß eine Behandlung mit einem Wirkstoff zur Hemmung des plazentalen Wachstumsfaktors (P1GF) von Vorteil ist, zu entwickeln. Mit Hilfe dieses Verfahren soll der behandelnden Arzt besser als bisher in die Lage versetzt werden, geeignete Maßnahmen auswählen zu können, um den Patienten positiv zu beeinflussen und/oder ein nachteiliges Ereignis zu verhindern oder zumindest in seiner Schwere für den betroffenen Patienten zu vermindern.

Aufgabe der vorliegenden Erfindung ist, ein Verfahren zu entwickeln, mit dem das Risiko, ein kardiovaskulär nachteiliges Ereignis, bedingt durch Koronarthrombose mit Hilfe eines individuellen Risikoprofils abgeschätzt werden kann. Dies soll durch die Messung der Konzentration eines Markers der Plättchenaktivierung erfolgen. Aufgabe der vorliegenden Erfindung ist weiterhin, ein Verfahren zur Evaluierung der Wahrscheinlichkeit, daß eine Behandlung mit einem Wirkstoff zur Hemmung der Plättchenaktivierung von Vorteil ist, zu entwickeln. Mit Hilfe dieses Verfahren soll der behandelnden Arzt besser als bisher in die Lage versetzt werden, geeignete Maßnahmen auswählen zu können, um den Patienten positiv zu beeinflussen, ein nachteiliges Ereignis zu verhindern oder zumindest in seiner Schwere für den betroffenen Patienten zu vermindern.

Es ist eine weitere Aufgabe der vorliegenden Erfindung, allgemeine Kombinationen von diagnostischen Marker für kardiovaskulären Ereignisse zu finden, die eine präzise Diagnose ermöglichen, an welchem kardiovaskulären Ereignis der Patient bereits gelitten hat und an welchem er möglicherweise in der Zukunft erkranken wird. Insbesondere ist es eine Aufgabe Markerkombination zu finden, die günstiger weise parallel durchgeführt werden können. Idealer weise würden diese Messungen gemeinsam mit TnT Messungen von Patienten mit Brustschmerzen ermöglichen.

Die Aufgabe der vorliegenden Erfindung wird durch ein Verfahren zur Analyse von Proben in Zusammenhang mit akuten kardiovaskulären Erkrankungen gelöst. Das erfindungsgemäße Verfahren umfaßt die Schritte aus: (a) zur Verfügung stellen einer zu untersuchenden biologischen Probe aus einem Subjekt, (b) Bestimmen der Konzentration des Markers PIGF in der Probe, (c) gegebenenfalls, Bestimmen der Konzentration mindestens eines weiteren Markers ausgewählt aus löslichem CD40-Ligand (sCD40L), PAPP-A, Troponin T (TnT), MPO, NT-proBNP, VEGF, BNP und weiteren entzündlichen Markern, und (d) Vergleichen des/der für die zu untersuchende Probe erhaltenen Ergebnisse/s mit Referenzwerten und/oder den Werten aus Referenzproben.

Die zur Analyse im Rahmen der vorliegenden Erfindung verwendeten Marker sind zum größten Teil aus dem Stand der Technik gut bekannte und charakterisierte Marker, die jedoch bisher immer noch unzureichend für die Verwendung zur Diagnose einer akuten kardiovaskulären Erkrankung charakterisiert waren.

So sind das C-reaktive Protein (CRP) und hsCRP als Marker der systemischen Inflammation, Troponin cTnI/T als Marker für Nekrose; das Schwangerschaft assoziierte Plasmaprotein A (PAPP-A) als Marker für Makrophagen Aktivierung; IL-10 (Interleukin 10) als Marker für die inflammatorische Balance, sCD40L als Marker für die Thrombo-inflammatorische Aktivierung, MPO (Myeloperoxidase) als Marker für oxidativen Streß, der plazentale Wachstumsfaktor (P1GF) als Marker für Vaskuläre Inflammation und die Marker Hirn natriuretisches Peptid (BNP) und NT-pro Hirn natriuretisches Peptid (NT-proNP) als Marker neurohumoraler Aktivierung und der Ischämie charakterisiert.

Andere ähnliche und auch verwendbare Marker sind Interleukin-18 (IL-18/IL-18b), ICAM-1, VCAN-1, E-Selektin, P-Selektin, IL-6, VEGF, Serum Amyloid A (SAA), CKMB, LpPLAz, GP-BB, IL-1RA, TAF-1, lösliches Fibrin, anti-oxLDL, MCP-1, Gewebefaktor (TF), MMP-9, Ang-2, Tffi-2, bFGF, PCM und VEGF-A.

Die WO 03/040692 (und Circulation 2001; 104:2266-2268) beschreibt, daß sCD40L vorteilhaft in Kombination mit einem inflammatorischen Marker, insbesondere CRP verwendet werden kann. Diese Kombination wird jedoch ausschließlich für die Diagnose nicht-akuter kardialer Erkrankungen verwendet, eine Stratifizierung wird von der WO 03/040692 aufgrund unsicherer Ergebnisse abgelehnt.

Bevorzugt ist ein erfindungsgemäßes Verfahren, wobei die zu untersuchende Probe und/oder die Referenzprobe aus einem Säugetier, insbesondere aus dem Menschen, stammen. Weiter bevorzugt ist ein erfindungsgemäßes Verfahren, wobei die zu untersuchende Probe und/oder die Referenzprobe ausgewählt ist aus der Gruppe bestehend aus peripherem Blut oder Fraktionen davon und Zellkultur-Suspensionen oder Fraktionen davon. Bevorzugt ist weiterhin, daß die untersuchende Probe und/oder Referenzprobe Blutserum oder Blutplasma ist. Besonders bevorzugt ist peripheres Vollblut als zu untersuchende Probe und/oder Referenzprobe.

Gemäß einem weiteren Aspekt des erfindungsgemäßen Verfahrens kann die zu untersuchende Probe und/oder die Referenzprobe vorbehandelt sein, wobei dem peripheren Blut z.B. ein Gerinnungshemmer, insbesondere Heparin, zugesetzt wird.

Erfindungsgemäß konnte überraschenderweise gefunden werden, daß neben der Einzelanalyse des Markers P1GF zur Diagnose oder der Verfolgung akuter kardialer Ereignisse auch Kombinationen von solchen Markern möglich sind, die eine erheblich verbesserte Analyse ermöglichen. Als besonders bevorzugt haben sich dabei Kombinationen von Markern erwiesen, die verschiedene Aspekte des nachteiligen kardialen Ereignisses betreffen, sich aber gleichzeitig (also simultan oder in einer mehr oder weniger zeitlich beanstandeten Meßreihe) analysieren lassen. In einem Aspekt dieser Erfindung sind diese weiteren Marker aus entzündlichen Marker ausgewählt, so zum Beispiel aus CRP, (hs)CRP und IL-10.

Gemäß einem weiteren Aspekt des erfindungsgemäßen Verfahrens sind die analysierten Marker und Kombinationen daraus ausgewählt aus P1GF; P1GF + TnT; sCD40L + P1GF; PAPP-A + P1GF; sCD40L + P1GF + TnT; PAPP-A + P1GF + TnT; sCD40L + PAPP-A + P1GF; und sCD40L + PAPP-A + P1GF + TnT. Bevorzugt ist dann die weitere Kombination mit mindestens einem weiteren der Marker MPO, NT-proBNP, BNP, CRP, (hs)CRP und IL-10.

Das erfindungsgemäß verwendete Verfahren zum Bestimmen der Konzentration der analysierten Marker kann aus allen dem Fachmann zum Nachweis von Proteinen in biologischen Proben bekannten geeigneten Verfahren ausgewählt sein. Das spezifische Verfahren ist im Rahmen der vorliegenden Erfindung solange nicht wichtig, wie das Verfahren empfindlich genug ist, um die für eine genaue Bestimmung der Konzentrationen der Marker erforderliche Nachweisgrenze zu unterschreiten. Geeignete Verfahren beruhen normalerweise auf der Bindung einer Markierung an den nachzuweisenden Marker und den anschließenden Nachweis dieser Markierung. Die Bindung kann dabei kovalent oder nicht-kovalent sein und/oder direkt oder indirekt erfolgen. Geeignete Messverfahren gemäß der vorliegenden Erfindung schließen z. B. die Elektrochemilumineszenz ein. Turbidimetrie, Nephelometrie und Latex-verstärkte Turbidimetrie oder Nephelometrie können ebenfalls verwendet werden.

Auf Grund ihrer hohen Sensitivität und der Tatsache, daß sich diese Verfahren auch auf Hoch-Durchsatz-Umgebungen anpassen lassen, sind erfindungsgemäß Verfahren bevorzugt, wobei das Bestimmen der Konzentration mittels eines immunologischen Verfahrens mittels an die Marker bindenden Moleküle erfolgt. Beispiele für solche Verfahren sind ELISA (enzyme-linked immunosorbent assay), Sandwich Enzymimmuntests oder Festphasen Immuntests. Bevorzugt ist somit, daß die an die Marker bindenden Moleküle ausgewählt sind aus der Gruppe bestehend aus anti-Marker-Antikörpern oder Teilen davon und Marker-Rezeptoren oder Teilen davon.

Diese Moleküle können aus einer sehr großen Vielzahl von für die Marker spezifischen Molekülen ausgewählt sein. Bevorzugt ist, daß die an die Marker bindenden Moleküle ausgewählt sind aus der Gruppe bestehend aus Antikörpern, die spezifisch gegen Marker oder gegen Teile davon gerichtet sind, oder Teilen oder Fragmenten davon und einem Marker-Rezeptor oder Teilen davon oder einem Integrin, z.B. dem Plättchen-Integrin αIIbβ3, oder Teilen davon. Besonders bevorzugt ist ein erfindungsgemäßes Verfahren, wobei die Antikörper, Teile oder Fragmente davon polyklonale Antikörpern, monoklonale Antikörpern, Fab-Fragmente, scFv-Antikörper und Diabodies umfassen.

Gemäß eines weiteren Aspekts des Verfahrens der vorliegenden Erfindung können Komponenten des Verfahrens an eine feste Phase gebunden vorliegen, somit können die an einen Marker bindenden Moleküle in Lösung oder Matrix-immobilisiert vorliegen. Als Matrices können eine Vielzahl von dem Fachmann bekannten Materialien verwendet werden, wie zum Beispiel Harz-Matrices und/oder herkömmliche Säulenmatrices. Besonders bevorzugt ist weiterhin ein erfindungsgemäßes Verfahren, bei dem die an einen Marker bindenden Moleküle an eines oder mehrere Detektionsmoleküle aus der Gruppe bestehend aus Fluoresceinthioisocyanat, Phycoerythrin, Enzymen (zum Beispiel Meerrettich-Peroxidase) und magnetisches Bead gekoppelt sind.

Gemäß einem weiteren Aspekt des erfindungsgemäßen Verfahrens können die an die Marker bindenden Moleküle mit einem Antikörper, an den eine oder mehrere Detektionsmoleküle gekoppelt sind, nachgewiesen werden. Es handelt sich somit um einen indirekten Nachweis der Bindung des Moleküls. Solche zwei-stufigen Nachweise sind dem Fachmann zum Beispiel aus der anti-Antikörper-Nachweistechnik bestens bekannt.

Gemäß einem weiteren Aspekt des Verfahrens gemäß der vorliegenden Erfindung können zur Analyse der Probe immunzytologischen Verfahren angewandt werden. Dabei sind alle Verfahren geeignet, die eine spezifische Bestimmung anhand der Marker/Molekül-Interaktion erlauben. Bevorzugt sind Verfahren, die ausgewählt sind aus der Gruppe bestehend aus Sandwich-Enzym-Immuntest, ELISA und Festphasen-Immuntests.

Die für die zu untersuchende Probe ermittelten Ergebnisse werden üblicherweise mit einer Referenzprobe verglichen. Welche Probe als Referenzprobe dienen kann, wird insbesondere von der Art der untersuchten Probe und der Krankengeschichte des Individuums, aus dem die zu untersuchende Probe stammt, abhängen. Bevorzugt ist ein erfindungsgemäßes Verfahren, bei dem die Referenzprobe aus einem oder dem Mittelwert mehrerer Säugetiere stammt, in dem/in denen eine kardiovaskuläre Erkrankung ausgeschlossen wurde. Dies muß aber nicht zwangsläufig so sein, wenn z.B. das Fortschreiten einer Erkrankung bestimmt werden soll, kann auch eine "alte" Probe desselben Patienten als Referenzprobe verwendet werden. Dem Fachmann ist offensichtlich, welche Proben als Referenzprobe für das erfindungsgemäße Verfahren geeignet sind.

Gemäß einem weiteren Aspekt des Verfahrens gemäß der vorliegenden Erfindung können die zu diagnostizierenden und/oder prognostizierenden und/oder deren Therapie zu überwachenden akuten kardiovaskulären Erkrankungen ausgewählt sein aus der Gruppe bestehend aus instabiler Angina, Myokardinfarkt, akutem Herzsyndrom, koronarer Arterienerkrankung und Herzinsuffizienz. Es soll jedoch nicht ausgeschlossen werden, daß sich das erfindungsgemäße Verfahren noch für weitere akute kardiologische Erkrankungszustände eignet und anwenden läßt.

Ein weiterer Aspekt der vorliegenden Erfindung betrifft die Verwendung eines diagnostischen Kits, wobei der Kit Mittel zur Durchführung des erfindungsgemäßen Verfahrens, weitere Komponenten und/oder Hilfsstoffe umfaßt. Solche Mittel sind ein Antikörper zum Nachweis von Marker und Mittel zur anschließenden Quantifizierung der Marker. Der Kit kann außerdem andere Komponenten und/oder Enzyme zur Durchführung der Verfahren der vorliegenden Erfindung, z.B. Gebrauchsanweisungen zur Interpretation der Ergebnisse des Tests im Hinblick auf das Risikoprofil des Patienten und entsprechende Gegenmaßnahmen und Therapievorschläge enthalten.

Bevorzugt ist, das erfindungsgemäßes Verfahren mit Hilfe eines einen diagnostischer Kits durchzuführen, der goldmarkierte polyklonale Maus-Indikatorantikörper, biotinylierte polyklonale Nachweisantikörper und eine Testvorrichtung, umfassend ein Fiberglas-Vlies, umfaßt.

Ein weiterer Aspekt der vorliegenden Erfindung betrifft somit die Verwendung des erfindungsgemäßen Verfahrens zur Diagnose und/oder Prognose akuter kardiovaskulärer Erkrankungen und/oder zur Überwachung von deren Therapie. Dies geschieht durch die quantitative und kritische Bestimmung von Markern. Aufgrund des daraufhin erstellbaren Risiokoprofils können dann geeignete Gegenmaßnahmen durch den behandelnden Arzt durchgeführt werden, um den Patienten positiv zu beeinflussen und das nachteilige Ereignis zu verhindern oder zumindest in seiner Schwere für den betroffenen Patienten zu vermindern. Eine solche Therapie kann erfindungsgemäß z.B. die Verabreichung von Statinen oder Inhibitoren des Glycoprotein IIb/III-Rezeptors insbesondere Abciximab umfassen. Dem Fachmann sind jedoch weitere mögliche Therapien bekannt, um kardiovaskuläre Erkrankungen zu therapieren, die nach herkömmlichen Schemata erfolgen können.

In einer weiteren Ausführungsform der Erfindung wird ein anti-inflammatorisches Mittel coverabreicht. Dieses Mittel kamen ausgewählt sein aus nicht-Steroid oder Steroid anti-inflammatorischen Mitteln, die z. B. einschließen können: Alclofenac; Alclometason; Dipropionat; Algestonacetonide; Alpha-Amylase; Amcinafal; Amcinafid; Amfenac Natrium; Amiprilosehydrochlorid; Anakinra; Anirolac; Anitrazafen; Apazon; Balsalazid Dinatrium; Bendazac; Benoxaprofen; Benzydamin Hydrochlorid; Bromelain; Broperamol; Budesonid; Carprofen; Cicloprofen; Cintazon; Cliprofen; Clobetasolpropionat; Clobetasonbutyrat; Clopirac; Cloticasonpropionat; Cormethasonacetat; Cortodoxon; Deflazacort; Desonid; Desoximetason; Dexamethasondipropionat; Diclofenac Kalium; Diclofenac Natrium; Diflorasondiacetat; Diflunudon Natrium; Diflunisal; Difluprednat; Diftalon; Dimethylsulfoxid; Drocinonid; Endryson; Enlimomab; Enolicam Natrium; Epirizol; Etodolac; Etofenamat; Felbinac; Fenamol; Fenbufen; Fenclofenac; Fenclorac; Fendosal; Fenpipalon; Fentiazac; Flazalon; Fluazacort; Flufenaminsäure; Flumizol; Runisolidacetat; Plunixin; Flunixin Meglumin; Fluocortin Butyl; Fluormetholonacetat; Fluquazon; Flurbiprofen; Fluretofen; Fluticasonpropionat; Puraprofen; Furobufen; Halcinonid; Halobetasolpropionat; Halopredonacetat; Ibufenac; Ibuprofen; Ibuprofen Aluminium; Ibuprofen Piconol; Ilonidap; Indomethacin; Indomethacin Natrium; Indoprofen; Indoxol; Mitrazol; Isoflupredonacetat; Isoxepac; Isoxicam; Ketoprofen; Lofemizol Hydrochlorid; Lomoxicam; Loteprednol Etabonat; Meclofenamat Natrium; Meclofenaminsäure; Meclorison Dibutyrat; Mefenaminsäure; Mesalamin; Meseclazon; Methylprednisolon Suleptanat; Momiflumat; Nabumeton; Naproxen; Naproxen Natrium; Naproxol; Nimazon; Olsalazin Natrium; Orgotein; Orpanoxin; Oxaprozin; Oxyphenbutazon; Paranylin Hydrochlorid; Pentosan Polysulfat Natrium; Phenbutazon Natriumglycerat; Pirfenidon; Piroxicam; Piroxicam Cinnamat; Piroxicam Olamin; Pirprofen; Prednazat; Prifelon; Prodolinsäure; Proquazon; Proxazol; Proxazolcitrat; Rimexolon; Romazarit; Salcolex; Salnacedin; Salsalat; Salicylate; Sanguinariumchlorid; Seclazon; Sermetacin; Sudoxicam; Sulindac; Suprofen; Talmetacin; Talniflumat; Talosalat; Tebufelon; Tenidap; Tenidap Natrium; Tenoxicam; Tesicam; Tesimid; Tetrydamin; Tiopinac; Tixocortol Pivalat; Tolmetin; Tolmetin Natrium; Triclonid; Triflumidat; Zidometacin; Glucocorticoide; Zomepirac Natrium.

Im Kontext der vorliegenden Erfindung betrifft "Diagnose" die Überprüfung, ob ein Individuum an einem bestimmten kardiovaskulären Ereignis gelitten hat. Im Kontext der vorliegenden Erfindung betrifft "Prognose" die Vorhersage der Wahrscheinlichkeit (in %) ob ein Individuum an einem bestimmten kardiovaskulären Ereignis leiden wird. Im Kontext der vorliegenden Erfindung betrifft "Stratifizierung der Therapie" die Ermittlung der geeigneten therapeutischen Behandlung für das kardiovaskuläre Ereignis, das auftreten wird oder aufgetreten ist. Im Kontext der vorliegenden Erfindung betrifft "Überwachung der Therapie" die Kontrolle und, gegebenenfalls, ein Einstellen der therapeutischen Behandlung eines Individuums. Im Kontext der vorliegenden Erfindung betrifft "Therapeutische Behandlung" schließt jede Behandlung ein, die möglicherweise den pathophysiologischen Zustand eines Individuums und schließt z.B. die Verabreichung von Pharmazeutika sowie chirurgische Behandlung (z.B. Ballondilatation).

*Base. Line-Werte* der sCD40L-Konzentration machen Informationen von prognostischem Wert bei Patienten mit akuten koronaren Syndromen unabhängig vom Auftreten einer Myokardnekrose verfügbar. Darüber hinaus läßt sich mittels sCD40L die Hochrisikogruppe von Patienten, welche den größten Nutzen aus einer Anti-Plättchen-Behandlung mit Abciximab ziehen können, identifizieren.

Die vorliegende Studie liefert direkte Hinweise darauf, daß sCD40L ein aussagekräftiger biochemischer Marker einer Plättchen-Aktivierung ist. Eine Erhöhung der Konzentration an CD40L identifiziert zuverlässig eine bestimmte Untergruppe von Patienten mit akuten Koronarsyndromen, die ein hochgradiges Risiko dafür tragen, ein kardiales Ereignis zu erleiden, und die den größten Nutzen von einer Behandlung mit dem Glycoprotein IIb/IIIa-Rezeptor-Antagonisten Abciximab haben. Dementsprechend trägt CD40L nicht nur erheblich zu der Pathophysiologie akuter Koronarsyndrome bei, sondern stellt einen zuverlässigen und aussagekräftigen klinischen Marker zur Verfügung, mit dessen Hilfe Patienten mit einer hoch risikoreichen Läsionsbildung und/oder koronaren Thrombose identifiziert werden kann (Andre P, Prasad KS, Denis CV, et al., Nat Med 2002; 8:247-52; Andre P., Nannizzi-Alaimo L, Prasad SK, Phillips DR.; Circulation 2002; 106:896-9). '

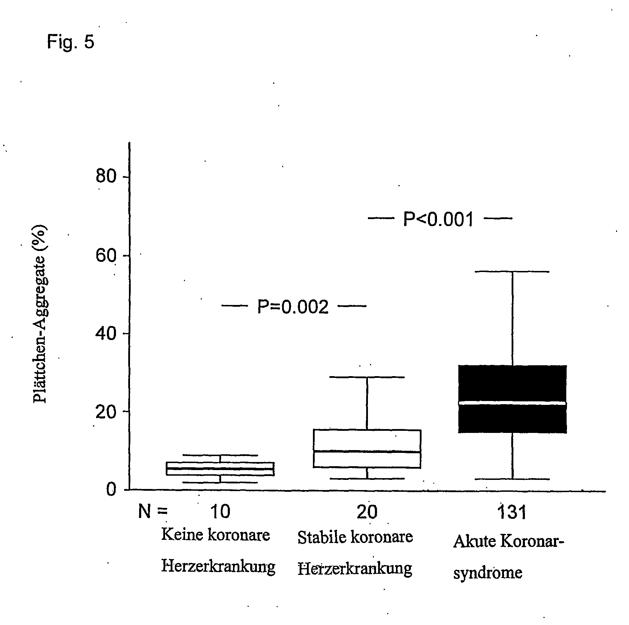

Bei 40,5% der CAPTURE-Patienten lagen die sCD40L-Konzentrationen in der Zirkulation oberhalb der berechneten Schwellenkonzentration von 5,0 µg/l. Diese Patienten mit erhöhten sCD40L-Konzentrationen trugen ein hochgradiges kardiales Risiko, einen tödlichen oder nicht-tödlichen Myokardinfarkt zu erleiden. Dieses gesteigerte kardiale Risiko bei Patienten mit hohen sCD40L-Konzentrationen, die ein Placebo erhielten, war in besonderem Maße während der ersten 72 Stunden offensichtlich *(Figur 5a).* Allerdings entwickelte sich die Häufigkeit der Ereignisse während der gesamten sechs Monate immer weiter auseinander *(Figur 5b).* sCD40L erwies sich als ein aussagekräftiger prognostischer Marker, der unabhängig vom Nachweis einer Myokardnekrose und unabhängig von den Entzündungsmarkern CRP und TNF-α ebenso wie von dem Adhäsionsmolekül ICAM-1 ist. In einem Multivarianz-Regressionsmodell lieferten TnT, CRP und sCD40L zuverlässige prognostische Informationen *(Tabelle* 2). Mit Hilfe der sCD40L-Konzentration wurden Patienten ohne Anzeichen einer Myokardnekrose identifiziert, die ein erhöhtes kardiales Risiko aufwiesen. Unter Verwendung festgesetzter Schwellenkonzentrationen für TnT und CRP sowie einer Einteilung der Patienten anhand der Anzahl kardialer Markern, die erhöht waren, wurde festgestellt, daß eine simultane Abschätzung dieser drei pathobiologisch unterschiedlichen biochemischen Markern zum Zeitpunkt, zu dem der Patient vorstellig wird, eine aussagekräftige Vorhersage des Risikos des Patienten, nachteilige kardiale Ereignisse während der nachfolgenden sechs Monate zu erleiden, ermöglichte.

Troponine stellen Marker einer Myokardnekrose dar, sind jedoch nicht aktiv in die Pathophysiologie akuter Koronarsyndrome involviert. Sie sind eher Surrogat-Marker für die fragile Thrombus-Bildung (Lindahl B, Diderholm E, Lagerqvist B, Venge P, Wallentin L; J Am Coll Cardiol 2001; 38:979-86; Heeschen C, van Den Brand MJ, Hamm CW, Simoons ML; Circulation 1999; 100:1509-14; Benamer H, Steg PG, Benessiano J, et al.; Am Heart J 1999; 137:815-20). Bei *post mortem-*Studien an Patienten mit akuten Koronarsyndromen wurde eine Erosion oder Ruptur der fibrösen Käppchen der atherosklerotischen Plaques, die der Pathophysiologie zugrunde liegen, identifiziert (Lindahl B, Diderholm E, Lagerqvist B, Venge P, Wallentin L; J Am Coll Cardiol 2001; 38:979-86; Heeschen C, van Den Brand MJ, Hamm CW, Simoons ML; Circulation 1999; 100:1509-14). Eine Exposition von Bestandteilen der Plaques, Kollagen und anderen Komponenten der Gefäßwand führt zu einer Zunahme des Gefäßtonus und der Plättchen-Aktivierung (Farb A, Burke AP, Tang AL, et al.; Circulation 1996; 93:1354-63; Davies MJ, Thomas AC; Br Heart J 1985; 53:363-73; Davies MJ; N. Engl J Med 1997; 336:1312-4). Die Thromboembolie einer Koronararterie mit gesteigerter mikrovaskulärer Perfusion und Nekrose ist ein wesentlicher Bestandteil von akuten Koronarsyndromen (Heeschen C, van Den Brand MJ, Hamm CW, Simoons ML; Circulation 1999; 100:1509-14; Benamer H, Steg PG, Benessiano J, et al.; Am Heart J 1999; 137:815-20). Dementsprechend dienen empfindliche Marker für den Nachweis einer kleineren Verletzung des Myokards, insbesondere Troponine, als Surrogat-Marker einer arteriellen Thromboembolie, die auf einen aktiven thrombotischen Prozeß in der auslösenden Läsion zurückzuführen ist.

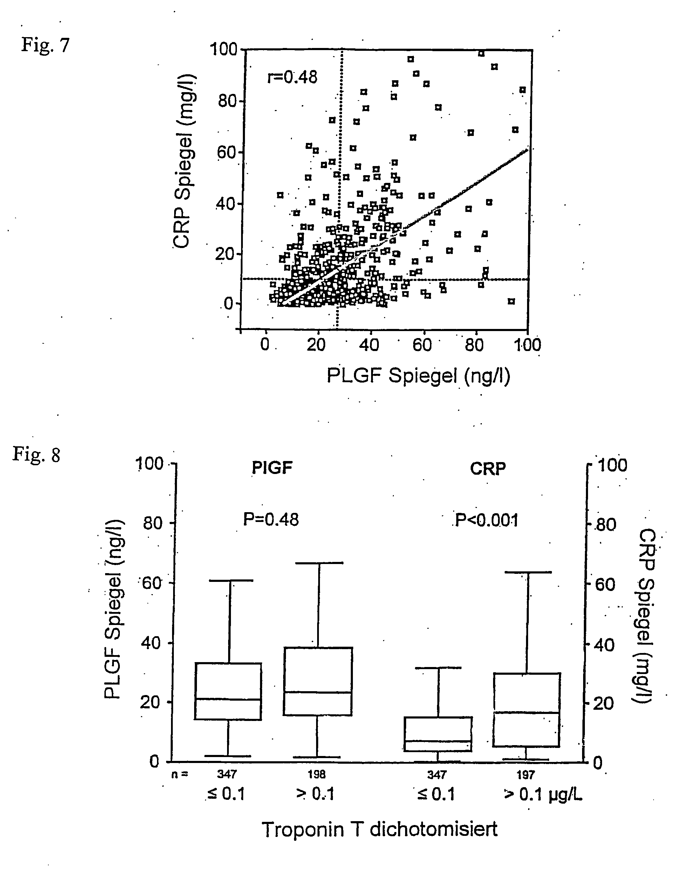

Im Gegensatz dazu könnte sCD40L in mehrfacher Weise direkt in die Pathophysiologie akuter Koronarsyndrome involviert sein. Jüngste Hinweise legen nahe, daß CD40L wesentlich zur Progression einer Atherosklerose und dementsprechend zu einer Destabilisierung von atherosklerotischen Plaques beiträgt (Mach F, Schonbeck U, Sukhova GK, Atkinson E, Libby P; Nature 1998; 394:200-3; Lutgens E, Gorelik L, Daemen MJ, et al.; Nat Med 1999; 5:1313-6). Es wurde vorgeschlagen, daß CD40/CD40L-Interaktionen Komplikationen durch Atherome fördern, indem sie die Expression von Zytokinen, Chemokinen, Wachstumsfaktoren, Matrix-Metalloproteinasen und Prokoagulantien in verschiedenen Atherom-assozüerten Zelltypen induziert (Schonbeck U, Libby P.; Cell Mol Life Sci 2001; 58:4-43; Henn V, Slupsky JR, Grafe M, et al.; Nature 1998; 391:591-4; Henn V, Steinbach S, Buchner K, Presek P, Kroczek RA; Blood 2001; 98:1047-54; Mach F, Schonbeck U, Bonnefoy JY, Pober JS, Libby P.; Circulation 1997; 96:396-9; Miller DL; Yaron R, Yellin MJ; J Leukoc Biol 1998; 63-373-9; Kotowicz K, Dixon GL, Klein NJ, Peters MJ, Callard RE; Immunology 2000; 100:441-8). Neuere Studien haben gezeigt, daß neben Leukozyten und nicht-leukozytischen Zellen einschließlich Granulozyten, mononukleäre Phagozyten, Endothelzellen und Zellen der glatten Muskulatur (Schonbeck U, Libby P; Cell Mol Life Sci 2001; 58:4-43) aktivierte Plättchen große Mengen an sCD40L produzieren und freisetzen (Henn V, Steinbach S, Buchner K, Presek P, Kroczek RA; Blood 2001; 98:1047-54). Eine andere Studie zeigt, daß ein Kardiopul*monar-Bypass* einen Anstieg der Konzentration von sCD40L im Plasma verursacht, der mit einer Abnahme des CD40L-Gehalts in Plättchen korrespondiert, was nahe legt, daß sCD40L primär aus den Plättchen stammt und zu den thrombotischen Komplikationen, die mit einem derartigen *Bypass* verbunden sind, beitragen könnte (Nannizzi-Alaimo L, Rubenstein MH, Alves VL, Leong GY, Phillips DR, Gold HK; Circulation 2002, 105:2849-2854). Auch wurde festgestellt, daß sCD40L positiv mit löslichem P-Selektin im Plasma und 11-Dehydro-Thromboxan B₂-Konzentrationen im Urin korreliert (Cipollone F, Mezzetti A, Porreca E, et al.; Circulation 2002; 106:399-402). Darüber hinaus zeigten experimentelle Untersuchungen, daß CD40L für eine Stabilisierung arterieller Thromben erforderlich ist (Andre P, Prasad KS, Denis CV et al.; Nat Med 2002; 8:247-52). Die vorliegende Studie liefert jetzt direkten Hinweis darauf, daß CD40L tatsächlich ein Marker der Plättchen-Aktivierung ist. Die Plättchen-Aktivierung, bestimmt durch Durchflußzytometrie bei Patienten mit akuten Koronarsyndromen, korrelierte signifikant mit den Serumkonzentrationen an sCD40L *(**Figur 7*). Interessanter weise erwies sich sCD40L als unabhängiger Vorhersagefaktor einer Plättchen-Aktivierung mit größter Aussagekraft. Die Ergebnisse der vorliegenden Studie etablieren sCD40L-Konzentrationen als hoch informativen prognostischen Marker bei Patienten mit akuten Koronarsyndromen, die gefährdet sind, eine Thrombose zu erleiden. Diese Befunde werden durch die Tatsache unterstützt, daß eine Hemmung des Glycoprotein IIb/IIIa-Rezeptors durch Abciximab das erhöhte Risiko bei Patienten mit akuten Koronarsyndromen und erhöhten sCD40L-Konzentrationen aufhob. Während Troponin die Neigung eines Thrombus, eine Embolie hervorzurufen und zu Myokardnekrose zu führen, positiv anzeigt, spiegeln erhöhte Konzentrationen an sCD40L bei Patienten mit akuten Koronarsyndromen die thrombotische Aktivität der auslösenden Läsion, Plättchen zu rekrutieren und aktivieren, wider.

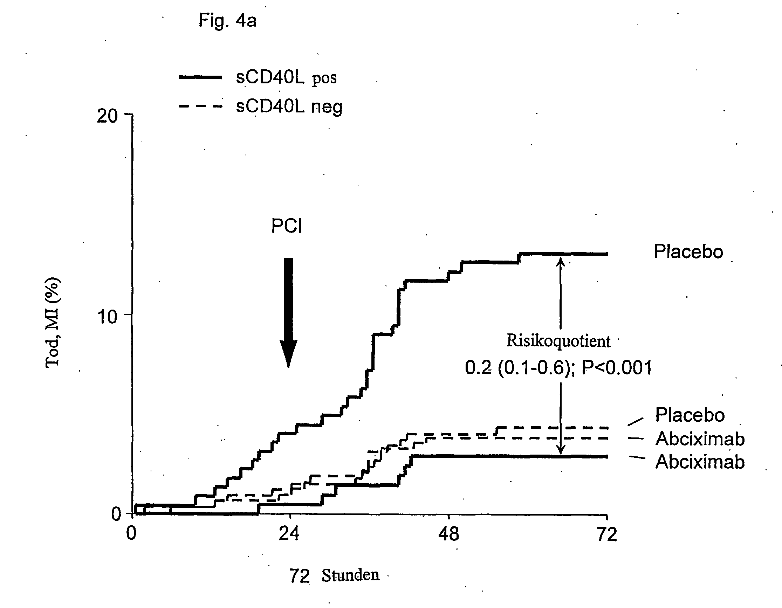

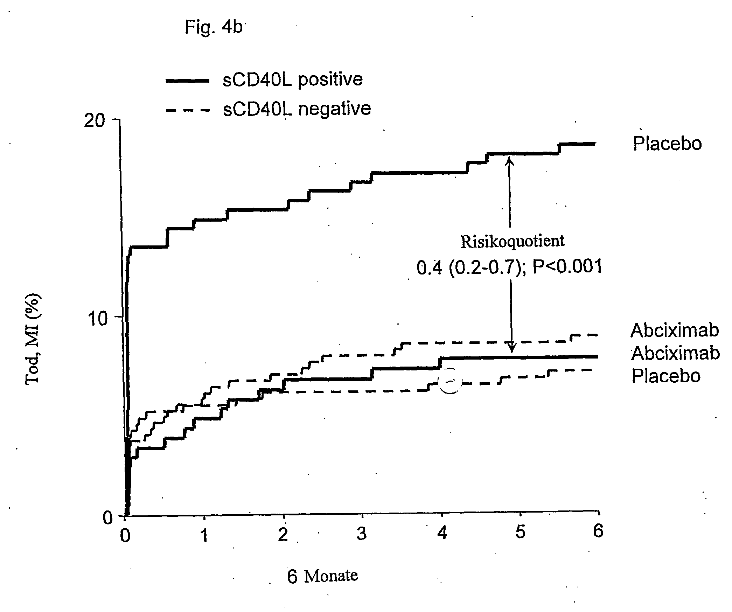

In einer früheren Analyse einer Untergruppe von Patienten der CAPTURE-Studie wurde gezeigt, daß eine zusätzliche Behandlung mit dem Glycoprotein IIb/IIIa-Rezeptor-Antagonisten Abciximab das erhöhte Risiko Troponin-positiver Patienten auf das Maß von Troponin-negativen Patienten reduziert (Hamm CW, Heeschen C, Goldmann B, et al.; N Engl J Med 1999; 340:1623-9). Diese Patienten stellen ungefähr 1/3 der Patienten mit akuten Koronarsyndromen dar (Hamm CW, Braunwald E; Circulation 2000; 102:118-22; Antman EM, Tanasijevic MJ, Thompson B, et al; N Engl J Med 1996; 335:1342-9; Ohman EM, Armstrong PW, Christenson RH, et al.; N Engl J Med 1996; 335:1333-41; Hamm CW, Ravkilde J, Gerhardt W, et al.; N Engl J Med 1992; 327:146-S0; Hamm CW, Goldmann BU, Heeschen C, Kreymann G, Berger J, Meinertz T; N Engl J Med 1997; 337:1648-53). Ähnliche Befunden hinsichtlich Troponin T und Troponin I ergaben sich später aus anderen Studien (Newby LK, Ohman EM, Christenson RH, et al.; Circulation 2001; 103:2891-6; Januzzi JL, Chae CU, Sabatine MS, Jang IK; J Thromb Thrombolysis 2001; 11:211-5; Heeschen C, Hamm CW, Goldmann B, Deu A, Langenbrink L, White HD; Lancet 1999; 354:1757-62), und anschließend wurden Troponine in die neuen Richtlinien aufgenommen als Teil der Risikostratifizierung bei Patienten mit akuten Koronarsyndromen (Hamm CW, Bertrand M, Braundwald E; Lancet 2001; 358:1533-8; Braunwald E, Maseri A, Armstrong PW, et al.; Eur Heart J 1998; 19:D22-30). In der vorliegenden Studie wird gezeigt, daß ein derartiger ausgeprägt positiver Effekt einer Anti-Plättchen-Therapie auch bei Patienten mit erhöhten sCD40L-Konzentrationen offenkundig ist. Die vorliegenden Analyse legt nahe, daß Patienten mit akutem Koronarsyndromen, die erhöhte Konzentrationen an sCD40L aufweisen, wirksam durch den Glycoprotein IIb/IIIa-Rezeptor-Antagonisten Abciximab stabilisiert werden (*Figur 5a, b*). Bei einer berechneten Schwellenkonzentration von 5,0 µg/l wurde eine abrupte Änderung des Risikoquotienten von 0,87 für das zweite Quintil und 1,12 für das dritte Quintil hin zu signifikant niedrigeren Werten von 0,36 für das vierte Quintil und 0,38 für das fünfte Quintil beobachtet *(**Figur* 4). Interessanter weise lieferten die Konzentrationen an TnT und sCD40L unabhängige Vorhersagewerte im Hinblick sowohl auf das Risiko ischämischer Ereignisse als auch auf die positive Wirkung einer Glycoprotein IIb/IIIa-Rezeptor-Hemmung durch Abciximab. Patienten ohne Anzeichen einer Myokardverletzung (Troponin-Erhöhung fehlt), die jedoch gesteigerte Konzentrationen an sCD40L aufwiesen, zogen erheblichen Nutzen aus der Behandlung mit dem Glycoprotein IIb/IIIa-Inhibitor Abciximab. Folglich zogen Patienten mit hohem Risiko zu einer Thrombose der Koronargefäße, belegt entweder durch eine Erhöhung der sCD40L-Konzentration oder einer Erhöhung der TnT-Konzentration, die schließlich 54% der insgesamt in die CAPTURE-Studie involvierten Patienten ausmachten, einen deutlichen Vorteil aus der Behandlung mit Abciximab mit einem Risikoquotienten von 0,38 [0,21-0,72]; p < 0,001).

Zusammenfassend läßt sich sagen, daß die vorliegende Studie die bedeutende und unabhängige Rolle von sCD40L als einen Marker der Plättchenaktivierung für die diagnostische und therapeutische Risikostratifizierung dokumentiert. Das erhöhte kardiale Risiko von Patienten mit hohen sCD40L-Konzentrationen, die eine Standardtherapie mit Heparin und Aspirin erhielten, wurde durch den Glycoprotein IIb/IIIa-Rezeptorantagonisten Abciximab aufgehoben. Die kombinierte Verwendung von Troponinen und sCD40L, welche beide wesentliche Bestandteile der Pathophysiologie bei Patienten mit akuten Koronarsyndromen darstellen, liefert wichtige Einblicke in die Aktivität der Erkrankung, dem kardiologischen Risiko und der Wirksamkeit einer Behandlung mittels Glycoprotein IIb/IIIa-Hemmung durch Abciximab, welche der Verwendung eines einzelnen Markers überlegen ist. Die Verwendung von sCD40L als einziger Marker oder in Kombination mit anderen Markern ohne eine Verwendung von PIGF ist nicht Gegenstand der vorliegenden Erfindung.

Die Ergebnisse der vorliegenden Studie etablieren den PIGF Serumspiegel als eine neue wirksame unabhängige prognostische Determinante des klinischen Ausgangs bei Patienten mit akuten Koronarsyndromen. Besonders zu bemerken ist, daß in Patienten mit niedrigen hsCRP Serumspiegeln, erhöhte P1GF Serumspiegel eine Untergruppe von Patienten identifizieren, die an einem signifikant erhöhten kardialen Risiko leiden (angepaßtes Gefahren-Verhältnis 3,58 [95% CI 1,48 - 7,72]; p = 0,001).

Der prädiktive Wert von PIGF Serumspiegel ist unabhängig von Evidenz für myokardiale Nekrose, wie durch den Troponin Serumspiegel bestimmt. Zuletzt identifizieren erhöhte PIGF Serumspiegel nicht nur diejenigen Patienten mit akuten Brustschmerzen, die akute Koronarsyndrome entwickeln, sondern auch diejenigen Patienten, die an einem erhöhten Risiko von wiederkehrender Instabilität nach der Entlassung von einem anfänglichen akuten Koronarsyndrom leiden. Daher kann das Messen des PIGF Serumspiegels nicht nur ein verläßliches und wirksames klinisches Werkzeug für die Identifizierung von Patienten mit hoch-Risiko Läsions-Bildung sein, sondern auch von andauernder vaskulärer Entzündung der Koronarzirkulation.

Die Rolle von P1GF als einem primären Entzündungsmarker von arteriosklerotischer Läsions-Instabilität kann gut durch seine gut dokumentierten pro-inflammatorischen Effekte in Tiermodellen von Arteriosklerose oder Arthritis erklärt werden [Luttun A, Tjwa M, Moons L, et al. Revascularization of ischemic tissues by P1GF treatment, and inhibition of tumor angiogenesis, arthritis and atherosclerosis by anti-FIt1. Nat Med 2002;8(8):831-40.]. Obwohl P1GF zu der Familie von VEGF gehört, scheint seine ätiopathogenetische Rolle eher mit der Entzündung als mit der Angiogenese zusammenzuhängen [Luttun A, Tjwa M, Moons L, et al. Revascularization of ischemic tissues by P1GF treatment, and inhibition of tumor angiogenesis, arthritis and atherosclerosis by anti-Flt1 . Nat Med 2002;8(8):831-40.]. In der Tat, während die VEGF-Erhöhung durch Hypoxie und die Erhöhung des VEGF-Serumspiegels als eine frühe Anpassung des Myokards an den abnehmenden Blutfluß angesehen werden [Lee SH, Wolf PL, Escudero R, Deutsch R, Jamieson SW, Thistlethwaite PA. Early expression of angiogenesis factors in acute myocardial ischemia and infarction. N Engl J Med 2000;342(9):626-33.], wird PIGF nicht durch Hypoxie beeinträchtigt oder herunter-reguliert [Khaliq A, Dunk C, Jiang J, et al. Hypoxia down-regulates placenta growth factor, whereas fetal growth restriction up-regulates placenta growth factor expression: molecular evidence for "placental hyperoxia" in intrauterme growth restriction. Lab Invest 1999;79(2):151-70., Cao Y, Linden P, Shima D, Browne F, Folkman J. In vivo angiogenic activity and hypoxia induction of heterodimers of placenta growth factor/vascular endothelial growth factor. J Clin Invest 1996;98(11):2507-11.]. In Übereinstimmung mit diesen Daten, ergaben die Ergebnisse dieser Studie keine Korrelation zwischen dem P1GF Serumspiegel und dem Troponin T Serumspiegel als ein Marker von myokardialer Nekrose, wohingegen der VEGF Serumspiegel positiv mit dem Troponin T Serumspiegel korrelierte. Übereinstimmend damit korrelierte P1GF nicht mit dem VEGF Serumspiegel.

Daher scheint der PIGF Serumspiegel nicht durch myokardiale Nekrose beeinträchtigt zu werden. Im Gegenteil sind die VEGF Serumspiegel mit einer Erhöhung von Troponin T, beeinträchtigtem TIMI Fluß, und klinischen Anzeichen von myokardialer Ischämie gekoppelt [Heeschen C, Dimmeler S, Hamm CW, Boersma E, Zeiher AM, Simoons ML. Prognostic significance of angiogenic growth factor serum levels in patients with acute coronary syndromes. Circulation 2003;107:524-530.]. Der P1GF Serumspiegel als nicht sensitiv gegenüber kleineren myokardialen Verletzungen könnte in Patienten mit akuten Koronarsyndromen spezifisch wichtig sein, von denen ungefähr ein Drittel bei Einlieferung positiv für Troponin sind [Hamm CW, Braunwald E. A classification of unstable angina revisited. Circulation 2000;102(1):118-22.].

Ähnlich könnte eine myokardiale Verletzung auch den Wert des hsCRP Serumspiegels kompromittieren, um so den Ausgang in Patienten mit akuten Koronarsyndromen vorhersagen zu können. Als ein klassischer unspezifischer stromabwärts akute-Phase-Marker, sind die hsCRP Serumspiegel in Patienten mit myokardialer Verletzung erhöht, wie durch Troponin T Erhöhung gemessen. Es ist gut etabliert, daß erhöhte Serumspiegel von hsCRP vor dem Auftreten eines Markers von myokardialer Nekrose in nahezu allen Patienten gefunden werden, in de nen vor einem Infarkt eine instabile Angina auftritt [Liuzzo G, Baisucci LM, Gallimore JR, et al. Enhanced inflammatory response in patients with preinfarction unstable angina. J Am Coll Cardiol 1999;34(6): 1696-703.]. Die Spezifität von erhöhtem hsCRP Serumspiegel, der eine verstärkte vaskuläre Entzündung bestätigt, ist daher in Anwesenheit von myokardialer Verletzung sehr begrenzt.

Daher können in Troponin-positiven Patienten erhöhte hsCRP Serumspiegel lediglich ein erhöhtes Risiko sekundär zu myokardialer Verletzung als ein Surrogatmarker für Thrombembolie darstellen, die eher von einem aktiven thrombotischen Prozeß innerhalb der kulpritischen Läsion als einer andauernden vaskulären Entzündung stammen.

Tatsächlich sind, wenn jeweils Troponin T und. VEGF als Marker von myokardialer Nekrose und Ischämie in eine multivariante Analyse einbezogen werden, erhöhte hsCRP Serumspiegel nicht länger für eine erhöhtes Risiko in Patienten mit akuten Koronarsyndromen prädiktiv.

Noch wichtiger variiert die berichtete Prävalenz von erhöhtem hsCRP Serumspiegel in akuten Koronarsyndromen beträchtlich. (Mehr als 30% der Patienten mit schwerer instabiler Angina und mehr als 50% der Patienten mit einem akuten myokardialen Infarkt zeigen keine erhöhten hsCRP Serumspiegel). Erhöhte hsCRP Serumspiegel fehlen in mehr als 30 % von Patienten mit schwerer instabiler Angina und in mehr als 50 % von Denjenigen mit einem akuten myokardialen Infarkt, der nicht nach einer instabilen Angina folgt [Liuzzo G, Baisucci LM, Gallimore JR, et al. Enhanced inflammatory response in patients with preinfarction unstable angina. J Am Coll Cardiol 1999;34(6): 1696-703.], was eine wichtige Heterogenität der Rolle von entzündlichen Auslösern des klinischen Syndroms der koronaren Instabilität vermuten läßt [Libby P, Ridker PM, Maseri A. Inflammation and atherosclerosis. Circulation 2002;105(9): 1135-43.]. Jedoch ist es auch gut etabliert, daß Individuen in ihrer systemischen Antwort auf einen bestimmten entzündlichen Stimulus variieren können [Liuzzo G, Buffon A, Biasucci LM, et al. Enhanced inflammatory response to coronary angioplasty in patients with severe unstable angina. Circulation 1998;98(22):2370-6., Liuzzo G, Angiolillo DJ, Buffon A, et al. Enhanced response of blood monocytes to in vitro lipopolysaccharide-challenge in patients with recurrent unstable angina. Circulation 2001;103(18):2236-41. Biasucci LM, Vitelli A, Liuzzo G, et al. Elevated levels of interleukin-6 in unstable angina. Circulation 1996;94(5):874-7.]. Die Zunahme in hsCRP oder IL-6, die in Antwort auf das durch Ballondilatation induzierte vaskuläre Trauma oder sogar durch unkomplizierte Herzkatheterisierung beobachtet wurde, korreliert linear mit Basislinien hsCRP oder Interleukin-6 Serumspiegel [Liuzzo G, Buffon A, Biasucci LM, et al. Enhanced inflammatory response to coronary angioplasty in patients with severe unstable angina. Circulation 1998;98(22):2370-6.]. Zusätzlich ist die IL-6 Produktion durch Monozyten, die von Patienten mit instabiler Angina isoliert werden, signifikant in Patienten mit erhöhtem hsCRP Serumspiegel erhöht, verglichen mit Patienten mit normalem hsCRP Serumspiegel [Liuzzo G, Angiolillo DJ, Buffon A, et al. Enhanced response of blood monocytes to in vitro lipopolysaccharide-challenge in patients with recurrent unstable angina. Circulation 2001;103(18):2236-41.]. Diese individellen Unterschiede im Ausmaß der Antwort auf einen bestimmten entzündlichen Stimulus können eine genetische Ursache haben [Westendorp RG, Langermans JA, Huizinga TW, Verweij CL, Sturk A. Genetic influence on cytokine production in meningococcal disease. Lancet 1997;349(9069):1912-3.]. Unglücklicherweise schränken solche heterogenen Antworten die Brauchbarkeit von stromabwärts akute-Phase Reaktanten, wie zum Beispiel hsCRP, als entzündlichen Marker für die Risikoabschätzung ein. Im Gegensatz dazu scheint P1GF ein direkter proximaler Stimulus für entzündliche Prozesse innerhalb der Gefäßwand zu sein [Luttun A, Tjwa M, Moons L, et al. Revascularization of ischemic tissues by P1GF treatment, and inhibition of tumor angiogenesis, arthritis and atherosclerosis by anti-FIt1. Nat Med 2002;8(8):831-40.]. Tatsächlich waren erhöhte PIGF Serumspiegel extrem informativ spezifisch in Patienten mit akuten Koronarsyndromen, jedoch nicht nicht-erhöhte hsCRP Serumspiegel. In dieser Patientenkohorte, identifizierten erhöhte PIGF Serumspiegel eine Untergruppe von Patienten mit deutlich erhöhtem kardialem Risiko, das ähnlich zu den durch erhöhte Troponin Serumspiegel definierten hoch-Risiko Patienten war. Daher können erhöhte P1GF Serumspiegel in der Tat einen primären entzündlichen Anzeiger koronarer Instabilität darstellen.

Weiterhin können, weil die pro-inflammatorischen Effekte von P1GF spezifisch durch Blokkieren seines Rezeptors Flt-1 inhibiert werden können, diese Ergebnisse auch einen Ansatz für ein neues anti-inflammatorisches therapeutisches Ziel in Patienten mit koronarer arterieller Erkrankung zur Verfügung stellen [Luttun A, Tjwa M, Canneliet P. Placental Growth Factor (P1GF) and Its Receptor Flt-1 (VEGFR-1): Novel Therapeutic Targets for Angiogenic Disorders. Ann N Y Acad Sci 2002;979:80-93.]. Die pro-inflammatorischen Effekte von P1GF können spezifisch durch Blockieren seines Rezeptors Flt-1 inhibiert werden und stellen eine neue anti-inflammatorische therapeutische Option in Patienten mit Koronar-Arterienerkrankung zur Verfügung.

Die Ergebnisse der vorliegenden Studie zeigen, das erhöhte Blutspiegel der Metalloproteinase PAPP-A mit negativem Ausgang in Patienten mit ACS zusammenhängen. In Übereinstimmung mit einer erst kürzlich durchgeführten Untersuchung war der Vorhersagewert von PAPP-A Plasmaspiegeln am deutlichsten in Patienten ohne eine Erhöhung von Troponin. Daher ist ein erhöhter PAPP-A Plasmaspiegel nicht nur ein Marker der Plaque-Instabilität, der die Entwicklung von ACS fördert, sondern, noch wichtiger, zeigt eine schlechte Prognose an, vor dem Auftreten eines akuten ischämischen Ereignisses, das durch Plaque-Instabilität verursacht wird. Zusätzlich stellten erhöhte PAPP-A Spiegel weitere prognostische Information sogar in Patienten mit erhöhten hsCRP Plasmaspiegeln zur Verfügung, was vermuten läßt, daß zumindest in einigen Patienten erhöhte hsCRP Spiegel nicht mit vaskulärer Entzündung zusammenhängen. Das Ergebnis, daß der Vorhersage. Wert von PAPP-A auf Patienten mit niedrigen Spiegeln des anti-inflammatorischen Cytokins Interleukin-10 beschränkt waren unterstützt weiter das Konzept, daß die Balance zwischen pro- und anti-inflammatorischen Cytokinen für den Ausgang für den Patienten bei ACS ist. Durch multivariante Regressionsanalyse wurden mehrere biochemische Marker, einschließlich Troponin T, löslicher CD40 Ligand, Interleukin-10 und PAPP-A als unabhängige Vorhersagemarker für den Ausgang für den Patienten während der anschließenden sechs Monate der Nachverfolgung identifiziert.

Kardiale Troponine sind sensitive und spezifische Markers von myokardialer Nekrose, sekundär zu thrombotischen Komplikationen während eines akuten Koronarsyndroms und sind hoch prädiktiv für den frühen klinischen Verlauf nach dem Ausbruch von ACS. Jedoch bleibt die Risikostratifizierung in Troponin-negativen Patienten mit akuten Koronarsyndrom eine Herausvorderung.

Ungefähr zwei Drittel der Patienten mit ACS jedoch ohne ST-Segmenterhöhungen weisen normale Troponinwerte auf und mehr als die Hälfte der Patienten weisen inkonklusive elektrokardiographische Ergebnisse auf. Während den ersten Wochen nach dem Ausbruch eines akuten Koronarsyndroms ist das Todesrisiko oder nicht-fataler Myokardinfarkt in Troponin-negativen Patienten bei ungefähr 5 bis 8 %. Daher verbleibt das Kurzeit-Auftreten von wesentlichen kardiovaskulären Ereignissen relativ substantiell in Patienten ohne Hinweise auf myokardiale Nekrose, die das Bedürfnis für eine weitere diagnostische Aufarbeitung hervorruft. Ein kontinuierliche ST-Segment Überwachung, Streßtests und Perfusions-bildgebende Verfahren können von begrenzter Verfügbarkeit für die unmittelbare Risikostratifizierung von Patienten sein, von denen vermutet wird, daß sie ein akutes Koronarsyndrom aufweisen. Die vorliegende Studie zeigt, daß PAPP-A Spiegel eine Untergruppe von Patienten ohne Troponin-Erhöung anzeigen, die während des frühen Zeitverlaufs nach dem Ausbruch von Symptomen ein wesentlich höheres Risiko auf kardiale Ereignisse aufweisen (72 Stunden OR 3,17; 30 Tage: OR 3,33). Im Unterschied dazu unterlagen die Patienten ohne die ST-Segmentveränderungen, die negativ für sowohl TnT und PAPP-A waren, einem sehr niedrigen Risiko (0,9% Auftrittsrate).Daher ist die PAPP-A Bestimmung in Patienten mit ACS ein wirksames Werkzeug für die Kurzzeit-Risikostratifizierung von Patienten ohne erhöhte Troponinspiegel.

Das Fortschreiten und die anschließende Destabilisierung von arteriosklerotischen Plaques schließt wichtige Veränderungen in der Struktur der arteriellen Wand. Das Auftreten eines lokalen Entzündungs-Zustands in Patienten mit ACS ist gut etabliert, wie durch inflammatorsiche Marker, wie zum Beispiel CRP nachgewiesen. Metalloproteinasen sind auch potentielle Indikatoren von arterieller Inflammation und können durch Abbauen von extrazellulärer Matri zu der Fragilität des Lipid-reichen, arteriosklerotischen Plaques und eventuell zu seiner Ruptur beitragen. Wie früher für mehrere andere Metalloproteinasen (MMP-1, MMP-3, MMP-12 oder MMP-13) beschrieben, wurde von PAPP-A nur erst kürzlich gefunden, daß sie in in erodierten und gelösten Plaques exprimiert wurde, wohingegen die PAPP-A Expression in stabilen Plaques nicht nachweisbar war. Andere Studien haben auch gezeigt, daß Patienten mit hyperechonischen oder isoechoinischen Karotidplaques signifikant höhere PAPP-A Spiegel zeigen, als diejenigen mit hypoechonischen frühen Karotid-Läsionen. Die genaue Rolle von PAPP-A in der Pathophysiologie von ACS bleibt unklar. Von PAPP-A wurde gezeigt, daß er ein spezifischer Aktivator des Insulin-ähnlichen Wachstumsfaktor -I (IGF-I) ist, einem potenter Mediator der Arteriosklerose. Als eine Matrixmetalloproteinase könnte PAPP-A an der Prozessierung der extrazellulären Matrix der Plaques beteiligt sein und konsequenterweise die fibröse Kappe beeinträchtigen. Dies führt zu einer Plaquemorphologie, die empfindlicher gegenüber Erosion, Ruptur und anschließender Thrombose ist. Die vorliegende Studie zeigt, daß eine einzelne PAPP-A Messung, die 8,7 Stunden nach dem Auftreten der Symptomse erhalten wird, einen signifikant prädiktiven Wert für das Auftreten von Tod und nicht-fatalem Myokardinfarkt während der anschließende 6 Monate Nachfolge zur Verfügung zu stellen. Diese Daten lassen vermuten, daß PAPP-A eine bedeutende pathophysiologische Rolle in der Destabilisierung der arteriosklerotischen Plaques währen ACS spielt. Die Produktion von PAPP-A durch aktivierte Zellen innerhalb der arteriosklerotischen Läsionen und seine Freisetzung in die extrazellulären Matrix scheinen eng mit dem lokalen inflammatorischen Prozeß verbunden zu sein, der innerhalb der arteriellen Wand auftritt, wie durch die signifikante positive Korrelation angezeigt, die zwischen CRP und PAPP-A Spiegeln beobachtet wurde. In der Tat waren die PAPP-A-Spiegel hoch prädiktiv in Patienten mit erhöhten CRP Spiegeln, wo hingegen in Patienten mit niedrigen CRP Spiegeln PAPP-A nicht als ein signifikanter Prädiktor für den Ausgang des Patienten diente (Figur. 26a). Während CRP Spiegel mit der Troponin-Erhöhung verbunden sind, scheinen PAPP-A Spiegel weniger sensitiv gegenüber kleinerer myokardialer Verletzung zu sein, was besonders wichtig bei Patienten mit ACS sein kann, von denen ungefähr ein Drittel zum Zeitpunkt der Ankunft im Krankenhaus auf Troponin positiv sind. Zusätzlich interferierten PAPP-A Spiegel weder mit der prädiktven Aussagekraft von sCD40L, einem Marker der Plättchenaktivierung in Patienten mit ACS, noch beeinträchtigten sie diese. Durch multivariante Analyse ergaben sich PAPP-A, sCD40L und TnT alle als unabhängige Prädiktoren von nachteiligem Ausgang (Tabelle 2). Eine Kombination von PAPP-A und sCD40L war besonders deutlich in Patienten, die auf TnT negativ waren, was vermuten läßt, daß beide Marker distinkte Signalwege widerspiegeln, die eventuell zu einem pro-inflammatorischen und pro-koagulierenden Milieu in der koronaren Zirkulation Unterstützend für eine komplementäre, anders als eine kompetetive Rolle, um einen nachteiligen Ausgang in Patienten mit ACS vorherzusagen, sind die Ergebnisse der Erfinder, daß die aggressive Inhibierung der Plättchenaggregation durch Abciximab besonders in Patienten mit erhöhten sCD40L Spiegeln brauchbar war.

In der Zusammenfassung zeigen die Ergebnisse der vorliegenden Erfindung, daß erhöhte Plasmaspiegel von PAPP-A als ein Marker der vaskulären Inflammation mit einem erhöhten Risiko auf anschließende kardiale Ereignisse in Zusammenhang stehen. Der prädiktive Wert von PAPP-A Plasmaspiegeln war unabhängig von erhöhten Troponinspiegel, die das aktuelle Risiko sekundär zu thrombotischen Komplikationen widerspiegeln, die zu myokardialer Verletzung während einem akuten Koronarsyndrom führen. Daher ist ein erhöhter PAPP-A Plasmaspiegel nicht nur ein Marker der Plaqueinstabilität, was das Fortschreiten zu einem Myokardinfarkt betrifft, sondern zeigt auch eine schlechte Prognose sogar nach dem Auftreten eines akuten ischämischen Vorfalls an, der durch Plaqueinstabilität verursacht wird.

Zusammenfassend läßt sich sagen, daß die vorliegende Studie die bedeutende und unabhängige Rolle von PIGF als einen Marker für die diagnostische und therapeutische Risikostratifizierung dokumentiert. Das erhöhte kardiale Risiko von Patienten mit hohen PIGF-Konzentrationen, die eine Standardtherapie mit Heparin und Aspirin erhielten, wurde durch den Glycoprotein IIb/IIIa-Rezeptorantagonisten Abciximab aufgehoben. Die kombinierte Verwendung von Troponinen und P1GF, welche beide wesentliche Bestandteile der Pathophysiologie bei Patienten mit akuten Koronarsyndromen darstellen, liefert wichtige Einblicke in die Aktivität der Erkrankung, dem kardiologischen Risiko und der Wirksamkeit einer Behandlung mittels Glycoprotein IIb/IIIa-Hemmung durch Abciximab, welche der Verwendung eines einzelnen Markers überlegen ist.

Zusammenfassend stellt der PIGF Serumspiegel einen wirksamen und verläßlichen Biomarker der vaskulären Entzündung und negativem Ausgang in Patienten mit akuten Koronarsyndromen dar. Ein Messen der P1GF Serumspiegel erweitert die von herkömmlichen entzündlichen Markern in akuten Koronarsyndromen erhaltene prädiktive und prognostische Information signifikant. Die Verwendung von PAPP-A als einziger Marker oder in Kombination mit anderen Markern ohne eine Verwendung von PIGF ist nicht Gegenstand der vorliegenden Erfindung.

Die Erfindung soll nun im folgenden anhand von Beispielen unter Bezugnahme auf die beigefügten Figuren näher erläutert werden, ohne jedoch dadurch begrenzt zu werden. In den Figuren zeigt: .
**Figur 1** den Zusammenhang zwischen der Serumkonzentration an sCD40L und der Häufigkeit eines kardialen Ereignisses nach 24 Stunden, 72 Stunden, 30 Tagen und 6 Monaten in der Patientengruppe, die ein Placebo erhalten hatte (n = 544). Die Patienten wurden in 5 Quintil eingeteilt. Der Bereich der sCD40L-Konzentrationen war wie folgt: 0,003-1,9 µg/l (Quintil 1), 1,9-3,5 µg/l (Quintil 2), 3,5-5,0 µg/l (Quintil 3), 5,0-6,3 µg/l (Quintil 4) und > 6,3 µg/l (Quintil 5). p < 0,001 nach 72 Stunden, 30 Tagen und 6 Monaten.
**Figur 2** eine Darstellung nach Kaplan-Meier der kumulativen Inzidenz von Myokardinfarkt mit tödlichem oder nicht-tödlichem Ausgang nach 72 Stunden (a) und 6 Monaten (b) gemäß den *Base* Line-Werten der sCD40L-Konzentration (diagnostischer Schwellenwert 5,0 µg/l) in der Placebo-Gruppe (n = 544).
**Figur 3** den angepaßten Risikoquotienten (einschließlich 95% Vertrauensintervall) bei der Behandlung mit Abciximab gemäß den sCD40L-Quintilen. Eine erfolgreiche Behandlung wird definiert als Reduktion von tödlichen oder nicht-tödlichen Myokardinfarkten im Verlauf von 6 Monaten. Ein Risikoquotient < 1,0 gibt die erfolgreiche Behandlung mit Abciximab im Vergleich zur Placebo-Behandlung an.
**Figur 4** eine Kalpan-Meier-Darstellung von Mortalität und nicht-tödlichem Myokardinfarkt 72 Stunden (a) und 6 Monate (b) gemäß der sCD40L-Konzentration bei Patienten, die entweder ein Placebo oder Abciximab erhalten hatten.
**Figur 5** die Plättchenaktivierung in Abhängigkeit von der Aktivität der Erkrankung. Die Plättchenaktivität, bestimmt durch Monozyt-Plättchen-Aggregate, war bei Patienten mit stabiler koronarer Herzerkrankung signifikant erhöht. Eine weitere signifikante Zunahme der Plättchenaktivierung wurde in Patienten mit akuten Koronarsyndromen beobachtet.
**Figur 6**, daß die sCD40L-Serumkonzentrationen eng mit dem Ausmaß der Plättchenaktiverung bei Patienten mit und ohne akute Koronarsyndrome korrelieren. Die unterbrochen dargestellten Linien teilen die Patienten in Tertil gemäß der Plättchenaktivierung (< 15%, 15%-30%, > 30%) bzw. sCD40L-Serumkonzentrationen (< 2,5 g/l; 2,5-4,5 µg/l; > 4,5 µg/l) ein.
**Figur 7** den Zusammenhang zwischen P1GF und hsCRP als einem stromabwärts akute-Phase Reaktanten (n = 1088).
**Figur 8** jeweils die PIGF und hsCRP Serumspiegel, gemäß dem Basislinien Troponin T Status (n = 1088).

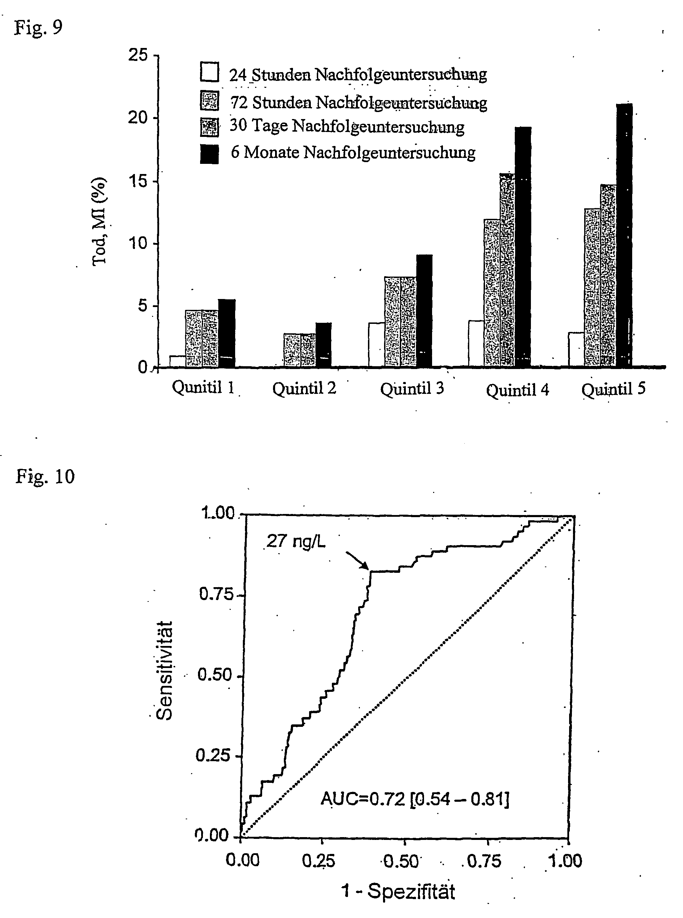

**Figur 9** den Zusammenhang zwischen der Serumkonzentration an PIGF und der Häufigkeit eines kardialen Ereignisses nach 24 Stunden, 72 Stunden, 30 Tagen und 6 Monaten in der Patientengruppe, die ein Placebo erhalten hatte (n = 547): Der Bereich der PIGF-Konzentrationen war wie folgt: unterhalb von gleich zu 13,3 ng/l (1. Quintil), 13,4 bis 19,2 ng/l (2. Quintil), 19,3 bis 27,3 ng/l (3. Quintil), 27,4 bis 40,0 ng/l (4. Quintil), und oberhalb 40,0 ng/l (5. Quintil). Die Unterschiede in den Raten eines Vorkommens zwischen den Quartilen waren signifikant bei 30 Tagen (p 0,001) und 6 Monaten (p < 0,001) anschließender Untersuchung.
**Figur 10** Receiver-Operating-Charakteristia Kurvenanalyse für den prädiktiven Wert von PIGF Serumspiegeln für das Auftreten von Mortalität oder nicht-tödlichem Myokardinfarkt bei 6 Monaten Nachuntersuchung.
**Figur 11** eine Darstellung nach Kaplan-Meier der kumulativen Inzidenz von Myokardinfarkt mit tödlichem oder nicht-tödlichem Ausgang nach 72 Stunden (a) und 6 Monaten (b) gemäß den *Base* Line-Werten der PIGF-Serumspiegel (diagnostischer Schwellenwert 27,0 ng/l; n = 547).
**Figur 12** den prädiktiven Wert von PIGF für die Inzidenz von Myokardinfarkt mit tödlichem oder nicht-tödlichem Ausgang nach hsCRP Serumspiegeln (a) und Troponin T Serumspiegeln (b). Diagnostische Schwellenwerte waren 27,0 ng/l für P1GF, 0,1 ug/l für Troponin T und 10 mg/l für hsCRP; n = 547).
**Figur 13** den prädiktiven Wert von Entlassungs-P1GF Serumspiegeln für das Patientenergebnis bei 6-Monaten Nachuntersuchung. Patienten mit erhöhten P1GF Serumspiegeln unterlagen einem höheren kardialen Risiko, mit einer Auftrittsrate von 7,4 %, verglichen mit 2,2 % für Patienten mit P1GF Serumspiegeln unterhalb von 27,0 ng/l (p = 0,005).
**Figur 14**: jeweils PAPP-A und hsCRP-Spiegel gemäß des Basislinien Troponin T Status. Kreise zeigen Ausreißer an.
**Figur 15**: jeweils löslicher CD40 Ligand und hsCRP-Spiegel gemäß des Basislinien PAPP-A Status. Kreise zeigen Ausreißer an.
**Figur 16**: Zusammenhang zwischen den PAPP-A Plasmaspiegeln und der kardialen Ereignisrate bei 24 h, 72h, 30 Tagen und 6 Monaten gemäß der PAPP-A Placebo Gruppe (n = 547). Der Bereich von PAPP-A war wie folgt: (PAPP-A_1) < 4,5 mIU/l (n=111);. (PAPP-A_2) 4,5 - 7,5 mIU/l (n = 108); (PAPP-A_3) 7,6 - 12,6 mIU/l (n = 109); (PAPP-A 4) 12,7 - 24,0 mIU/l (n = 110) und (PAPP-A_5) > 24,0 mIU/1. Die Unterschiede in den Auftrittsraten waren signifikant bei 72 Stunden (p = 0,019), 30 Tagen (p = 0,008) und 6 Monaten (p = 0,004).
**Figur 17**: *Receiver Operating* Charakteristika Kurven Analyse für den Vorhersagewert von PAPP-A Plasmaspiegeln für das Auftreten von Tod und nicht-fatalem Myokardinfarkt bei 6 Monaten Nachverfolgung.
**Figur 18**: Kaplan-Meier Ereignisrate Kurven, die das kumulative Auftreten von Tod und nicht-fatalem Myokardinfarkt bei 72 Stunden (a) und 6 Monaten (b) gemäß den PAPP-A Basislinien Plasmaspiegeln zeigen. Diagnostischer Schwellenwert 12,6 mIU/l; n = 547
**Figur 19**: der Vorhersagewert von PAPP-A für das Auftreten von Tod und nicht-fatalem Myokardinfarkt wurde auf Patienten mit erhöhten hsCRP Spiegeln beschränkt (a) und Patienten mit niedrigen Spiegeln des anti-inflammatorischen Cytokins IL-10 (b). Diagnostischer Schwellenwerte 12,6 mIU/l für PAPP-A, 10 mg/l für hsCRP und 3,5 ng/l für IL-10; n = 547
**Figur 20**: der Vorhersagewert von PAPP-A für das Auftreten von Tod und nicht-fatalem Myokardinfarkt war besonders informativ in Patienten ohne Troponin T Erhöhung (a). In Patienten, die sowohl negativ für TnT und sCD40L waren, identifizierte PAPP-A eine Subgruppe, die an einem erhöhten kardiovaskulären Risiko bei 6 Monaten Nachfolge litt (b). Diagnostischer Schwellenwerte 12,6 mIU/l für PAPP-A, 0,1 µg/l für TnT und 5,0 µg/l für sCD40L; n = 547

### Beispiele

### I. sCD40L

### Die Verwendung von sCD40L als einziger Marker oder in Kombination mit anderen Markern ohne eine Verwendung von PIGF ist nicht Gegenstand der vorliegenden Erfindung_

### 1. Patienten

Die CAPTURE-Studie registriert 1,265 Patienten mit refraktärer instabiler Angina (61% männlich, im Alter von 61 [48-72, 95% Vertrauensintervall]) zwischen Mai 1993 und Dezember 1995. Alle CAPTURE-Patienten klagten über wiederkehrende Brustschmerzen im Ruhezustand, assoziiert mit EKG-Änderungen während einer Behandlung mit intravenösem Heparin und Glycerintrinitrat im Mittel für 14 Stunden. Die gesamte Patientenpopulation wurde einer Koronarangiographie unterzogen, bevor das Auftreten einer deutlichen koronaren Arterienerkrankung mit auslösenden Läsionen ≥ 17%, die für eine Angioplastie geeignet waren, dokumentiert wurden. Die Patienten wurden einer Behandlung durch Abciximab oder Placebo zufalls-verteilt zugeordnet. Die Behandlung wurde innerhalb von 2 Stunden nach der Zuordnung begonnen. Bei allen Patienten waren innerhalb von 18 bis 24 Stunden nach Beginn der Behandlung koronare Interventionen vorgesehen (CAPTURE. Lancet 1997; 349:1429-35). Es wurden im Mittel 8,7 (3,6-11,3) Stunden nach Einsetzen der Symptome Blutproben (n = 1096)gewonnen *(Base Line).*

Primäre Endpunkte der Studie waren Mortalität, Myokardinfarkt oder die Notwendigkeit zu sofortiger Intervention (Angioplastie, *Bypass-Operationen* der Koronararterie) aufgrund einer Instabilität während 30 Tagen oder 6 Monaten. Bei Patienten, die während des Krankenhausaufenthalts einen Herzinfarkt erlitten hatten, wurde ein solcher diagnostiziert, wenn ihre Werte der Kreatinkinase-Enzymaktivität in mindestens zwei Proben mehr als dreimal so hoch waren wie die obere Grenze des Normalbereichs oder wenn ihr EKG neue deutliche Q-Zacken in mehr als zwei aufeinanderfolgenden Intervallen aufwiesen. Diese strikte Definition wurde gewählt, um jeden unbedeutend geringen Anstieg der Kreatinkinase nach PTCA auszu schließen. Bei Patienten mit einem Myokardinfarkt nach der Entlassung wurde ein solcher definiert, wenn ihre Werte der Kreatinkinase-Enzymaktivität mehr als doppelt so hoch waren, wie die obere Grenze des Normalbereichs oder wenn ihr EKG neue deutliche Q-Zacken in zwei oder mehr aufeinanderfolgenden Intervallen aufwies. Der sekundäre Endpunkt war die symptomatische koronare Restenose der behandelten Läsion mit einem Stenosedurchmesser >_ 70% und die Notwendigkeit zu wiederholter Revaskularisierung während der nachfolgenden 6 Monate.

### 2. Validierung von Patienten mit akutem Brustschmerz

Eine separate Gruppe von 626 Patienten mit Brustschmerz (161 Frauen und 465 Männer, mittleres Alter 61 [38-82] Jahre), die in fortlaufender Reihe mit akutem Brustschmerz, der weniger als 12 Stunden (im Mittel 5,1 [2,1-10,4] Stunden) andauerte, in die Notaufnahme kamen. Nicht berücksichtigt wurden Patienten mit einer charakteristischen ST-Hebung im Basis-EKG bzw. einem dokumentierten akuten Myokardinfarkten während der vorangegangenen 2 Wochen. Blutproben wurden zum Zeitpunkt der Aufnahme (vor Beginn der Behandlung) und 4 Stunden später gewonnen; auf Eis gehalten, innerhalb von 20 Minuten nach Probennahme zentrifugiert und zur späteren Analyse bei -80°C gelagert. Es hatte sich gezeigt, daß diese Behandlung zu reproduzierbaren Ergebnissen bei der Messung der sCD40L-Konzentrationen führte (Nannizzi-Alaimo L, Rubenstein MH, Alves VL, Leong GY, Phillips DR, Gold HK. Circulation 2002; 105:2849-54). Die Patienten wurden bis zu ihrer Entlassung aus dem Krankenhaus und 30 Tage danach beobachtet, um tödliche und nicht-tödliche Myokardinfarkte zu verzeichnen. Das Vorhandensein einer koronaren Arterienerkrankung wurde durch eines der folgenden Kriterien nachgewiesen: EKG-Anzeichen auf eine Myokard-Ischämie (neue Änderungen in der ST-Strecke oder Inversion der T-Zacke), eine koronare Herzerkrankung in der Anamnese (Myocardinfarkt oder koronare Revaskularisierung, ein positiver Belastungstest oder Einengung des Durchmessers einer Hauptkoronararterie um mindestens 50% in einem früheren Angiogramm). Patienten ohne koronare Herzerkrankung hatten ein normales Koronar-Angiogramm aufzuweisen. An einer Untergruppe von Patienten, die 131 Patienten mit akuten Koronarsyndromen, 20 Patienten mit stabiler koronarer Herzerkrankung und 10 Patienten mit ausgeschlossener koronarer Herzerkrankung einschloß, wurde die Plättchenaktivierung mittels Durchflußzytometrie bestimmt.

### 3. Biochemische Analyse

Plasmaproben, antikoaguliert mit Natriumheparin, wurden zentral bei -80°C gelagert. Die Bestimmung der kardialen Marker wurde in Unkenntnis der Krankengeschichte der Patienten und der angeordneten Behandlung im Forschungslabor der Universität Frankfurt durchgeführt. Die Plasmaspiegel von sCD40L (Nachweisgrenze 0,005 µg/l), lösliches P-Selektin (0,5 µg/l), Tumornekrosefaktor-α (TNF-α, hochempfindlich; 0,12 ng/l) und lösliches intrazelluläres Adhäsionsmolekül-1 *(Intracellular Adhesion Molecule*-1*,* ICAM-1; 0,35 µg/l) wurden mittels ELISA (R&D Systems, Wiesbaden) gemessen. Zur Quantifizierung von Troponin T (TnT) wurde ein Ein-Schritt-Enzym-Immuntest auf der Grundlage der Elektrochemilumineszenz-Technologie (Elecsys 2010, Roche Diagnostics; Nachweisgrenze 0,01 µg/l) verwendet. C-reaktives Protein (CRP) wurde mit Hilfe des Behring BN II Nephelometers (Behring Diagnostics; Nachweisgrenze 0,2 µg/l) gemessen.

### 5. Quantitative Bestimmung von sCD40L

Die sCD40L-Konzentration wurde unter Verwendung der Sandwich-Enzym-Immuntest-Technik (R&D Systems, Wiesbaden) bestimmt. Eine Mikrotiterplatte wurde mit einem polyklonalen Antikörper, der spezifisch gegen sCD40L gerichtet war, beschichtet. Standards und Proben wurden in die *Wells* pipettiert und vorhandenes sCD40L wurde durch den immobilisierten Antikörper gebunden. Nachdem nicht-gebundenes Material weggewaschen worden war, wurde ein Enzym-gekoppelter polyklonaler Antikörper, der spezifisch gegen sCD40L gerichtet war, zu den *Wells* gegeben. Nach einem Waschschritt, um nicht gebundenes Antikörper-Enzym-Reagenz wegzuwaschen, wurde eine Substrat-Lösung zu den *Wells* gegeben, und die Farbe entwickelte sich im Verhältnis zur Menge an sCD40L, die im ersten Schritt gebunden wurde. Die Farbentwicklung wurde abgestoppt und die Farbintensität wurde gemessen.

### 6. Schnelltest für den Nachweis von CD40L

Ein Schnelltest auf der Basis der Chromatographie-Festphase-Technologie mit einem Cocktail aus goldmarkierten polyklonalen Indikator-Antikörpern aus der Maus und biotinylierten polyklonalen Capture-Antikörpern wurde entwickelt. Das Testsystem enthielt mindestens 0,3 µg jedes Antikörpers. 200 µl heparinisiertes Vollblut oder zentrifugiertes Plasma wurde der Testvorrichtung zugesetzt. Nach Trennung der zellulären Blutkomponenten von der Plasmafraktion über ein Fiberglas-Vlies wurde das Plasma, das durch das Vlies hindurchwanderte, in einem Puffer aufgenommen und zu dem adsorbierten Antikörper gegeben. Die Antikörper und die sCD40L-Moleküle der Proben bildeten Sandwich-Komplexe, die zu der Signalzone wanderten und in dem Lesefenster mittels Interaktion mit Biotin-Streptavidin akkumulierten. Positive Ergebnisse (sCD40L ≥ 4,7µg/µl wurden durch eine farbige Linie angezeigt, die sich innerhalb von 15 Minuten entwickelte. Die Indikator-Antikörper, die nicht gebunden wurden, wanderten weiter und wurden an einer Kontrollinie, bestehend aus Festphase-Anti-Maus-IgG-Antikörpem (≥ 0,2 µg) gebunden. Das Auftreten dieser Kontrollinie abwärts von der Signallinie bestätigte die fehlerfreie Testfunktion einschließlich des ungehinderten Durchflusses des Plasma.

### 7. In vivo-Plättchenaktivierung

Blutproben, antikoaguliert mit Natriumcitrat, wurden sofort 10 Minuten mit 1,1 % Paraformaldehyd in PBS, 4,6-fach verdünnt in destilliertem Wasser zur Lyse der Erythrozyten, behandelt und die fixierten Zellen in PBS gewaschen. Um P-Selektin bei Plättchen zu bestimmen, wurde das resuspendierte Pellet 60 Minuten mit Phycoerythrin (PE)-konjugiertem Glycoprotein IIb- spezifischem monoklonalem Antikörper (CD41; Dako Carpenteria, Kalifornien) und Fluoresceinisothiocyanat (FITC)-konjugiertem P-Selektin-spezifischem monoklonalem Antikörper (BD Pharmingen, San Diego, Kalifornien) inkubiert. Die Plättchen wurden durch ihre charakteristische Vorwärts- und Seitwärts-Lichtstreuung und die Bindung des PEkonjugierten Glycoprotein IIb-spezifischen Antikörpers charakterisiert. Die Plättchenaktivierung wird in % der P-Selektin-positiven Plättchen ausgedrückt. Um zirkulierende Monozyt-Plättchen-Aggregate zu identifizieren, wurden die Zellen mit FITC-konjugiertem Glycoprotein IIIa-spezifischem monoklonalem Antikörper (CD61; Dako) und PE-konjugiertem monoklonalem Antikörper gegen CD14 (BD Pharmingen) gefärbt. Die Monozyten wurden durch ihre charakteristischen Vorwärts- und Seitwärts-Lichtstreuungseigenschaften und die Bindung von PE-konjugiertem CD14-spezifischem Antikörper identifiziert. Monozyt-Plättchen-Aggregate wurden definiert als Monozyten, bei denen sich Glycoprotein IIIa nachweisen ließ, und werden in Prozent der Monozyten -Gesamtzahl angegeben (Michelson AD, Barnard MR, Krueger LA, Valeri CR, Furman MI, Circulation 2001; 104:1533-7). Jede Färbung wurde in Gegenwart gesättigter Konzentrationen eines monoklonalen unkonjugierten Ratte-Antikörpers gegen Fc-Rezeptoren (anti-CD16/32, BD Pharmingen) inkubiert um die unspezifische Bindung zu reduzieren und Isotyp-identische Antikörper dienten als Kontrolle (IgG₁-PE und IgG₂ₐ-FITC; BD Pharmingen). Insgesamt wurden 50.000 Signale mit Hilfe von FACS-Calibur (Becton/Dickinson, Heidelberg) und der CellQuest-Software (BD Pharmingen) analysiert.

### 8. Statistische Verfahren

Nach einer blinden Beurteilung der biochemischen Marker und der Plättchenaktivierung wurden die Testergebnisse mit der Datenbank abgeglichen. Um Patienten mit verschiedenen Graden eines kardiologischen Risikos unterscheiden zu können, wurde eine orientierende Datenanalyse gewählt. CAPTURE-Patienten wurden nach der sCD40L-Konzentration der Quintile eingeteilt. Für jeden der vier Zeitpunkte (24 Stunden, 72 Stunden, 30 Tage und 6 Monate) wurde eine logistische Regressionsanalyse durchgeführt, und Patienten in dem ersten Quintil (sCD40L < 2,0 µg/l) dienten als Referenz. *Receiver Operating Characteristics* (ROC) *Curve Analysis* über den dynamischen Bereich des sCD40L-Tests wurde verwendet, um die Schwellenkonzentration für sCD40L zu identifizieren, die den höchsten Vorhersagewert für die Risikostratifikation von Patienten mit akuten Koronarsyndromen lieferte. Die Auswirkung einer Erhöhung der biochemischen Marker auf das Schicksal des Patienten wurde unter Verwendung eines *Cox Proportional-Hazards Regression Model,* welches die *Base Line-Werte* der prognostischen Faktoren (z.B. EKG-Befunde, kardiale Risikofaktoren, Alter und Geschlecht) und die zufällig ausgewählte Behandlung evaluiert (Harrell FE, Jr., Lee KL, Pollock BG. J Natl Cancer Inst 1988; 80:1198-202). Alle Ergebnisse für kontinuierliche Variablen werden als Mediane mit 95% Vertrauensintervall ausgedrückt. Gleiche Zwischengruppen wurden durch den T-Test (zweiseitig) oder ANOVA bei Experimenten mit mehr als zwei Untergruppen analysiert. *Post hoc*-Bereichstests und paarweise multiple Vergleiche wurden mit dem T-Test (zweiseitig) mit Bonferroni-Abgleich durchgeführt. Der Vergleich von kategorischen Variablen wurde mit dem Pearson-chi²-Test durchgeführt. Alle Analysen wurden mit SPSS 11,0 (SPSS, Inc.) durchgeführt. Werte von p < 0,05 wurden als statistisch signifikant beurteilt.

### Beispiel 1a: Zusammenhang zwischen kardialem Risiko und sCD40L-Konzentration

Die Ausgangscharakteristika der für diese Studie ausgewählten Population (n = 1088, 86% der CAPTURE-Patienten) unterschied sich nicht von der Gesamtpopulation der Studie im Hinblick auf Alter, Geschlecht, kardiovaskuläres Risikoprofil und begleitende Behandlung vor und nach der zufälligen Auswahl. Die Reduktion kardialer Ereignisse in der Abciximab-Gruppe der Population war mit der gesamten CAPTURE-Population vergleichbar (vor PTCA: 2,2% Placebo vs. 0,9% Abciximab, p = 0,094; nach PTCA: 7,9% vs. 3,5%, p = 0,002 ; nach 30 Tagen: 9,0% vs. 4,2%, p = 0,002) (CAPTURE. Lancet 1997; 349:1429-35).

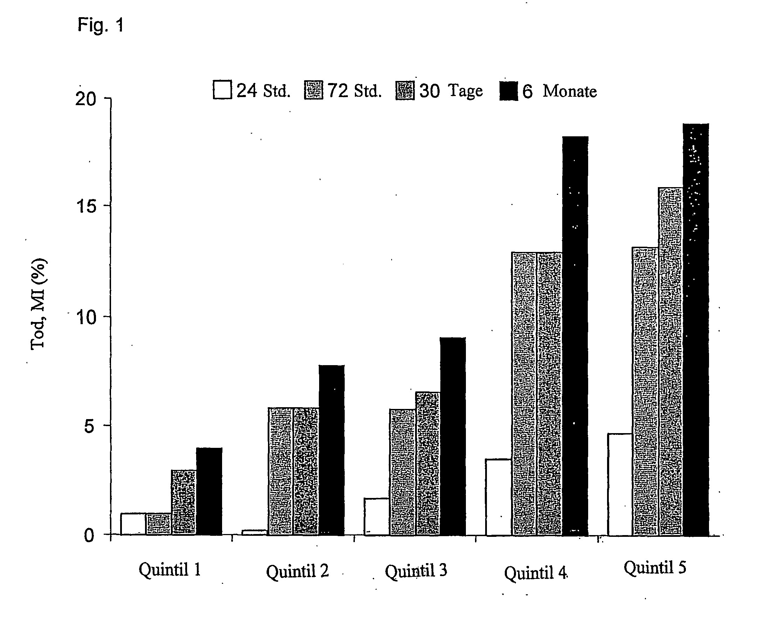

sCD40L war in den *Base* Line-Serumproben aller 1088 Patienten mit einem Mittelwert von 4,5 µg/l (Bereich 0,003-20,4) nachweisbar. Die sCD40L-Konzentration korrelierte nicht mit den gemessenen Konzentrationen an TnT (r = 0,14) und CRP (r = 0,11). Die Patienten der Placebo-Gruppe (n = 544) wurden nach ihren gemessenen sCD40L-Konzentrationen in Quintile eingeteilt: (sCD40L 1) < 1,93 µg/l (n = 100), (sCD40L 2) 1,93-3,50 µg/l (n = 102), (sCD40L 3) 3,50-5,00 µg/l (n = 121), (sCD40L 4) 5,00-6,30 µg/l (n = 115) bzw. (sCD40L 5) > 6,30 µg/l (n = 106). Während der ersten 24 Stunden waren die kombinierten Endpunkte Mortalität und nicht-tödlicher Myokardinfarkt nur in dem fünften sCD40L-Quintil im Vergleich zu dem ersten Quintil leicht erhöht (p = 0,13) (*Figur 1*). Zu späteren Zeitpunkten (72 Stunden, 30 Tage, 6 Monate) traten diese Ereignisse signifikant häufiger sowohl in dem vierten als auch dem fünften Quintil (p = 0,003, p = 0,0004 bzw. p = 0,001) auf.

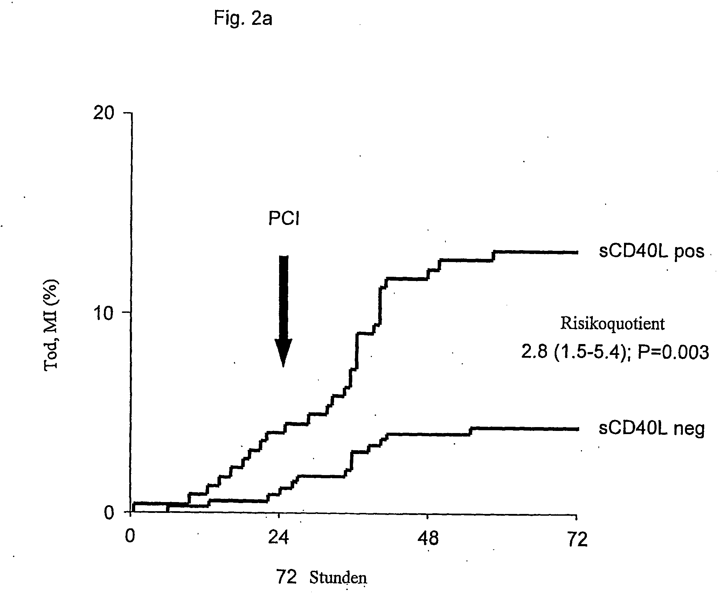

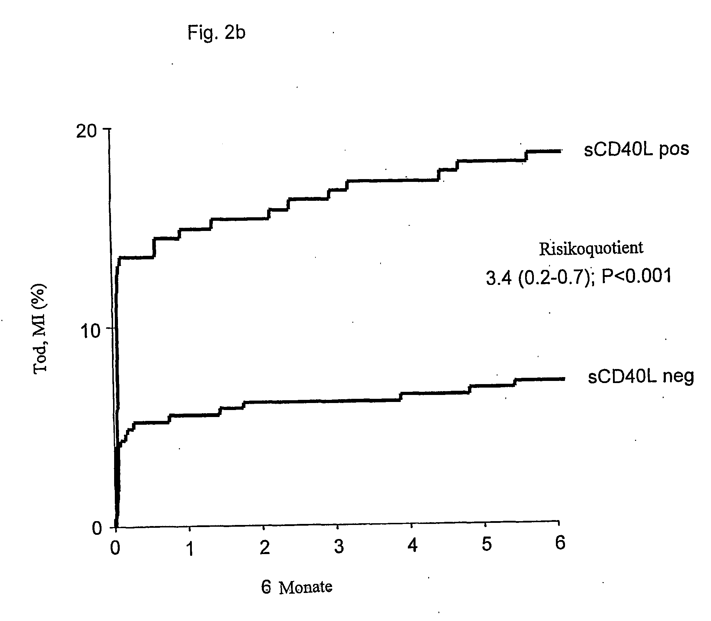

Auf der Grundlage der oben beschriebenen Befunde wurden die Patientenproben nach der berechneten Schwellenkonzentration aufgeteilt. 221 Patienten (40,6%) wiesen sCD40L-Konzentrationen von 5,0 µg/l oder darüber auf, und 323 Patienten wiesen Werte < 5,0 µg/l auf. Wie *Tabelle 1* zeigt, traten keine signifikanten Unterschiede in den Ausgangscharakteristika der beiden Gruppen auf. Bei Patienten mit niedrigen sCD40L-Konzentrationen waren die kombinierten Endpunkte tödlicher und nicht-tödlicher Myokardinfarkt signifikant- von denen von Patienten mit erhöhten sCD40L-Konzentrationen sowohl 24 Stunden vor dem Verfahren (4,1 % vs. 0,9%; p = 0,016) als auch 72 Stunden danach (einschließlich koronarer Interventionen bei allen Patienten) (13,1% vs. 4,3%; vs. 4,3%; p < 0,001) verschieden *(**Figur* 2a). Während der 6-monatigen Folgezeit divergierten die Kurven, welche die Häufigkeit eines Ereignisses anzeigen, bei Patienten mit hohen bzw. niedrigen sCD40L-Konzentrationen weiter *(**Figur 2b**).* Signifikante Unterschiede gab es sowohl nach 30 Tagen (14,5% vs. 5,3%; p < 0,001) als auch nach 6 Monaten (18,6% vs. 7,1%; p < 0,001). Aufgrund der relativ niedrigen Mortalität der CAPTURE-Gruppe unterschied sich die Endpunktmortalität nach 6 Monaten nicht signifikant zwischen den beiden Gruppen (2,3% vs. 1,5%; p = 0,72). Der Vorhersagewert von sCD40L war von einer Myokardnekrose unabhängig. Bei TnT-negativen Patienten wurde mittels der sCD40L-Konzentration eine Gruppe von Patienten mit erhöhtem kardialen Risiko (13,6%) identifiziert, das sich nicht signifikant vom kardialen Risiko TnT-positiver Patienten (14,0%; p = 1,00) unterschied. *Receiver Operating Characteristics Curve Analysis* bestätigte eine Schwellenkonzentration von 5,0 µg/l für den maximierten Vorhersagewert von sCD40L. Wiederholte, nicht dringende kardiologische Interventionen während der ersten 6 Monate waren bei Patienten mit hohen sCD40-Konzentrationen nicht signifikant verschieden von denen bei Patienten mit niedrigen sCD40L-Konzentrationen (6,2% vs. 4,5%; p = 0,45).

Von 626 Patienten ohne ST-Hebung, die mit akutem Brustschmerz in der Notaufnahme vorstellig wurden, litten 308 Patienten an akutem Koronarsyndrom (117 Patienten hatten einen akuten Myokardinfarkt, gestützt auf einer Troponin-Erhöhung ≥ 0,1 µg/1, erlitten). Bei den anderen Patienten wurden folgende Diagnosen gestellt: n = 91 stabile Angina, n = 10 Lungenembolie, n = 11 kongestive Herzininsuffizienz, n = 7 Myokarditis und n = 199 kein Hinweis auf eine Herzerkrankung. sCD40L-Konzentrationen waren bei Patienten mit akuten Koronarsyndromen signifikant höher (4,53 [3,19-5,87] µg/l) im Vergleich zu Patienten mit stabiler Angina (2,41 [1,99-3,52] µg/l; p < 0,001) bzw. Patienten ohne Anzeichen einer Herzerkrankung (1,57 [0,88-1,76] µg/l; p < 0,001). Der Durchschnittswert der oberen Referenzgrenze (*Upper Reference Limit,* URL) von 97,5 bei Patienten ohne Anzeichen kardialer Beschwerden betrug 4,7 µg/l und der URL-Durchschnittswert von 99 lag bei 6,2 µg/ml. Ähnlich wie bei den Ergebnissen, die in der CAPTURE-Studie erhalten wurden waren, korrelierten die sCD40L-Serumkonzentrationen nicht mit Nekrosemarkern (Troponin T), Entzündungsmarkern (CRP, TNF-α) und Adhäsionsmolekülen (ICAM-1). Bei Patienten mit akuten Koronarsyndromen wiesen 43,5% sCD40L-Serumkonzentrationen oberhalb des 97,5 URL-Durchschnittswerts und 21,8% sCD40L-Serumkonzentrationen oberhalb des 99 URL-Durchschnittswerts auf. Unter Verwendung einer festgesetzten Schwellenkonzentration für sCD40L von 5,0 µg/l wurden Patienten mit erhöhten sCD40L-Serumkonzentrationen als Hochrisiko-Population identifiziert (festgesetzter Risikoquotient 3,00 [1,35-6,71]; p = 0,009). Innerhalb der gesamten heterogenen Population von Patienten mit Brustschmerz, war die festgesetzte Schwellenkonzentration von 5,0 µg/l ebenfalls zuverlässig, um Patienten zu identifizieren, die ein hochgradiges Risiko für kardiologische Ereignisse während der nach folgenden 30 Tage aufwiesen (festgesetzter Risikoquotient 6,65 [3,18 bis 13,89]; p < 0,001). Die Fläche unter der ROC-Kurve betrug 0,75 [0,67-0,83] und ein maximierter Vorhersagewert wurde bei einer Schwellenkonzentration von 4,8 µg/l erreicht.

### Beispiel 2a: Angiographische Befunde und sCD40L-Konzentration

Die *Base* Line-Koronarangiogramme bei Patienten mit erhöhten sCD40L-Konzentrationen zeigten komplexere Charakteristika der Läsionen. Bei 40,6% der Patienten mit hohen sCD40L-Konzentrationen wurden Läsionen vom Typ B2+ oder C dokumentiert, während nur 27,5% der Patienten mit niedrigen sCD40L-Konzentrationen komplexere Läsionscharakteristika aufwiesen (p = 0,004).

Der *Base Line-Wert* für den TIMI-Durchfluß war bei 59,6% der Patienten mit hohen sCD40L-Konzentrationen und bei 58,9% der Patienten mit niedrigen sCD40L-Konzentrationen normal (p = 0,049). Ein TIMI-Durchfluß = 1 wurde für 7,8% der Patienten mit hohen sCD40L-Konzentrationen im Vergleich zu 5,7%, Patienten mit niedrigen sCD40L-Konzentrationen dokumentiert (p = 0,67).

Ein Thrombus war zum Zeitpunkt der Aufnahme bei 11,1 % der Patienten mit hohen sCD40L-Konzentrationen im Vergleich zu 4,8% Patienten mit niedrigen sCD40L-Konzentrationen sichtbar (p = 0,009). Alle Patienten mit sichtbarer Thrombusbildung wiesen sCD40L-Konzentrationen > 2,5 µg/l auf.

### Beispiel 3a: Wirkung von Abciximab auf die sCD40L-Konzentration

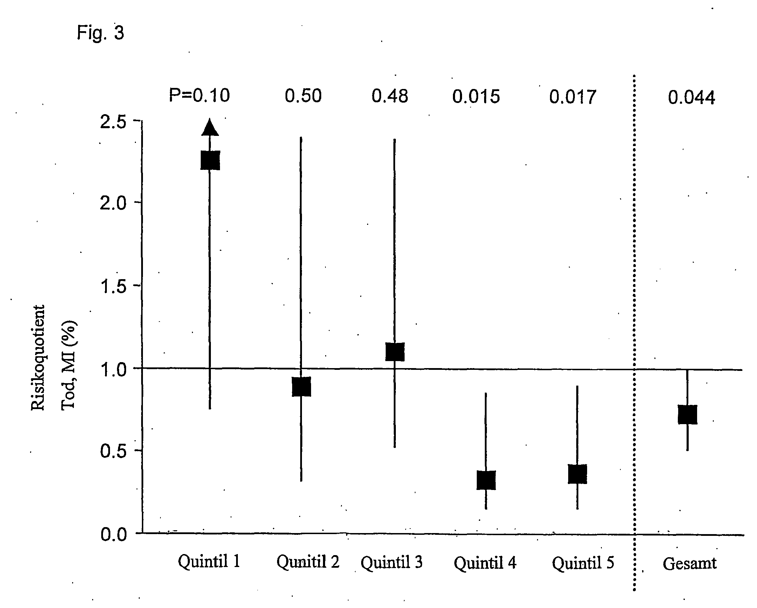

Eine logistische Regressionsanalyse wies auf einen signifikanten Zusammenhang zwischen der Wirksamkeit einer Behandlung mit Abciximab und der sCD40L-Konzentrationen hin (p < 0,001). Die Patienten wurden wie oben angegeben in Quintile eingeteilt. Für die ersten zwei Quintile wurden keine Unterschiede hinsichtlich eines kardialen Risikos bei einer Behandlung mit Placebo oder Abciximab beobachtet *(**Figur* 3). Eine signifikante und gleichermaßen ausgeprägte Reduktion kardialer Ereignisse wurde für die oberen beiden Quintile dokumentiert. Der Befund einer Änderung der Ungleichheit auf 0,35 zwischen den zweiten und dritten Quintilen legt eine günstige Schwellenkonzentration in diesem Bereich der sCD40L-Konzentrationen nahe. Demgemäß wurden die Kurven, welche die Häufigkeit eines kardialen Ereignisses in Gestalt eines tödlichen oder nicht-tödlichen Myokardinfarkts darstellen, unter Verwendung einer Schwellenkonzentration von 5,0 µg/l erzeugt. Ereignisse vor diesem Verfahren waren bei Patienten mit niedrigen sCD40L-Konzentrationen, die Placebo erhielten, selten (0,9%), während bei 3,4% der Patienten Ereignisse im Zusammenhang mit einer coronaren Intervention auftraten *(**Figur 4a**).* Bei Patienten mit niedrigen sCD40L-Konzentrationen wurde kein Unterschied zwischen Patienten, die Abciximab und denen, die Placebo erhielten, beobachtet (24 Stunden: 1,2% vs. 0,9%; 72 Stunden: 3,8% vs. 4,3%). Nur wenige zusätzliche Ereignisse wurden in den anschließenden 6 Monaten registriert. Die Gesamthäufigkeit betrug 7,1 % führte *(**Figur 4b**).*

Im Gegensatz dazu war die Häufigkeit von Anfällen signifikant höher bei Patienten mit hohen sCD40L-Konzentrationen, die Placebo erhalten hatten, sowohl vor PTCA (4,1%) als auch unter PTCA (9,0%), ebenso wie in der nachfolgenden Zeit nach der Entlassung, was zu einer Gesamthäufigkeit von 18,6% nach sechs Monaten führte. Ereignisse, die vor und unter PTCA auftraten, wurden durch Behandlung mit Abciximab wirksam auf 0,5% vor PTCA (Risikoquotient 0,12 [0,01-0,92]; p = 0,013) und 2,9% für PTCA-bezogene Ereignisse (Risikoquotient 0,19 [0,08-0,49]; p < 0,001) reduziert. Diese Verbesserung blieb über sechs Monate nach dem Ereignis bestehen, was zu einer kumulativen Häufigkeit der Ereignisse von 7,8% vs. 18,6% in der Placebo-Gruppe führte (Risikoquotient 0,37 [0,20-0,68]; p = 0,001). Dieser Wert ist vergleichbar mit dem, der bei Patienten mit niedrigen sCD40L-Konzentrationen (7,1 %) beobachtet wurde. Weiterhin war die Wirkung der Glycoprotein IIb/IIIa-Hemmung ohne das Auftreten einer Myokardnekrose offensichtlich. Bei TnT-negativen Patienten wurde mittels der sCD40L-Konzentration eine Untergruppe von Patienten identifiziert, die eine signifikante Reduktion kardialer Ereignisse zeigten, wenn sie Abciximab (2,8% vs. 10,2%; Placebo vs. Abciximab; p = 0,022) erhielten.

Die Auflösung eines Thrombus während der angeordneten Behandlung vor Koronarintervention war bei Patienten mit hohen sCD40L-Konzentrationen besonders auffällig, wenn sie mit Abciximab behandelt wurden, mit einer relativen Reduktion von 63% (Placebo: -21%; p < 0,01). Eine Thrombusbildung bei Patienten mit niedrigen sCD40L-Konzentrationen war selten und eine signifikante Auflösung wurde weder in der Placebogruppe (p = 0,75) noch in der Abciximab-Gruppe (p = 0,82) erreicht.

### Beispiel 4a: sCD40L als Marker einer Plättchen-Aktivierung in vivo

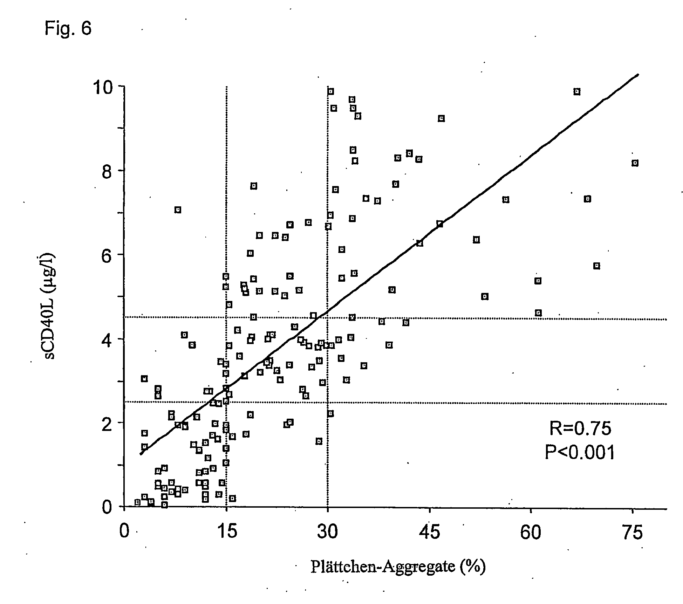

Um die Hypothese zu verifizieren, daß sCD40L tatsächlich einen Marker für die Plättchen-Aktivierung darstellt, wurde das Verhältnis der Plättchen-Aktivierung und der sCD40L-Serumkonzentrationen in einer Untergruppe von Patienten mit akutem Brustschmerz (n = 151) prospektiv untersucht. Das Ausmaß der Plättchen-Aktivierung war bei Patienten mit aktuten Coronarsyndromen im Vergleich sowohl zu Patienten mit stabiler koronarer Herzerkrankung als auch zu Patienten ohne koronare Herzerkrankung signifikant erhöht *(**Figur 5**).* Es wurde eine strenge Korrelation zwischen der Plättchen-Aktivierung, bestimmt mittels Monozyt-Plättchen-Agregate (%), und der sCD40L-Konzentration beobachtet (r = 0,75; p < 0,0001) *(**Figur 6*). Ähnliche Ergebnisse wurden für P-Selektin (p < 0,001) erhalten. Die Patienten wurden nach ihren gemessenen sCD40L-Konzentrationen in Tertile eingeteilt: (sCD40L 1) < 2,5 µg/l (n = 55), (sCD40L 2) 2,5-4,5 µg/l (n = 50), bzw. (sCD40L 3) > 4,5 µg/l (n = 56). Bei Patienten des ersten sCD40L-Tertils betrug der prozentuale Anteil von Monozyt-Plättchen-Aggregaten 11,3% [9,6-12,9]. In dem zweiten und dritten sCD40L-Tertil war die Plättchen-Aktivierung signifikant höher (22,3 [19,8-24,8] % bzw. 34,1 [30,0-38,3] %; p < 0,001). Besonders interessant ist, daß bei allen Patienten (mit Ausnahme eines Patienten) die sCD40L-Serumkonzentrationen > 4,5 µg/l aufwiesen, mindestens 15% der Monozyten mit Plättchen aggregiert waren, was auf eine deutliche Plättchen-Aktivierung bei diesen Patienten hinweist. Im Gegensatz dazu zeigten die Serumkonzentrationen an löslichem P-Selektin einen deutlich weniger ausgeprägten Zusammenhang mit der Plättchen-Aktivierung (r = 0,41 für Plättchen-Monozyt-Aggregate bzw. r = 0,36 die P-Selektin). In einer Multivarianz-Regressionsanalyse zeigte sich, daß eine Diabetes-Erkrankung (p = 0,015), eine Hypercholesterinämie (p = 0,006) und die sCD40L-Konzentration (p < 0,0001), nicht jedoch die Konzentration an löslichem P-Selektin signifikant mit der Plättchen-Aktivierung assoziiert war (Monozyt-Plättchen-Aggregate > 30%).

**Tabelle 1: Base Line-Charakteristika von sCD40L in der Placebo-Gruppe**

| | | |
|---|---|---|
| | sCD40L niedrig | sCD40L hoch |
| n | 323 | 221 |
| männlich | 73,3% | 70,2% |
| Alter | 60,5 ± 9,9 | 62,3 ± 10,5 |
| Anamnese | | |
| Angina mehr als 4 Wochen | 57,5% | 54,6% |
| Infarkt 14-30 Tage | 3,5% | 2,4% |
| Infarkt mehr als 30 Tage | 19,5% | 21,0% |
| PTCA | 19,1 % | 15,9% |
| CABG | 3,5% | 3,4% |
| Risikofaktoren | | |
| Diabetes | 12,1 % | 9,6% |
| Hypercholesterinämie | 32,5% | 33,6% |
| Bluthochdruck | 39,3% | 34,5% |
| Rauchre | 40,9% | 42,1% |
| Medikation vor Registrierung | | |
| Aspirin | 98,1% | 97,7% |
| Heparin i.v. | 99,2% | 98,6% |
| Nitrate i.v. | 98,8% | 99,7% |
| Beta-Blocker | 65,0% | 61,4% |
| Ca²⁺-Antagonisten | 56,0% | 55,2% |

**Tabelle 2: Tödlicher und nicht-tödlicher Myokardinfarkt innerhalb der ersten 6 Monate (Multivarianz-Regressionsanalyse)**

| **Variable** | **OR** | **95% CI** | **p-Wert** |
|---|---|---|---|
| Geschlecht | 0,91 | 0,68-1,39 | 0,16 |
| Alter mehr als 65 Jahre | 1,36 | 0,91-1,82 | 0,34 |
| Diabetes mellitus | 1,22 | 0,83-1,49 | 0,61 |
| Hypercholesterinämie | 0,90 | 0,68-1,13 | 0,59 |
| Bluthochdruck | 1,00 | 0, 89-1,04 | 1,00 |
| Geschichte einer CHD | 0,86 | 0,65-1,19 | 0,72 |
| ST-Senkung | 1,04 | 0,76-1,54 | 0,74 |
| TnT > 0,1 µg/l | 2,94 | 1,75-7,26 | < 0,001 |
| CRP > 10 mg/l | 2,03 | 1,11-3,59 | 0,018 |
| sCD40L > 5,0 µg/l | 2,71 | 1,51-5,35 | 0,001 |

### II. PIGF

PIGF wurde ursprünglich in der Plazenta identifiziert und stimuliert vaskuläres glattes Muskelwachstum, rekrutiert Makrophagen in arteriosklerotischen Läsionen, reguliert die Produktion von TNF-α und MCP-1 durch Makrophagen hoch und stimuliert die pathologische Angiogenese. Weit wichtiger, von der Inhibierung der Effekte von PIGF durch Blockieren seines Rezeptors Tyrosinkinase Flt-1 wurde experimentell gezeigt, daß dies sowohl arteriosklerotisches Plaque-Wachstum als auch die Verwundbarkeit durch die Inhibierung der entzündlichen Zellinfiltration unterdrückt. Diese Daten lassen vermuten, daß PIGF vielleicht als ein primärer inflammatorischer Anzeiger der arteriosklerotischen Plaque-Instabilität dient.

Daher wurde die prognostische Signifikanz von PIGF in Patienten mit akuten Koronarsyndromen unter der Verwendung der Daten der Patienten mit akuten Koronarsyndromen, die in die CAPTURE Untersuchung aufgenommen wurden (c7E3 "Anti Platelet Therapy in Unstable Refractory angina"), verwendet, und dann die diagnostische und prognostische Signifikanz in einer großen Patientenpopulation, die mit Schmerzen in der Brust eingeliefert wurden, vorläufig validiert. Die P1GF Serumspiegel wurden in 1088 Patienten mit akuten Koronarsyndromen aus der CAPTURE Untersuchung gemessen. Weiterhin wurde die diagnostische und prognostische Signifikanz von PIGF Serumspiegeln in einer heterogenen Gruppe von 619 Patienten mit akuten Schmerzen in der Brust vorläufig validiert. Die Inzidenz von Myokardinfarkt mit tödlichem oder nicht-tödlichem Ausgang wurde während der Folgezeit aufgezeichnet.

### 1. Patienten

Aufbau des Sets von Patienten mit akuten Koronarsyndromen. Die CAPTURE-Studie registrierte 1265 Patienten mit akuten Koronarsyndromen (61% männlich, im Alter von 61 ± 10 Jahren). Alle CAPTURE-Patienten klagten über wiederkehrende Brustschmerzen im Ruhezustand, assoziiert mit EKG-Änderungen während einer Behandlung mit intravenösem Heparin und Glycerintrinitrat. Die gesamte Patientenpopulation wurde vor der Randomisierung einer Koronarangiographie unterzogen, die das Auftreten einer deutlichen koronaren Arterienerkrankung mit auslösenden Läsionen von > 70 %, die für eine Angioplastie geeignet waren, signifikant aüfzeigte. Heparin von vor der Randomisierung bis mindestens 1 h nach der PTCA . Prozedur verabreicht. Bei allen Patienten waren innerhalb von 18 bis 24 Stunden nach Beginn der Behandlung koronare Interventionen vorgesehen. Die Patienten wurden einer Behandlung durch Abciximab oder Placebo zufallsverteilt zugeordnet. Primäre Endpunkte der Studie waren Mortalität und nicht-fataler Myocardinfarkt währen der 6-Monate Nachuntersuchungs-Periode. Serumproben wurden 8,7 [75% CI 3,6-11,3] Stunden nach Einsetzen der Symptome entnommen.

### 2. Validierung von Patienten mit akutem Brustschmerz

Eine separate Gruppe von 626 Patienten mit Brustschmerz (161 Frauen und 465 Männer, mittleres Alter 61 [38-82] Jahre), die in fortlaufender Reihe mit akutem Brustschmerz, der weniger als 12 Stunden (im Mittel 5,1 [2,1-10,4] Stunden) andauerte, in die Notaufnahme kamen, wurden als Set erstellt. Nicht berücksichtigt wurden Patienten mit einer charakteristischen ST-Hebung im Basis-EKG bzw. einem dokumentierten akuten Myokardinfarkten während der vorangegangenen 2 Wochen. Serumproben wurden zum Zeitpunkt der Aufnahme (vor Beginn der Behandlung) und 4 Stunden später gewonnen, auf Eis gehalten, innerhalb von 20 Minuten nach Probennahme zentrifugiert und zur späteren Analyse bei -80°C gelagert. Die Patienten wurden bis zu ihrer Entlassung aus dem Krankenhaus und 30 Tage danach beobachtet, um tödliche und nicht-tödliche Myokardinfarkte zu verzeichnen. Das Vorhandensein einer koronaren Arterienerkrankung wurde durch eines der folgenden Kriterien nachgewiesen: EKG-Anzeichen auf eine Myokard-Ischämie (neue Änderungen in der ST-Strecke oder Inversion der T-Welle), eine koronare Herzerkrankung in der Anamnese (Myocardinfarkt oder koronare Revaskularisierung, ein positiver Belastungstest oder Einengung des Durchmessers einer Hauptkoronararterie um mindestens 50% in einem früheren Angiogramm). Patienten ohne koronare Herzerkrankung hatten ein normales Koronar-Angiogramm aufzuweisen.

Primäre Endpunkte der Studie waren Mortalität, Myokardinfarkt oder die Notwendigkeit zu sofortiger Intervention (Angioplastie, Bypass-Operationen der Koronararterie) aufgrund einer Instabilität während 30 Tagen oder 6 Monaten. Bei Patienten, die während des Krankenhausaufenthalts einen Herzinfarkt erlitten hatten, wurde ein solcher diagnostiziert, wenn ihre Werte der Kreatinkinase-Enzymaktivität in mindestens zwei Proben mehr als dreimal so hoch waren wie die obere Grenze des Normalbereichs oder wenn ihr EKG neue deutliche Q-Zacken in mehr als zwei aufeinanderfolgenden Intervallen aufwiesen. Diese strikte Definition wurde gewählt, um jeden unbedeutend geringen Anstieg der Kreatinkinase nach PTCA auszuschließen. Bei Patienten mit einem Myokardinfarkt nach der Entlassung wurde ein solcher definiert, wenn ihre Werte der Kreatinkinase-Enzymaktivität mehr als doppelt so hoch waren, wie die obere Grenze des Normalbereichs oder wenn ihr EKG neue deutliche Q-Zacken in zwei oder mehr aufeinanderfolgenden Intervallen aufwies. Der sekundäre Endpunkt war die symptomatische koronare Restenose der behandelten Läsion mit einem Stenosedurchmesser ≥ 70% und die Notwendigkeit zu wiederholter Revaskularisierung während der nachfolgenden 6 Monate.

### 3. Biochemische Analyse

Serumproben wurden zentral bei -80°C gelagert. Die Bestimmung der kardialen Marker wurde in Unkenntnis der Krankengeschichte der Patienten und der angeordneten Behandlung im Forschungslabor der Universität Frankfurt durchgeführt. Die Serumspiegel von P1GF und VEGF wurden mittels ELISA (R&D Systems, Wiesbaden) gemessen. Zur Quantifizierung von kardialem Troponin T (TnT) wurde ein Ein-Schritt-Enzym-Immuntest auf der Grundlage der Elektrochemilumineszenz-Technologie (Elecsys 2010, Roche Diagnostics) verwendet. Hoch-sensitives C-reaktives Protein (hsCRP) wurde mit Hilfe des Behring BN II Nephelometers (Behring Diagnostics) gemessen. Ein diagnostischer Schwellenwert von 10,0 mg/l wurde verwendet [9, 18].

### 4. Quantitative Bestimmung von P1GF

Die P1GF-Konzentration wurde unter Verwendung der Sandwich-Enzym-Immuntest-Technik (R&D Systems, Wiesbaden) bestimmt. Eine Mikrotiterplatte wurde mit einem polyklonalen Antikörper, der spezifisch gegen PIGF gerichtet war, beschichtet. Standards und Proben wurden in die Näpfe pipettiert und vorhandenes PIGF wurde durch den immobilisierten Antikörper gebunden. Nachdem nicht-gebundenes Material weggewaschen worden war, wurde ein Enzym-gekoppelter polyklonaler Antikörper, der spezifisch gegen PIGF gerichtet war, zu den Näpfen gegeben. Nach einem Waschschritt, um nicht gebundenes Antikörper-Enzym-Reagenz wegzuwaschen, wurde eine Substrat-Lösung zu den Näpfen gegeben, und die Farbe entwickelte sich im Verhältnis zur Menge an PIGF, die im ersten Schritt gebunden wurde. Die Farbentwicklung wurde abgestoppt und die Farbintensität wurde gemessen.

### 5. Statistische Verfahren

Nach einer blinden Beurteilung der biochemischen Marker wurden die Testergebnisse mit der Datenbank abgeglichen. Um Patienten mit verschiedenen Graden eines kardiologischen Risikos unterscheiden zu können, wurde eine orientierende Datenanalyse gewählt. Das *Cox Proportional-Hazards Regression Model* wurde verwendet, um das relative Risiko für kardiovaskuläre Vorkommnisse abzuschätzen, und die Patienten wurden gemäß der P1GF-Konzentration der Quintilen eingeteilt. Die Post-hoc Analyse der Quintilen wurde unter Verwendung des *Cox Proportional-Hazards Regression Model* mit den P1GF Quintilen als eine kategorische Variable durchgeführt, und Patienten in dem ersten Quintil dienten als Referenz. *Receiver Operating Characteristics* (ROC) *Curve Analysis* über den dynamischen Bereich des PIGF-Tests wurde verwendet, um die Schwellenkonzentration für P1GF zu identifizieren, die den höchsten Vorhersagewert für die Risikostratifikation von Patienten mit akuten Koronarsyndromen lieferte. Der Effekt der Basislinien-Charakteristika (wobei p = 0,10 erforderlich war, um eine Variable in das Model einzubringen) und anderer biochemischer Marker auf jede beobachtete Zusammenhänge zwischen P1GF Spiegeln und kardiovaskulären Vorkommnissen wurde unter der Verwendung schrittweiser *Cox Proportional-Hazards Regression Model* durchgeführt. Alle Ergebnisse der kontinuierlichen Variablen sind als Mittel ± Standardabweichung ausgedrückt. Der Vergleich zwischen den Gruppen wurde durch einen t-Test (zweiseitig) analysiert. Der Vergleich von kategorischen Variablen wurde durch den Pearson χ² Test generiert. p-Werte < 0,05 wurden als statistisch signifikant betrachtet. Alle Analysen wurden mit SPSS 11.0 (SPSS Inc., Chicago) durchgeführt.

### Beispiel 1b: Zusammenhang zwischen kardialem Risiko und P1GF-Konzentration

Die Ausgangscharakteristika der für diese Studie ausgewählten Population (n = 1088, 86% der CAPTURE-Patienten) unterschied sich nicht von der Gesamtpopulation der Studie im Hinblick auf Alter, Geschlecht, kardiovaskuläres Risikoprofil und begleitende Behandlung vor und nach der zufälligen Auswahl. Die Reduktion kardialer Ereignisse in der Abciximab-Gruppe der Population war mit der gesamten CAPTURE-Population vergleichbar sowohl vor PTCA (2,2% Placebo versus 0,9% Abciximab; p = 0,07), als auch nach PTCA (7,9% versus 3.5%; p = 0,001), und bei 30 Tagen (9,0% versus 4,2%; p = 0,001).

P1GF war in den *Base Line*-Serumproben von 95,6 % der Patienten der Studie, mit einem Mittelwert von 23,0 ng/l (Bereich 7,0-181,2) nachweisbar. Der P1GF Serumspiegel korrelierte nicht mit den gemessenen Konzentrationen an Troponin T (r = 0,14) und VEGF Spiegeln (r = 0.07), zeigte jedoch eine signifikante Korrelation mit hsCRP Serumspiegeln (r = 0.48) (Figur 7). Der PIGF Serumspiegel unterschied sich nicht zwischen Troponin T-positiven und Troponin T-negativen Patienten, wohingegen die hsCRP Serumspiegel in Troponin T-positiven Patienten signifikant höher waren (Figur 8). Die Patienten der Placebo-Gruppe (n=547) wurden nach ihren gemessenen PIGF Serumspiegeln in Quintile eingeteilt: Jeweils (PIGF 1) < 13,3 ng/l n = 109), (P1GF 2) 13,4-19,2 ng/l (n = 110), (PIGF 3) 19,3-27,3 ng/l (n = 110), (PIGF 4) 27,3-40,0 ng/L (n = 109), und (PIGF 5) > 40,0 ng/l (n = 109). Während der ersten 24 Stunden waren die kombinierten Endpunkte Mortalität und nicht-tödlicher Myokardinfarkt zwischen den P1GF-Quintilen nicht unterschiedlich (p = 0,11) (Figur 9). Zu späteren Zeitpunkten (72 Stunden, 30 Tage, 6 Monate) zeigten die Vorkommnis-Raten signifikante Unterschiede zwischen den PIGF Quintilen. Bei 72 Stunden Nachfolgeuntersuchung, waren die Vorkommnis-Raten in der vierten und fünften Quintil signifikant höher, wenn mit der ersten Quintil verglichen (jeweils p = 0,038 und p = 0,011). Während der anschließenden 6 Monate Nachfolgeuntersuchung, divergierten die Vorkommnis-Raten weiter, was zu signifikanten Unterschieden für die vierte und fünfte Quintil bei 30 Tagen (jeweils p = 0,005 und p = 0,017) und 6 Monate Nachfolgeuntersuchung (jeweils p = 0,002 und p = 0,001) führte.

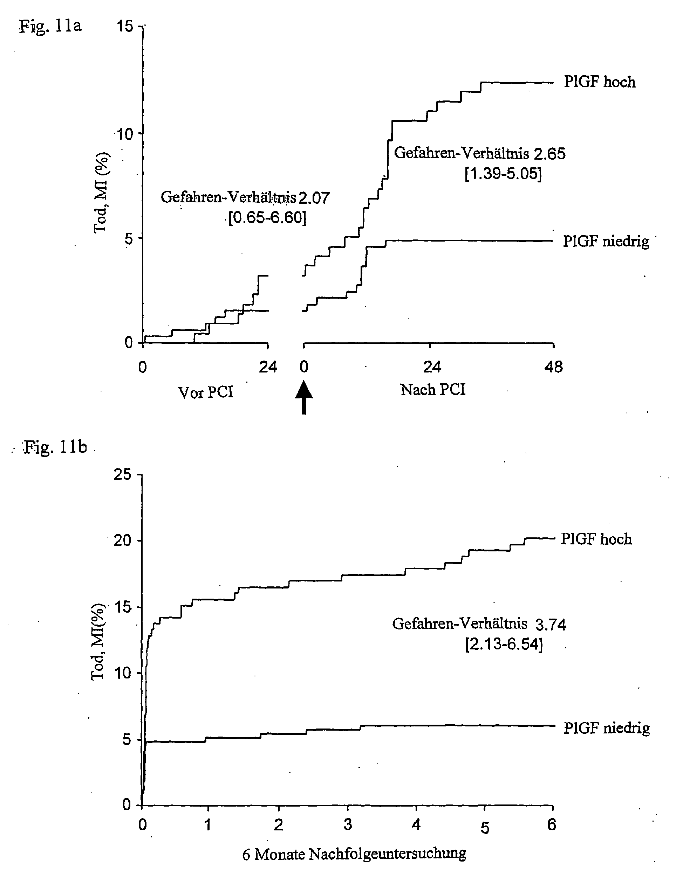

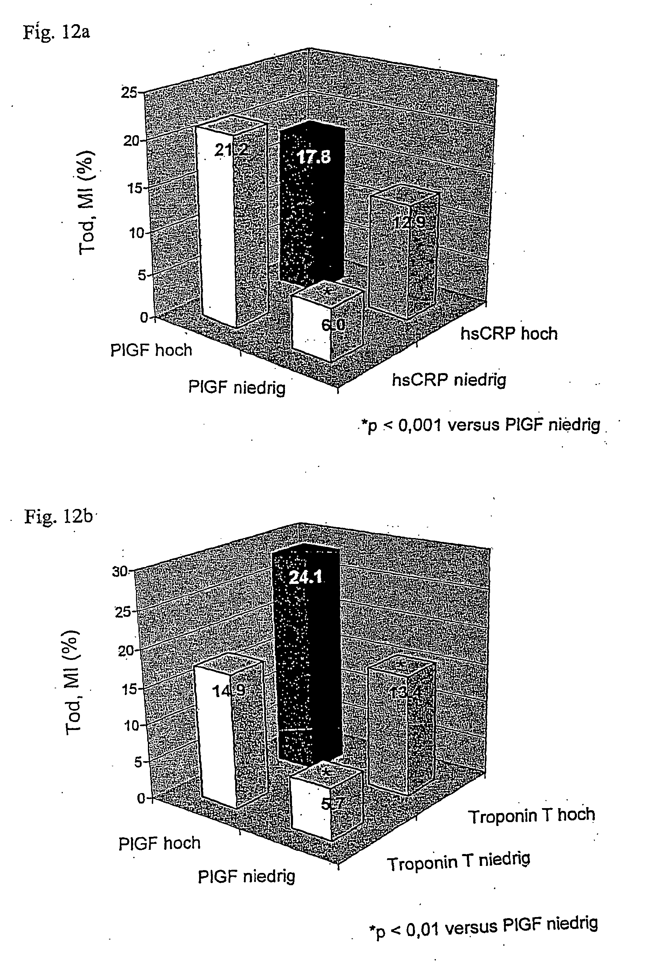

Die *receiver operating characteristics* Kurven-Analyse bestätigte einen Schwellenwert von 27,0 ng/l für den maximierten Vorhersagewert von P1GF (Figur 10). Auf der Grundlage diese Schwellenwertes, wiesen 223 Patienten (40,8 %) P1GF Serumspiegel oberhalb oder gleich zu 27,0 ng/l auf, und 324 Patienten unterhalb von 27,0 ng/l. Wie *Tabelle* 3 zeigt, traten geringe Unterschiede in den Ausgangscharakteristika der beiden Gruppen auf. Patienten mit erhöhten P1GF Serumspiegeln waren häufiger Diabetiker und wiesen Bluthochdruck auf und hatten signifikant höhere hsCRP Serumspiegel (Tabelle 3). Bei Patienten mit hohem P1GF Serumspiegel, waren die kombinierten Endpunkte tödlicher und nicht-tödlicher Myokardinfarkt signifikant verschieden von Patienten mit niedrigem PIGF Serumspiegel. Nach 72 Stunden (einschließlich koronarer Interventionen bei allen Patienten) hatten 12,1 % der Patienten mit hohem P1GF Serumspiegel ein negatives Ereignis, verglichen mit 4,9 % für Patienten mit niedrigem P1GF Serumspiegel (p = 0,002) (Figur 11a). Während der 6-monatigen Folgezeit divergierten die Kurven, welche die Häufigkeit eines Ereignisses anzeigen zwischen Patienten mit hohen bzw. niedrigen P1GF Serumspiegeln weiter (Figur 11b). Signifikante Unterschiede gab es sowohl nach 30 Tagen (15,8 % versus 3,6 %; p = 0,001) als auch nach 6 Monaten (20,3 % versus 4,9 %; p < 0,001). Trotz der relativ niedrigen Mortalität der CAPTURE-Gruppe unterschied sich die Endpunktmortalität nach 6 Monaten signifikant zwischen den beiden Gruppen (4,0 % versus 0,9 %; p = 0,021). In a multivarianten Analyse, die Basislinie Charakteristika und biochemische Marker (Troponin T, VEGF, hsCRP), verblieb P1GF als ein unabhängiger wirksamer Prädiktor erhöhten kardialen Risikos sowohl bei 30 Tagen Nachfolgeuntersuchung (eingestelltes Gefahren-Verhältnis 3,34 [95% CI 1,79- 6,24]; p < 0,001)-und bei 6 Monaten Nachfolgeuntersuchung (eingestelltes Gefahren-Verhältnis 3,58 [95 % CI 1,48 - 7,72]; p < 0,001) (Tabelle 4). Aufteilen der Patienten in vier Gruppen, basierend auf ihren P1GF und hsCRP Spiegeln ergab, daß P1GF eine Untergruppe von Patienten mit niedrigen hsCRP Serumspiegeln identifizierte, die an einem signifikant erhöhten kardialen Risiko litten. Patienten mit niedrigem hsCRP Serumspiegel, jedoch P1GF Serumspiegeln oberhalb 27,0 ng/l wiesen ein signifikant höheres Risiko auf, als Patienten, die niedrige Spiegel für sowohl hsCRP und P1GF aufwiesen (23,6 % versus 3,9 %; p = 0,001) (Figur 12a).

Weiterhin war der prädiktive Wert von PIGF unabhängig von myokardialer Nekrose. Hohe PIGF Serumspiegel zeigten ein erhöhte kardiales Risiko sowohl in Troponin T-positiven Patienten (15.4 % versus 4.1 %; P=0.005) als auch in Troponin T-negativen Patienten an (26,1 % versus 10,1 %; p = 0,001) (Figur 12b).

### Beispiel 2b: Wirkung von Abciximab im Hinblick auf PIGF-Serumspiegel

Eine logistische Regressionsanalyse wies auf einen Grenz-signifikanten Zusammenhang zwischen der Wirksamkeit einer Behandlung mit Abciximab und der PIGF-Konzentrationen hin (p = 0,043). Patienten mit erhöhten P1GF Serumspiegeln, die Abciximab erhielten hatten ein signifikant geringeres Risiko bei 30 Tage Nachfolgeuntersuchung (eingestelltes Gefahren-Verhältnis 0,38 [0,19 - 0,74]; p = 0,005). Dieser signifikante Unterschied wurde bei 6 Monaten Nachuntersuchung (0,57 [0,34 - 0,96]; p = 0,037) beibehalten. Im Unterschied dazu erhielten Patienten mit niedrigen PIGF Serumspiegeln keinen signifikant therapeutischen Vorteil von einer Behandlung mit Abciximab (30 Tage Nachfolgeuntersuchung: eingestelltes Gefahren-Verhältnis 0,59 [0,27 -1,33]; p = 0,23).

### Beispiel 3b: Entlassungs PIGF Serumspiegel sind für den Langzeitausgang prädiktiv

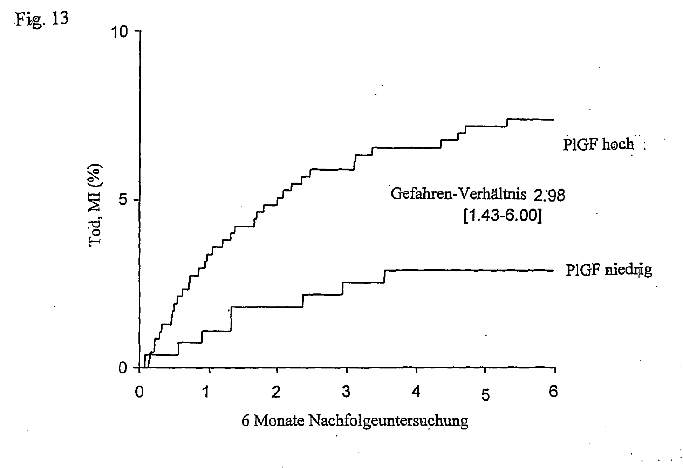

Eine zweite Blutprobe, die vor der Entlassung abgenommen wurde (7,2 ± 4,5 Tage nach Randomisierung), war für 489 Patienten der 547 Placebo-Patienten erhältlich (89,4%). Die P1GF Serumspiegel nahmen von einem Mittel von 27,12 ± 19,56 ng/l bei Basislinie auf 23,4 ± 26,2 ng/l bei Entlassung ab (p = 0,012). Für Patienten mit einem PIGF Serumspiegel oberhalb 27,0 ng/l bei Entlassung war das Auftreten von Mortalität und nicht-fatalem myokardialem Infarkt signifikant höher, wenn mit Patienten mit niedrigem PIGF Serumspiegel verglichen, sowohl bei 30 Tage (4,6 % versus 0,8 %; p = 0,019) und 6 Monate Nachfolgeuntersuchung (7,4 % versus 2,2 %; p = 0,005) (Figur 13).

### Beispiel 4b: Validierung des PIGF-Schwellenwerts in Patienten mit akuten Brustschmerzen

Von 626 Patienten mit akuten Brustschmerzen, litten 308 Patienten an einem akuten Koronarsyndrom (117 Patienten hatten einen nicht-ST-Erhöhungs myokardialen Infarkt). Die anderen Patienten wurden den folgenden Diagnosen zugeteilt: n = 91 stabile Angina, n = 10 pulmonare Embolie, n = 11 kongestives Herzversagen, n = 7 Myokarditis, und n = 199 kein Hinweis auf Herzerkrankung. Die PIGF Serumspiegel waren in Patienten mit akuten Koronarsyndromen signifikant erhöht (Mittel 28,3 [95 % CI 21,3 - 2,2] ng/l), wenn jeweils mit Patienten mit stabiler Angina verglichen (Mittel 16,2 [95 % CI 13,8 - 18,6 ng/l; p = 0,001), und Patienten ohne Anzeichen auf Herzerkrankung (Mittel 9,6 [95 % CI 10,4 - 12,9 ng/l; p = 0,001). Die P1GF Serumspiegel in Patienten mit nicht-ST-Erhöhungs myokardialem Infarkt unterschieden sich nicht signifikant von PIGF Serumspiegeln in Patienten mit instabiler Angina (30,5 [95 % CI 26,9 - 34,1] versus 28,3 [95 % CI 21,3 - 32,2] ng/1; p = 0,42) Die 97,5 Prozent obere Referenzgrenze in Patienten ohne Hinweis auf Herzerkrankung war 24,9 ng/l und die 99 Prozent obere Referenzgrenze war 27,3 ng/l. Der P1GF Serumspiegel korrelierte nicht mit Markern der Nekrose (Troponin T [r = 0,07]), korrelierte jedoch signifikant mit Entzündungsmarkern (C-reaktivem Protein [r = 0,43]).

In Patienten mit akuten Koronarsyndromen, wiesen 44,8 % der Patienten PIGF Serumspiegel oberhalb der 99 Prozent obere Referenzgrenze auf. Unter der Verwendung des Schwellenwerts für PIGF von 27,0 ng/l, unterlagen Patienten mit erhöhtem PIGF Serumspiegel einem signifikant höheren Risiko für Tod und myokardialem Infarkt (eingestelltes Gefahren-Verhältnis 2,97 [95 % CI 1,74 bis 9,06; p = 0,014). Innerhalb der gesamten heterogenen Population von Patienten mit Brustschmerzen identifizierte der Schwellenwert von 27,0 ng/l auch zuverlässig Patienten, die dem höchsten Risiko für Tod und myokardialem Infarkt ausgesetzt waren (eingestelltes Gefahren-Verhältnis 4.95 [95 % CI 2.50 to 9.79; P0.001).

### Zusammenfassung der Beispiele 1b bis 4b

In Patienten mit ACS, korreliert PIGF nicht mit VEGF, Troponin T, und ST-Segment Veränderungen, zeigte jedoch eine signifikante Korrelation mit hsCRP (p = 0,001). Patienten mit erhöhtem PIGF Serumspiegel (> 27,0 ng/l; 40,8 %) erfuhren ein erheblich erhöhtes kardiales Risiko (Tod und nicht-fataler myokardialer Infarkt) sowohl bei 30 Tagen (eingestelltes Gefahren-Verhältnis 3,34 [95 % CI 1,79 - 6,24]; p = 0,001) und bei 6 Monaten (eingestelltes Gefahren-Verhältnis 3,58 [95 % CI 1,48 - 7,72]; p = 0,001) nach ACS. Die PIGF Serumspiegel waren spezifisch in Patienten mit niedrigen hsCRP Spiegeln informativ. Die vorläufige Validierung in Patienten mit akuten Brustschmerzen ergab, daß P1GF Serumspiegel > 27 ng/l identifizierte zuverlässig diejenigen Patienten, die dem höchsten Risiko für Tod und myokardialem Infarkt (eingestelltes Gefahren-Verhältnis 4,95 [95 % CI 2,50 bis 9,79; p = 0,001) ausgesetzt waren. Das erhöhte Risiko in Patienten mit hohem PIGF Serumspiegel wurde durch die Behandlung mit dem Glycoprotein IIb/IIIa Rezeptorinhibitor Abciximab verringert (eingestelltes Gefahren-Verhältnis 0,38 [0,19 - 0,74]; p = 0,005).

**Tabelle 3: Base Line-Charakteristika gemäß dem P1GF Status für die Placebogruppe des CAPTURE Versuchs (n = 547)**

| | P1GF niedrig | P1GF hoch | p-Wert |
|---|---|---|---|
| n | 324 | 223 | |
| männlich | 71,4 % | 69,2 % | 0,34 |
| Alter | 61,4 ± 10,5 | 62,3 ± 10,5 | 0,32 |
| Troponin T ≥ 0,1 µg/l | 33,8 % | 40,4 % | 0,12 |
| CRP ≥ 10,0 µ/l | 29,1 % | 67,7 % - | < 0,001 |
| ST-Segment Depression | 46,0 % | 52,1 % | 0,18 8 |
| T-Wellen Inversion | 51,4 % | 52,1 % | 0,93 |
| Vorgeschichte von | | | |
| Angina > 4 Wochen | 55,3% | 57,4% | 0,64 |
| Infarkt < 30 Tage | 12,5% | 13,6 % | 0,84 |
| CABG | 3,2 % | 3,7 % | 0,88 |
| Risikofaktoren | | | |
| Diabetes | 8,2 % | 12,5 % | 0,034 |
| Bluthochdruck | 33,4 % | 39,9 % | 0,019 |
| akuter Raucher | 39,6 % | 41,8 % | 0,48 |
| Medikation vor Registrierung | | | |
| Aspirin | 97,9 % | 98,1 % | 1,00 |
| Heparin i.v. | 99,0 % | 98,9% | 0,98 |
| Nitrate i.v. | 99,4% | 99,3% | 1,00 |
| Beta-Blocker | 63,5% | 62,9% | 0,91 |

**Tabelle 4: Multivariantes Cox proportionales-Gefahren-Regressionsmodell für tödlichen und nicht-tödlichen Myokardinfarkt innerhalb der ersten 6 Monate der Nachuntersuchung, abgeleitet von der Placebogruppe der CAPTURE Untersuchung**

| **Variable** | **eingestelltes Gefahren-Verhältnis** | **95% CI** | **p-Wert** |
|---|---|---|---|
| Geschlecht | 0,95 | 0,72 bis 1,68 | 0,38 |
| Alter > 65 Jahre | 1,22 | 0,65 bis 1,47 | 0,50 |
| Diabetes mellitus | 1,22 | 0,83 bis 1,49 | 0,61 |
| Hypercholesterinämie | 0,90 | 0,68 bis 1,13 | 0,59 |
| Akuter Raucher | 0,66 | 0,42 bis 1,25 | 0,18 |
| Bluthochdruck | 1,04 | 0,91 bis 1,25 | 0,95 |
| Geschichte einer Koronar- | 0,86 | 0,65 bis 1,19 | 0,72 |
| Revaskularisierung | | | |
| ST-Senkung | 0,96 | 0,55 bis 1,42 | 0,81 1 |
| hsCRP > 10,0 mg/l | 0,95 | 0,62 bis 1,57 | 0,88 |
| Troponin T > 0,1 µg/l | 1,76 | 0,98 bis 3,46 | 0,084 |
| VEGF > 300 ng/l | 2,16 | 1,05 bis 4,11 | 0,031 |
| PIGF > 27,0 ng/l | 3,58 | 1,48 bis 7,72 | < 0,001 |

### III. PAPP-A und Kombinationen

### Die Verwendung von PAPP-A als einziger Marker oder in Kombination mit anderen Markern ohne eine Verwendung von PIGF ist nicht Gegenstand der vorliegenden Erfindung.

Es wurde die prognostische Signifikanz von PAPP-A in Patienten mit akuten Koronarsyndromen unter der Verwendung der Daten der Patienten mit akuten Koronarsyndromen, die in die CAPTURE Untersuchung aufgenommen wurden (c7E3 "Anti Platelet Therapy in Unstable Refractory angina"), verwendet, und dann die diagnostische und prognostische Signifikanz in einer großen Patientenpopulation, die mit Schmerzen in der Brust eingeliefert wurden, vorläufig validiert. Die PAPP-A Serumspiegel wurden in Patienten mit akuten Koronarsyndromen aus der CAPTURE Untersuchung gemessen. Die Inzidenz von Myokardinfarkt mit tödlichem oder nicht-tödlichem Ausgang wurde während der Folgezeit aufgezeichnet.

### 1. Patienten

Aufbau des Sets von Patienten mit akuten Koronarsyndromen. Die CAPTURE-Studie registrierte 1265 Patienten mit akuten Koronarsyndromen (61 % männlich, im Alter von 61 ± 10 Jahre). Alle CAPTURE-Patienten klagten über wiederkehrende Brustschmerzen im Ruhezustand, assoziiert mit EKG-Änderungen während einer Behandlung mit intravenösem Heparin und Glycerintrinitrat. Die gesamte Patientenpopulation wurde vor der Randomisierung einer Koronarangiographie unterzogen, die das Auftreten einer deutlichen koronaren Arterienerkrankung mit auslösenden Läsionen von > 70 %, die für eine Angioplastie geeignet waren, signifikant aufzeigte. Heparin von vor der Randomisierung bis mindestens 1 h nach der PTCA Prozedur verabreicht. Bei allen Patienten waren innerhalb von 18 bis 24 Stunden nach Beginn der Behandlung koronare Interventionen vorgesehen. Die Patienten wurden einer Behandlung durch Abciximab oder Placebo zufallsverteilt zugeordnet. Primäre Endpunkte der Studie waren Mortalität und nicht-fataler Myokardinfarkt während der 6-Monate Nachuntersuchungs-Periode. Myokardinfarkt während des Krankenhausaufenthaltes, einschließlich der Angioplastie-Verfahren, wurde durch Werte der CK Enzymaktivität von mehr als dreifach der oberen Grenze von normal in mindestens zwei Proben und/oder neue signifikante Q-Zacken in zwei oder mehr kontinuierlichen Leads definiert. Myokardinfarkt nach Entlassung wurde definiert als Werte der CK Enzymaktivität von mehr als zweifach der oberen Grenze von normal in mindestens zwei Proben und/oder neue signifikante Q-Zacken in zwei oder mehr kontinuierlichen Leads definiert. Weil von anderen Markern, wie zum Beispiel Troponin T (TnT) und löslicher CD40 Ligand (sCD40L), gezeigt wurde, daß diese mit dem Behandlungseffekt des Glycoprotein IIb/IIIa Rezeptor Antagonisten Abciximab interferieren, wurde die vorliegende Analyse auf Placebo Patienten mit erhältlichen Blutproben (n=547; 86% der Placebo Patienten) eingeschränkt. Blutproben wurden 8,7 ± 4,9 Stunden nach Einsetzen der Symptome gesammelt.

### 2. Biochemische Analyse

Serumproben wurden zentral bei -80°C gelagert. Die Bestimmung der kardialen Marker wurde in Unkenntnis der Krankengeschichte der Patienten und der angeordneten Behandlung im Forschungslabor der Universität Frankfurt durchgeführt. Die Serumspiegel von PIGF und VEGF wurden mittels ELISA (R&D Systems, Wiesbaden) gemessen. Zur Quantifizierung von kardialem Troponin T (TnT) wurde ein Ein-Schritt-Enzym-Immuntest auf der Grundlage der Elektrochemilumineszenz-Technologie (Elecsys 2010, Roche Diagnostics) verwendet. Hoch-sensitives C-reaktives Protein (hsCRP) wurde mit Hilfe des Behring BN II Nephelometers (Behring Diagnostics) gemessen. Ein diagnostischer Schwellenwert von 10,0 ng/l wurde verwendet. Hoch-sensitives Interleukin-10 (IL-10), Vascular Endothelial Growth Factor (VEGF), und sCD40L wurden mittels ELISA (beide R&D Systems, Wiesbaden, DE) gemessen. Wir verwendeten die folgenden, früher etablierten diagnostischen Schwellenwerte: 5.0 ug/L für sCD40L, 300 ng/L für VEGF, 3.5 ng/L für IL-10. Kardialer PAPP-A wurde unter der Verwendung eines Ein-Schritt-Enzym-Immuntest auf der Grundlage der Elektrochemilumineszenz-Technologie bestimmt (Elecsys 2010, Roche Diagnostics, Mannheim, DE). Unter der Verwendung von internen Kontrollen war die gesamte Ungenauigkeit für PAPP-A über die 8-Wochen-Zeitdauer 8,5%.

### 3. Statistische Verfahren

Die Patienten wurden gemäß der PAPP-A Plasma Konzentrationen der Quintilen eingeteilt. Für jeden Zeitpunkt (24 h, 72 h, 30 Tage und 6 Monate), wurde das logistische Regressionsmodell dazu verwendet, um das relative Risiko für Tod und Myokardinfarkt zu bestimmen. Der Effekt der Basislinien-Charakteristika und anderer biochemischer Marker auf jede beobachtete Zusammenhänge zwischen PAPP-A Spiegeln und kardiovaskulären Vorkommnissen wurde unter der Verwendung schrittweiser *Cox Proportional-Hazards Regression Model* durchgeführt Alle Ergebnisse der kontinuierlichen Variablen sind als Mittel ± Standardabweichung ausgedrückt. Der Vergleich zwischen den Gruppen wurde durch einen t-Test (zweiseitig) analysiert. Der Vergleich von kategorischen Variablen wurde durch den Pearson χ² Test generiert. p-Werte < 0,05 wurden als statistisch signifikant betrachtet. Alle Analysen wurden mit SPSS 11.5 (SPSS Inc., Chicago) durchgeführt.

### Zusammenfassung der Ergebnisse

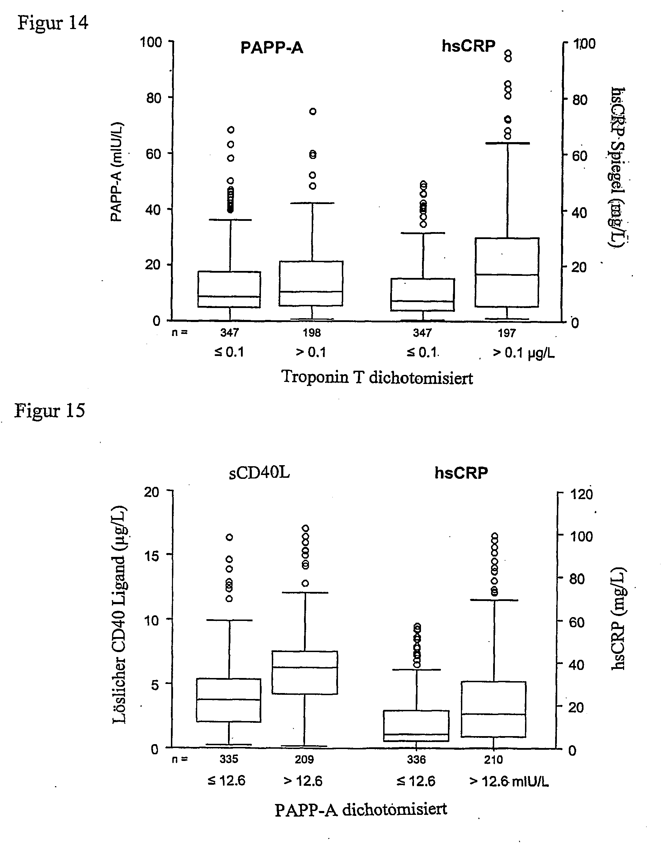

Basislinien PAPP-A Plasmaspiegel zeigten einen mittleren Spiegel von 14,8 ± 13,8 mIU/l (Bereich 0,2 bis 105,4). Wenn die PAPP-A Plasmaspiegel waren mit herkömmlichen Risikomarkem verbunden, PAPP-A Konzentrationen korrelierten nicht mit TnT Spiegeln (Spearman Reihe Korrelationskoeffizient r = 0,11; P = 0,16) und waren ähnlich in Patienten mit hohen TnT Plasmaspiegeln und in Patienten mit niedrigen TnT Plasmaspiegeln (Figur 14). Ähnlich zeigten VEGF (r = 0,08; P = 0,07) und IL-10 Plasmaspiegel (r = -0,04; P = 35) keine Assoziierung mit PAPP-A Plasmaspiegeln. Im Unterschied dazu waren die hsCRP Plasmaspiegel signifikant höher in Patienten mit erhöhtem TnT Plasmaspiegel. Die bivariante Korrelationsanalyse ergab eine signifikante Korrelation zwischen PAPP-A und hsCRP sowie zwischen PAPP-A und sCD40L, obwohl die Korrelationskoeffizienten mit r = 0,21 für hsCRP (P = 0,001) und r = 0,18 für sCD40L (P = 0,001) gering waren. Jedoch, wenn die Analyse auf Patienten ohne myokardiale Nekrose (keine Troponin-Erhöhung) begrenzt wurde, wurde die Korrelation zwischen hsCRP und PAPP-A deutlicher, mit einem r Wert von 0,68 (P = 0,001). Konsequenterweise wiesen Patienten mit erhöhten PAPP-A Spiegeln jeweils signifikant höhere hsCRP und sCD40L Spiegel auf (Figur 15).

### Interaktion zwischen PAPP-A Plasmaspie^{g}eln und kardialem Risiko

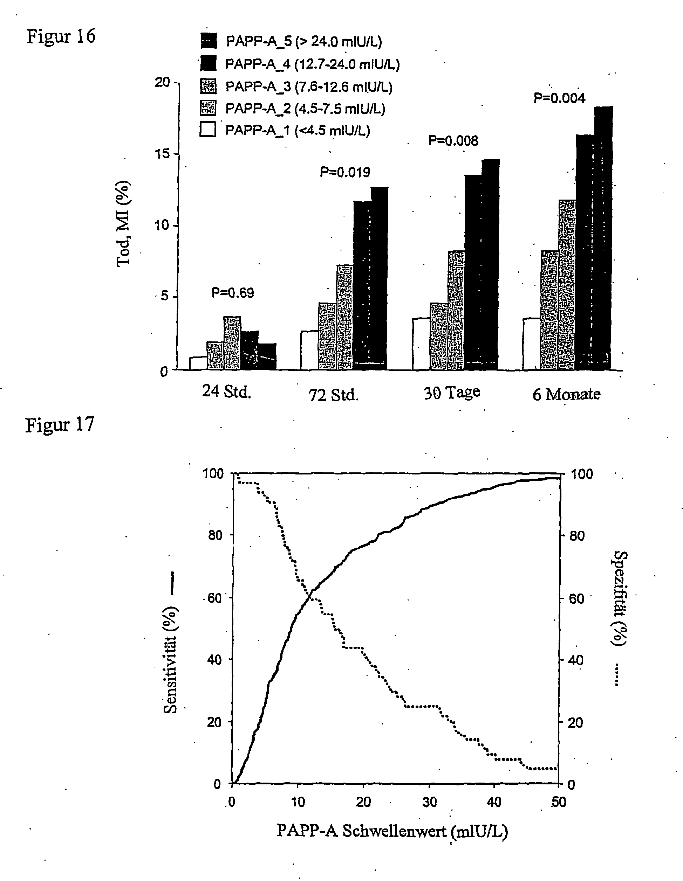

Patienten wurden in Quintilen nach ihrem gemessenen PAPP-A Spiegeln: jeweils (PAPP-A 1) < 4.5 mIU/L (n = 111), (PAPP-A-2) 4,5 - 7,5 mIU/L (n = 108), (PAPP-A-3) 7,6 - 12,6 mIU/l (n = 109), (PAPP-A_4) 12,7 - 24,0 mIU/l (n = 110) und (PAPP-A_5) > 24,0 mIU/I (n = 1 09) stratifiziert. Für dieanfängliche 24-Stunden Periode unterschieden sich die kombinierten Endpunkte Mortalität und nicht-fataler Myokardinfarkti nicht zwischen den Quintilen (P = 0,69) (Figur 16). Für die 72-Stunden Nachfolge einschließlich peri-interventionellen Vorkommnisse erreichten die Unterschiede in den kardialen Ereignissen zwischen den Quintilen erreichten Spiegel statistischer Signifikanz (P = 0,019). Während der 30-Tage und 6-Monate Nachfolge fuhren die Ereignisraten-Kurven fort, voneinander abzuweichen, was zu hoch signifikanten Unterschieden zwischen den Quintilen sowohl bei 30 Tagen (P = 0,008) und 6 Monaten Nachfolge (P = 0,004) führte. Für die 6-Monate Nachfolgedaten ergab die post hoc Analyse der PAPP-A Quintilen unter der Verwendung eines logistischen Regressionsmodells, daß nur die oberen zwei PAPP-A Quintilen (4. Quintile: P = 0,034; 5. Quintile: P = 0,002) sich signifikant von der ersten PAPP-A Quintile unterschieden, die als ein Referenz diente. In Übereinstimmung mit diesen Ergebnissen verifizierte die Receiver-operating Charakteristikakurven-Analyse einen Schwellenwert von 12,6 mIU/l PAPP-A für den maximierten Vorhersagewert (Figur 17).

### Stratifizierung gemäß PAPP-A Status

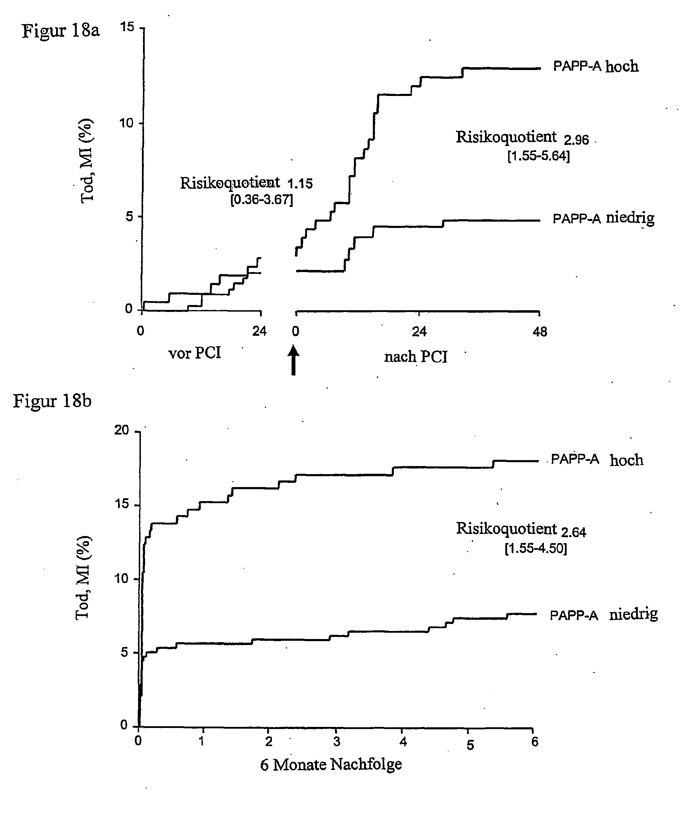

Basierend auf den oben genannten Ergebnissen wurde die Studienopulation gemäß dem berechneten Schwellenwert von 12,6 mIU/l dichotomisiert, was zu 219 Patienten mit erhöhten PAPP-A Spiegeln (40,0%) führte. Neben höheren Spiegeln von jeweils hsCRP und sCD40L in Patienten mit erhöhten PAPP-A Plasmaspiegeln, waren die Basislinien-Charakteristika in Patienten mit erhöhten PAPP-A Plasmaspiegeln nicht signifikantl verschieden von Patienten mit niedrigen PAPP-A Plasmaspiegeln (Tabelle 5). Die Odds-Verhältnisse für Tod und Myokardinfarkt (eingestellt auf Unterschiede in den Basislinien-Charakteristika) waren 1,15 (95% CI 0,36-3,67; P = 1,00) bei 24 h, 2,96 (95% CI 1,55-5,64; P = 0,002) bei 72 h (Figur 18a), 2,84 (95% CI 1,55-5,22; P = 0,001) bei 30 Tagen und 2,64 (95% CI 1,55-4,50; P 0,001) bei 6 Monaten (Figur 18b). Sechs-Monate kumulative Ereignisraten in Patienten mit niedrigen PAPP-A Spiegeln waren 7,9% versus 17,4% für Patienten mit hohen PAPP-A Spiegeln. Dieser Unterschied in den Ereignisraten wurde nicht nur durch eine höhere Rate von nicht-fatalem Myokardinfarkt verursacht, sondern auch durch eine höhere Mortalität in Patienten mit verringertem PAPP-A Plasmaspiegel (3,2% versus 1,2%; P = 0,098). Übereinstimmend waren eilige Revaskularisierungsverfahren, einschließlich perkutaner Koronarintervention und koronarer arterielles Bypass-Grafting signifikant höher in Patienten mit erhöhtem PAPP-A Plasmaspiegel (13,6% versus 7,9%; P = 0,012). Nicht-eilige Revaskularisierungsverfahren während der 6 Monate der Nachverfolgung zeigten ein höheres Auftreten in Patienten mit hohen PAPP-A Plasmaspiegel im Vergleich zu Patienten mit niedrigen PAPP-A Plasmaspiegeln (34,4% versus 19,7%; P = 0,005).

### Multimarker-Überlegungen

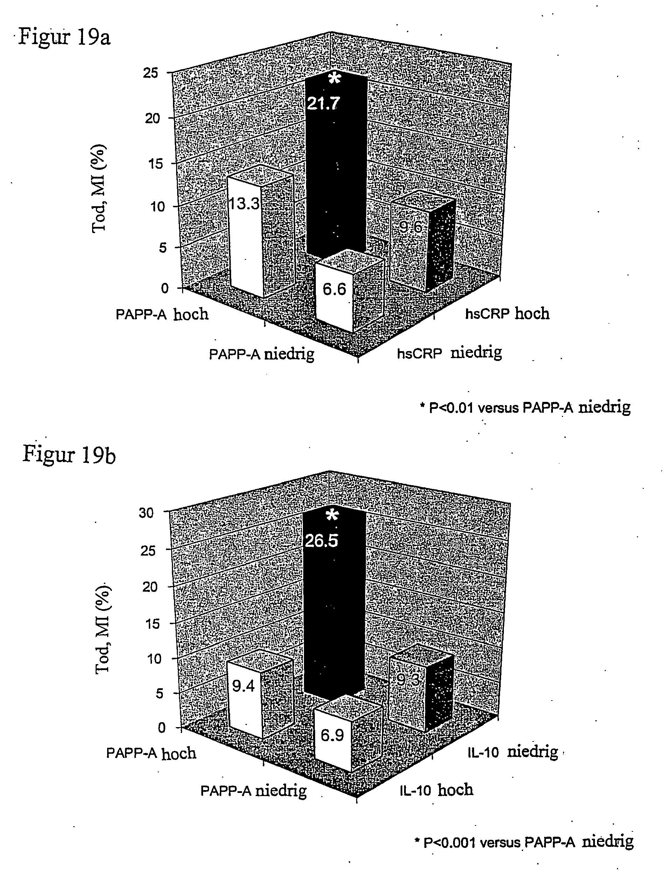

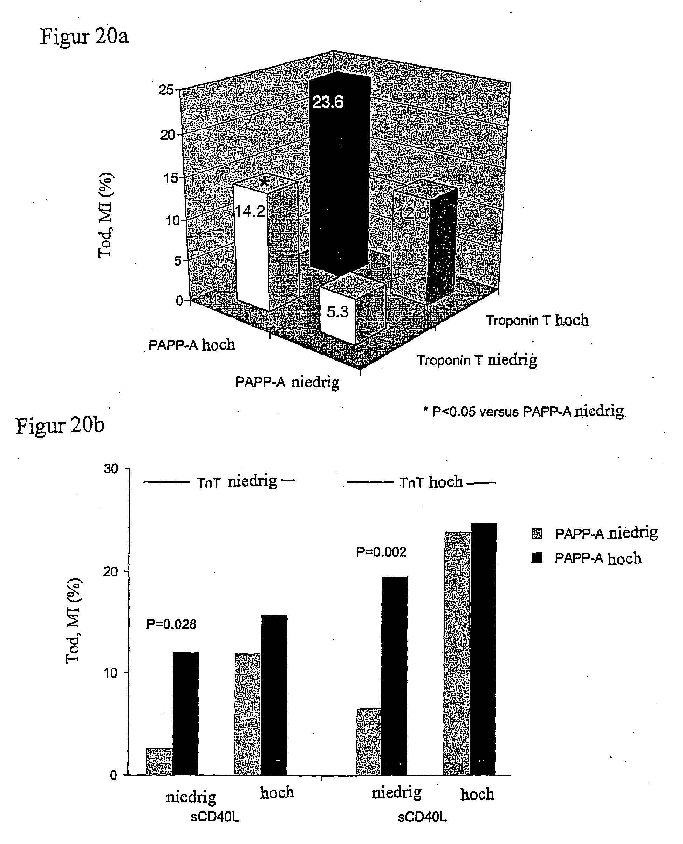

Die Erfinder maßen gleichzeitig Marker myokardialer Nekrose (TnT), Ischämie (VEGF), Inflammation (hsCRP, PAPP-A), anti-inflammatorischer Aktivität (IL-10) und Plättchen-Aktivierung (sCD40L). Bemerkenswerterweise war der prädiktive Wert of PAPP-A auf Patienten mit erhöhten hsCRP Spiegeln (Figur 19a) begrenzt. Wenn der hsCRP Plasmaspiegel erhöht war (oberhalb 10 mg/1), zeigten Patienten mit einem PAPP-A Plasmaspiegel oberhalb des berechneten Schwellenwerts von 12,6 mIU/l ein erhöhtes kardiales Risiko auf Tod und nicht-fatalen Myokardinfarkt (eingestelltes Odds-Verhältnis 2,61 [1,25-5,62]; P = 0,007) (Figur 19a). Im Unterschied dazu diente für Patienten mit hsCRP Werten unterhalb von 10 mg/L PAPP-A nicht als ein signifikanter Prädiktor für das kardiovaskuläre Risiko (P = 0,073). Darüber hinaus hing der Vorhersagewert von PAPP-A eng mit related to dem Plasmaspiegel des anti-inflammatorischen Cytokins IL-10 (Figur 19b) zusammen. Wenn die IL-10 Plasmaspiegel oberhalb des berechneten Schwellenwerts von 3,5 ng/l waren, waren Patienten mit erhöhten PAPP-A Plasmaspiegel (oberhalb 12,6 mIU/l) von einem erhöhten kardialen Risiko (eingestelltes Odds-Verhältnis 1,40 [0,60 - 3,23]; P < 0,001) (Figur 19b) geschützt. Jedoch identifizierten für Patienten mit niedrigem IL-10 Plasmaspiegel, PAPP-A Werte oberhalb 12,6 mIU/l, eine Subgruppe von Patienten, die an einem besonders hohen kardiovaskulären Risiko (eingestelltes Odds-Verhältnis 3,52 [1,71 - 7,23]; P 0,001) litten. Zusammen genommen zeigen diese Daten, daß der Vorhersagewert von PAPP-A Plasmaspiegeln wichtig durch die Balance zwischen pro- und anti-inflammatorischen Cytokin-Plasmaspiegeln moduliert wird. Wichtigerweise war der Vorhersagewert von PAPP-A auch in Patienten ohne Hinweise auf myokardiale Nekrose ersichtlich. TnT-negative Patienten (Schwellenwert 0,1 ug/l) mit erhöhten PAPP-A Spiegeln were at signifikantl higher risk as compared to TnT-negativen Patienten mit niedrigen PAPP-A Spiegeln (eingestelltes Odds-Verhältnis 2,72 [1,25 - 5,89]; P = 0,009) (Figur 20a). Im Unterschied dazu litten TnT-positive Patienten an einem erhöhtem kardiovaskulären Risiko, das unabhängig von PAPP-A Plasmaspiegeln ist. The Vorhersagewert von PAPP-A wurde auch für einen verringerten Schwellenwert von 0.01 ug/L für TnT (eingestelltes Odds-Verhältnis 3,97 [1,24 - 12,68]; P = 0,016) beobachtet. In Patienten, die negativ sowohl für TnT als auch sCD40L waren, identifizierte PAPP eine Subgruppe, die an einem erhöhtem kardiovaskulären Risiko während 6 Monate von Nachfolge (Figur 20b) litt. Um weiter eine potentiell unabhängige prognostische Signifikanz of individual biochemischen Markern abzuleiten, wurde eine schrittweise multivariante logistische Regressionsanalyse wdurchgeführt, die die biochemichen Marker TnT, VEGF, hsCRP, PAPP-A, IL-10 und sCD40L sowie Basislinien Charakteristika einschloß, die einen signifikanten Vorhersagewert in einem univariaten Modell ergab. Für die Endpunkte Tod und nicht-fataler Myokardinfarkt bei 30-Tage und 6-Monate Nachfolge war keiner der etablierten Risikofaktoren ein unabhängiger Prädiktor, nachdem die dichotomisierten biochemischen Marker TnT, hsCRP und sCD40L in das Modell eingeführt wurden (Tabelle 6). Daher wurde die schrittweise multivariante Analyse auf biochemische Marker begrenzt. TnT (P = 0,008) und PAPP-A (P = 0,007) verblieben unabhängige signifikante Prädiktoren des Patienten-Ausgangs, wohingegen hsCRP die Signifikanz verlor, nachdem PAPP-A in das Modell eingeführt wurde (P = 0,003 ohne PAPP-A; P = 0,16 nach Einführung von PAPP-A) (Tabelle 7; Schritt I). PAPP-A blieb ein signifikanter Prädiktor des Patienten-Ausgangs nach der Aufnahme des anti-inflammatorischen Cytokins IL-10 (Tabelle 7; Schritt II; P = 0,006) und nach der Aufnahme von sCD40L als ein Marker der Plättchenaktivierung (Tabelle 7; Schritt III; P = 0,015). Nach der Aufnahme of VEGF als Marker der myokardialen Ischämie verlor TnT seinen Vorhersagewert für den 6-Monats Ausgang (Tabelle 7; Schritt IV; P = 0,24 nach der Aufnahme von VEGF versus P = 0,16 vor der Aufnahme von VEGF), wohinhgegen PAPP-A ein signifikanterunabhängiger Prädiktor blieb (P = 0,014).

Die gleichzeitige Bestimmung von Biomarkern mit distinkten pathophysiologischen Profilen verbessert die Risikostratifizierung in Patienten mit ACS dramatisch. Weil die PAPP-A, PIGF und sCD40L Spiegel relativ stabil sind und keine spezifische Probenbedingungen für PAPP-A, PIGF und sCD40L erforderlich sind, scheinen diese Marker für die Routine klinische Verwendung geeignet. Obwohl inhärente Begrenzungen für Marker verbleiben, die nicht für die Koronararterien und/oder das Myokard spezifisch sind, können PAPP-A, P1GF und sCD40L ein wichtiges Werkzeug für die diagnostische und therapeutische Stratifizierung von Patienten mit ACS ohne Hinweise auf myokardiale Nekrose darstellen.

**Tabelle 5: Base Line-Charakteristika gemäß dem PAPP-A Status**

| | PAPP-A niedrig | PAPP-A hoch | p-Wert |
|---|---|---|---|
| n | 328 | 219 | |
| männlich | 70,2 % | 71,9 % | 0,62 |
| Alter | 60,5 ± 11 | 62,2 ± 10,4 | 0,39 |
| Troponin T ≥ 0,1 µg/l | 36,4 % | 39,2 % | 0,23 |
| VEGF > 300 ng/l | 50,8 % | 54,3 % | 0,43 |
| CRP ≥ 10,0 µg/l | 37,3 % | 56,2 % | < 0,001 |
| IL-10 < 3,5 ng/l | 57,3 % | 53,4 % | 0,38 |
| sCD40L > 5,0 µg/l | 33,4 % | 51,4 % | < 0,001 |
| ST-Segment Depression | 45,1 % | 53,6 % | 0,062 |
| T-Wellen Inversion | 51,7% | 51,7 % | 1,00 |
| Vorgeschichte von | | | |
| Angina > 4 Wochen | 55,5 % | 56,3 % | 0,64 |
| Infarkt < 30 Tage | 13,3 % | 12,5 % | 0,89 |
| Infarkt > 30 Tage | 20,3 % | 20,6 % | 0,90 |
| PTCA | 17,6% | 17,4 % | 0,75 |
| CABG | 3,4 % | 3,5 % | 0,98 |
| Risikofaktoren | | | |
| Diabetes | 9,5 % | 10,7 % | 0,97 |
| Bluthochdruck | 34,6 % | 37,4 % | 0,64 |
| akuter Raucher | 40,9 % | 42,6 % | 0,41 |
| Medikation vor Registrierung | | | |
| Aspirin | 98,1 % | 97,8 % | 1,00 |
| Heparin i.v. | 99,0 % | 98,8 % | 1,00 |
| Nitrate i.v. | 99,5% | 99,2 % | 1,00 |
| Beta-Blocker | 63,5 % | 62,9 % | 0,94 |

**Tabelle 6: Multivariantes Cox proportionales-Gefahren-Regressionsmodell für tödlichen und nicht-tödlichen Myokardinfarkt innerhalb der ersten 6 Monate der Nachuntersuchung**

| **Variable** | **eingestelltes Gefahren-Verhältnis** | **95% CI** | **p-Wert** |
|---|---|---|---|
| Geschlecht | 0,91 | 0,68 bis 1,39 | 0,16 |
| Alter > 65 Jahre | 1,36 | 0,91 bis 1,82 | 0,34 |
| Diabetes mellitus | 1,22 | 0,83 bis 1,49 | 0,61 |
| Hypercholesterinämie | 0,90 | 0,68 bis 1,13 | 0,59 |
| Bluthochdruck | 1,00 | 0,89 bis 1,04 | 1,00 |
| Geschichte einer Koronar- | 0,86 | 0,65 bis 1,19 | 0,72 |
| Revaskularisierung | | | |
| ST-Senkung | 1,29 | 0,72 bis 2,31 | 0,39 |
| Troponin T > 0,1 µg/l | 2,23 | 1,25 bis 3,98 | 0,007 |
| hsCRP > 10,0 mg/l | 2,03 | 1,11 bis 3,59 | 0,018 |
| PAPP-A > 12,6 mIU/l | 2,33 | 1,30 bis 4,17 | 0,005 |

**Tabelle 7: Multimarker Untersuchungen - schrittweises multivariantes/logistisches Regressionsmodell für tödlichen und nicht-tödlichen Myokardinfarkt innerhalb der ersten 6 Monate der Nachuntersuchung**

| Schritt I | | | | | | |
|---|---|---|---|---|---|---|
| Variable | Regressions-Koeffizient B | SE | Wald | P-Wert | Exp(B) | 95% CI |
| CRP | 0,38 | 0,28 | 1,82 | 0,16 | 1,49 | 0,86 bis 2,59 |
| TnT | 0,72 | 0,27 | 6,88 | 0,008 | 2,07 | 1,21 bis 3,56 |
| PAPP-A | 0,83 | 0,27 | 9,01 | 0,007 | 2,13 | 1,24 bis 3,68 |

| Schritt II | | | | | | |
|---|---|---|---|---|---|---|
| Variable | Regressions-Koeffizient B. | SE | Wald | P-Wert | Exp(B) | 95% CI |
| CRP | 0,20 | 0,29 | 0,46 | 0,50 | 1,44 | 0,67 bis 2,17 |
| TnT | 0,75 | 0,27 | 7,44 | 0,006 | 2,13 | 1,24 bis 3,69 |
| PAPP-A | 0,78 | 0,28 | 7,70 | 0,006 | 2,18 | 1,25 bis 3,78 |
| IL-10 | - 0,76 | 0,29 | 6,99 | 0,008 | 0,47 | 0,26 bis 0,82 |

| Schritt III | | | | | | |
|---|---|---|---|---|---|---|
| Variable | Regressions-Koeffizient B | SE | Wald | P-Wert | Exp(B) | 95% CI |
| CRP | 0,20 | 0,30 | 0,47 | 0,49 | 1,23 | 0,68 bis 2,19 |
| TnT | 0,66 | 0,28 | 5,51 | 0,019 | 1,93 | 1,12 bis 3,35 |
| PAPP-A | 0,70 | 0,29 | 5,60 | 0,015 | 2,05 | 1,18 bis 3,32 |
| IL-10 | - 0,86 | 0,28 | 9,29 | 0,002 | 0,42 | 0,24 bis 0,74 |
| SCD40L | 0,90 | 0,30 | 9,61 | 0,002 | 2,45 | 1,39 bis 4,32 |

| Schritt IV | | | | | | |
|---|---|---|---|---|---|---|
| Variable | Regressions-Koeffizient B | SE | Wald | P-Wert | Exp(B) | 95% CI |
| TnT | 0,36 | 0,31 | 1,38 | 0,24 | 1,43 | 0,79 bis 2,60 |
| PAPP-A | 0,69 | 0,29 | 5,85 | 0,014 | 2,01 | 1,14 bis 3,49 |
| IL-10 | - 0,84 | 0,28 | 8,66 | 0,003 | 0,43 | 0,25 bis 0,76 |
| SCD40L | 0,86 | 0,29 | 8,76 | 0,003 | 2,37 | 1,34 bis 4,18 |
| VEGF | 0,78 | 0,33 | 5,63 | 0;018 | 2,29 | 1,14 bis 4,18 |

## Patentansprüche

1. Verfahren zur Analyse von Proben in Zusammenhang mit akuten kardiovaskulären Erkrankungen, wobei das Verfahren folgende Schritte umfaßt:
(a) zur Verfügung stellen einer zu untersuchenden biologischen Probe aus einem Subjekt;
(b) Bestimmen der Konzentration des Markers P1GF in der Probe,
(c) gegebenenfalls, Bestimmen der Konzentration mindestens eines weiteren Markers ausgewählt aus löslichem CD40-Ligand (sCD40L), PAPP-A, Troponin T (TnT), MPO, NT-proBNP, VEGF, BNP und weiteren entzündlichen Markern, und
(d) Vergleich des/der für die zu untersuchende Probe erhaltenen Ergebnisse/s mit Referenzwerten und/oder den Werten aus Referenzproben.

2. Verfahren nach Anspruch 1, wobei die zu untersuchende Probe und/oder die Referenzprobe aus einem Menschen stammt.

3. Verfahren nach Anspruch 1 oder 2, wobei die zu untersuchende Probe ausgewählt ist aus der Gruppe bestehend aus peripherem Blut oder Fraktionen davon und Zellkultur-Suspensionen oder Fraktionen davon.

4. Verfahren nach Anspruch 3, wobei die zu untersuchende Probe Blutplasma oder Blutserum ist.

5. Verfahren nach Anspruch 3, wobei dem peripheren Blut ein Gerinnungshemmer, insbesondere Heparin, zugesetzt wird.

6. Verfahren nach einem der Ansprüche 1 bis 5, wobei die weiteren entzündlichen Marker ausgewählt sind aus CRP/(hs)CRP und IL-10.

7. Verfahren nach einem der Ansprüche 1 bis 5, wobei die analysierten Marker und Kombinationen daraus ausgewählt sind aus P1GF; P1GF + TnT; sCD40L + P1GF; PAPP-A + P1GF; sCD40L + P1GF + TnT; PAPP-A + P1GF + TnT; sCD40L + PAPP-A + P1GF; und sCD40L + PAPP-A + P1GF + TnT.

8. Verfahren nach Anspruch 7, weiterhin umfassend die Analyse mindestens eines der Marker MPO, NT-proBNP, BNP, CRP/(hs)CRP und IL-10.

9. Verfahren nach einem der Ansprüche 1 bis 8, wobei das Bestimmen der Konzentration mittels eines immunologischen Verfahrens mittels an die Marker bindenden Moleküle erfolgt.

10. Verfahren nach einem der Ansprüche 1 bis 5, wobei die an die Marker bindenden Moleküle ausgewählt sind aus der Gruppe bestehend aus anti-Marker-Antikörpern oder Teilen davon und Marker-Rezeptoren oder Teilen davon.

11. Verfahren nach Anspruch 10, wobei die Antikörper, Teile oder Fragmente davon polyklonale Antikörper, monoklonale Antikörper, Fab-Fragmente, scFv-Fragmente und Diabodies umfassen.

12. Verfahren nach Anspruch 10 oder 11, wobei die Marker und/oder die an die Marker bindenden Moleküle in Lösung oder Matrix-immobilisiert vorliegen.

13. Verfahren nach einem der Ansprüche 10 bis 12, wobei die an die Marker bindenden Moleküle an eines oder mehrere Nachweisgruppen aus der Gruppe bestehend aus Fluoresceinthioisocyanat, Phycoerythrin, einem Enzym und magnetisches Bead gekoppelt sind.

14. Verfahren nach einem der Ansprüche 10 bis 13, wobei die an die Marker bindenden Moleküle mit einem Antikörper, an den eine oder mehrere Nachweismoleküle gekoppelt sind, nachgewiesen werden.

15. Verfahren nach einem der Ansprüche 10 bis 14, wobei die immunologischen Verfahren ausgewählt sind aus der Gruppe bestehend aus Sandwich-Enzym-Immuntest, ELISA und Festphasen-Immuntests.

16. Verfahren nach einem der Ansprüche 1 bis 15, wobei die kardiovaskulären Erkrankungen ausgewählt sind aus der Gruppe bestehend aus instabiler Angina, Myokardinfarkt, akutem Herzsyndrom, koronarer Arterienerkrankung und Herzinsuffizienz.

17. Verfahren nach einem der Ansprüche 1 bis 16, wobei der Schwellenwert für die zu untersuchende Probe erhaltenen Ergebnisse bei 27,0 ng/l an P1GF im Serum oder Plasma liegt.

18. Verfahren nach Anspruch 17, wobei der Schwellenwert für die zu untersuchende Probe erhaltenen Ergebnisse bei 10,0 µg/l an CRP/(hs)CRP im Serum oder Plasma liegt.

19. Verwendung eines diagnostischen Kits, umfassend mindestens einen Antikörper zum Nachweis von PIGF in einer Probe eines Patienten und Mittel zur anschließenden Quantifizierung von PIGF, gegebenenfalls zusammen mit weiteren Komponenten und/oder Hilfsstoffen, zur Diagnose und/oder Prognose akuter kardiovaskulärer Erkrankungen und/oder zur Überwachung von deren Therapie.

20. Verwendung nach Anspruch 19, wobei der Kit goldmarkierte polyklonale Maus-Indikatorantikörper, biotinylierte polyklonale Nachweisantikörper und eine Testvorrichtung, umfassend ein Fiberglas-Vlies, umfasst.

21. Verwendung des Verfahrens nach einem der Ansprüche 1 bis 18 zur Diagnose und/oder Prognose akuter kardiovaskulärer Erkrankungen und/oder zur Überwachung von deren Therapie. '

22. Verwendung des Verfahrens nach einem der Ansprüche 1 bis 18 zur Identifikation einer Untergruppe von Patienten mit niedrigen CRP/(hs)CRP Serum- oder Plasmaspiegeln, die an einem signifikant erhöhten kardialen Risiko leiden.

23. Verwendung nach Anspruch 21, wobei die Therapie die Verabreichung von Statinen, und/oder Inhibitoren des Glycoprotein IIb/III-Rezeptors umfaßt.

24. Verwendung nach Anspruch 21, wobei die Therapie die Verabreichung eines anti-inflammatorischen Mittels und eine chirurgische Behandlung, insbesondere eine Ballondilatation, umfaßt.

25. Verwendung des Verfahrens nach Anspruch 18 zur Unterscheidung zwischen Risikogruppen mit einem CRP/(hs)CRP-Serumspiegel von ≤ 10,0 µg/l im Serum oder Plasma.

26. Verwendung des Verfahrens nach einem der Ansprüche 1 bis 18 zur Unterscheidung zwischen Patienten, die auf eine Therapie mit Abciximab reagieren oder nicht reagieren.

## Claims

1. A method for analysing samples in connection with acute cardiovascular diseases, wherein said method comprises the following steps:
(a) providing a biological sample to be examined from a subject;
(b) determining the concentration of the marker P1GF in the sample,
(c) optionally, determining the concentration of at least one additional marker selected from soluble CD40-ligand (sCD40L), PAPP-A, Troponin T (TnT), MPO, NT-proBNP, VEGF, BNP, and additional inflammatory markers, and
(d) comparing the result/s as obtained for the sample/s to be analysed with reference values and/or the values from reference samples.

2. The method according to claim 1, wherein said sample to be examined and/or the ref erence sample is derived from a human.

3. The method according to claim 1 or 2, wherein said sample to be examined is selected from the group consisting of peripheral blood or fractions thereof, and cell culture-suspensions or fractions thereof.

4. The method according to claim 3, wherein said sample to be examined is blood plasma or blood serum.

5. The method according to claim 3, wherein a coagulation inhibiting agent, in particular heparin, is added to the peripheral blood.

6. The method according to any of claims 1 to 5, wherein said additional inflammatory markers are selected from CRP/(hs)CRP and IL-10.

7. The method according to any of claims 1 to 5, wherein said analysed markers and combinations thereof are selected from P1GF; P1GF + TnT; sCD40L + P1GF; PAPP-A + P1GF; sCD40L + P1GF + TnT; PAPP-A + P1GF + TnT; sCD40L + PAPP-A + P1GF; and sCD40L + PAPP-A + P1GF + TnT.

8. The method according to claim 7, further comprising the analysis of at least one of the markers MPO, NT-proBNP, BNP, CRP/(hs)CRP, and IL-10.

9. The method according to any of claims 1 to 8, wherein said determining the concentration takes place by means of an immunological method by means of molecules binding to said markers.

10. The method according to any of claims 1 to 5, wherein said molecules binding to said markers are selected from the group consisting of anti-marker antibodies or parts thereof, and marker-receptors or parts thereof.

11. The method according to claim 10, wherein said antibodies, parts or fragments thereof comprise polyclonal antibodies, monoclonal antibodies, Fab-fragments, scFv-fragments, and diabodies.

12. The method according to claim 10 or 11, wherein said markers and/or said molecules binding to said markers are present in solution or are immobilised to a matrix.

13. The method according to any of claims 10 to 12, wherein said molecules binding to said markers are coupled to one or more detection moieties from the group consisting of fluorescein thioisocyanate, phycoerythrin, an enzyme, and a magnetic bead.

14. The method according to any of claims 10 to 13, wherein said molecules binding to said markers are detected with an antibody coupled with one or more detection molecules.

15. The method according to any of claims 10 to 14, wherein said immunological methods are selected from the group consisting of sandwich-enzyme-immune test, ELISA and solid phase-immune tests.

16. The method according to any of claims 1 to 15, wherein said cardiovascular diseases are selected from the group consisting of unstable angina, myocardial infarction, acute heart syndrome, coronary artery disease, and heart insufficiency.

17. The method according to any of claims 1 to 16, wherein said threshold value for the results as obtained for the sample to be examined is found at 27.0 ng/l of P1GF in serum or plasma.

18. The method according to claim 17, wherein said threshold value for the results as obtained for the sample to be examined is found at 10.0 µg/l of CRP/(hs)CRP in serum or plasma.

19. Use of a diagnostic kit, comprising at least one antibody for detecting P1GF in a sample from a patient, and means for the subsequent quantification of P1GF, optionally together with additional components and/or auxiliary agents, for the diagnosis and/or prognosis of acute cardiovascular diseases and/or for monitoring of their therapy.

20. Use according to claim 19, wherein said kit comprises gold-labelled polyclonal mouse-indicator antibodies, biotinylated polyclonal detection antibodies, and a testing device comprising a fibreglass-fleece.

21. Use of the method according to any of claims 1 to 18 for the diagnosis and/or prognosis of acute cardiovascular diseases and/or for monitoring of their therapy.

22. Use of the method according to any of claims 1 to 18 for the identification of a subgroup of patients with low serum or plasma levels of CRP/(hs)CRP that suffer from a significantly elevated cardiac risk.

23. Use according to claim 21, wherein said therapy comprises the administration of statins, and/or inhibitors of the glycoprotein IIb/III-receptor.

24. Use according to claim 21, wherein said therapy comprises the administration of an anti-inflammatory agent, and a surgical treatment, in particular a balloon dilatation.

25. Use of the method according to claim 18 for distinguishing between risk groups with a CRP/(hs)CRP-serum level of ≤ 10.0 µg/l in serum or plasma.

26. Use of the method according to any of claims 1 to 18 for distinguishing between patients that react or do not react to a therapy with Abciximab.

## Revendications

1. Procédé d'analyse d'échantillon en relation avec des maladies cardiovasculaires aiguës, le procédé comprenant les étapes suivantes :
(a) mettre à disposition un échantillon biologique à analyser d'un sujet ;
(b) déterminer la concentration du marqueur PIGF dans l'échantillon,
(c) éventuellement, déterminer la concentration d'au moins un autre marqueur choisi parmi le ligand CD40 soluble (sCD40L), PAPP-A, la troponine T (TnT), MPO, NT-proBNP, VEGF, BNP et autres marqueurs inflammatoires et
(d) comparer le/les résultats obtenu/obtenus pour l'échantillon à analyser avec des valeurs de référence et/ou les valeurs d'échantillons de référence.

2. Procédé selon la revendication 1, dans lequel l'échantillon à analyser et/ou l'échantillon de référence provient d'un être humain.

3. Procédé selon la revendication 1 ou 2, dans lequel l'échantillon à analyser est choisi dans le groupe comprenant le sang périphérique ou des fractions de celui-ci et des suspensions de culture cellulaire ou des fractions de celles-ci.

4. Procédé selon la revendication 3, dans lequel l'échantillon à analyser est le plasma sanguin ou le sérum sanguin.

5. Procédé selon la revendication 3, dans lequel on ajoute au sang périphérique, un inhibiteur de la coagulation, en particulier de l'héparine.

6. Procédé selon l'une quelconque des revendications 1 à 5, dans lequel les autres marqueurs inflammatoires sont choisis parmi CRP/(hs)CRP et IL-10.

7. Procédé selon l'une quelconque des revendications 1 à 5, dans lequel les marqueurs analysés et leurs combinaisons sont choisis parmi PIGF ; PIGF + TnT ; sCD40L + PIGF ; PAPP-A + PIGF ; sCD40L + PIGF + TnT ; PAPP-A + PIGF + TnT ; sCD40L + PAPP-A + PIGF ; et sCD40L + PAPP-A + PIGF + TnT.

8. Procédé selon la revendication 7, comprenant l'analyse d'au moins l'un des marqueurs MPO, NT-proBNP, BNP, CRP/(hs)CRP et IL-10.

9. Procédé selon l'une quelconque des revendications 1 à 8, dans lequel la détermination de la concentration s'effectue au moyen d'un procédé immunologique par le biais des molécules se liant au marqueur.

10. Procédé selon l'une quelconque des revendications 1 à 5, dans lequel les molécules se liant au marqueur sont choisies dans le groupe comprenant des anticorps anti-marqueur ou des parties de ceux-ci et des récepteurs de marqueurs ou des parties de ceux-ci.

11. Procédé selon la revendication 10, dans lequel les anticorps, parties ou fragments de ceux-ci comprennent des anticorps polyclonaux, des anticorps monoclonaux, des fragments Fab, des fragments scFc et des diacorps.

12. Procédé selon la revendication 10 ou 11, dans lequel les marqueurs et/ou les molécules se liant aux marqueurs se présentent en solution ou immobilisés dans une matrice.

13. Procédé selon l'une quelconque des revendications 10 à 12, dans lequel les molécules se liant aux marqueurs sont couplées à un ou plusieurs groupes de détection du groupe comprenant le thioisocyanate de fluorescéine, la phycoérythrine, une enzyme et des billes magnétiques.

14. Procédé selon l'une quelconque des revendications 10 à 13, dans lequel les molécules se liant aux marqueurs sont mises en évidence avec un anticorps auquel une ou plusieurs molécules de détection sont couplées.

15. Procédé selon l'une quelconque des revendications 10 à 14, dans lequel les procédés immunologiques sont choisis dans le groupe comprenant un test immunologique enzymatique en sandwich, un test ELISA ou des tests immunologiques en phase solide.

16. Procédé selon l'une quelconque des revendications 1 à 15, dans lequel les maladies cardiovasculaires sont choisies dans le groupe comprenant l'angine de poitrine instable, l'infarctus du myocarde, le syndrome cardiaque aigu, les coronaropathies et l'insuffisance cardiaque.

17. Procédé selon l'une quelconque des revendications 1 à 16, dans lequel la valeur seuil des résultats obtenus pour l'échantillon à analyser se situe à 27,0 ng/l de PIGF dans le sérum ou dans le plasma.

18. Procédé selon la revendication 17, dans lequel la valeur seuil des résultats obtenus pour l'échantillon à analyser se situe à 10,0 µg/l de CRP/(hs)CRP dans le sérum ou le plasma.

19. Utilisation d'un kit de diagnostic, comprenant au moins un anticorps pour détecter PIGF dans un échantillon d'un patient et moyen de quantification consécutive de PIGF, éventuellement associé à d'autres composants et/ou adjuvants, pour le diagnostic et/ou le pronostic de maladies cardiovasculaires aiguës et/ou pour surveiller leur traitement.

20. Utilisation selon la revendication 19, dans laquelle le kit comprend des anticorps indicateurs de souris polyclonaux marqués à l'or, des anticorps de détection polyclonaux biotinylés et un dispositif de test comprenant un tapis en fibres de verre.

21. Utilisation du procédé selon l'une quelconque des revendications 1 à 18 pour le diagnostic et/ou le pronostic de maladies cardiovasculaires aiguës et/ou pour la surveillance de leur traitement.

22. Utilisation du procédé selon l'une quelconque des revendications 1 à 18, pour l'identification d'un sous-groupe de patients présentant de faibles taux sériques ou plasmatiques de CRP/(hs)CRP qui entraînent un risque cardiaque significativement accru.

23. Utilisation selon la revendication 21, dans laquelle le traitement comprend l'administration de statines et/ou d'inhibiteurs du récepteur de la glycoprotéine IIb/III.

24. Utilisation selon la revendication 21, dans laquelle le traitement comprend l'administration d'un agent anti-inflammatoire et un traitement chirurgical, en particulier une dilatation par ballonnet.

25. Utilisation du procédé selon la revendication 18, pour différencier des groupes à risque présentant un taux sérique de CRP/(hs)CRP ≤ 10,0 µg/l dans le sérum ou le plasma.

26. Utilisation du procédé selon l'une quelconque des revendications 1 à 18 pour différencier des patients qui réagissent ou ne réagissent pas à un traitement par Abciximab.
